(19) Europäisches Patentamt
European Patent Office
Office européen des brevets

(11) Publication number: **0 252 670 B1**

(12) **EUROPEAN PATENT SPECIFICATION**

(45) Date of publication of patent specification: **15.01.92**

(51) Int. Cl.$^5$: **C07D 241/18**, C07D 241/44, A61K 31/495

(21) Application number: **87305796.2**

(22) Date of filing: **30.06.87**

(54) **2-Substituted alkoxy-3-substituted-pyrazines.**

(30) Priority: **30.06.86 JP 153742/86**
**30.06.86 JP 153743/86**

(43) Date of publication of application:
**13.01.88 Bulletin 88/02**

(45) Publication of the grant of the patent:
**15.01.92 Bulletin 92/03**

(84) Designated Contracting States:
**DE ES FR IT**

(56) References cited:
**FR-A- 1 473 815**
**FR-A- 2 070 687**
**FR-A- 2 367 065**

**CHEMICAL ABSTRACTS, vol. 102, no. 25, 24th June 1985, page 597, abstract no. 220898p, Columbus, Ohio, US; TERUMO CORP.: "Pyrazine derivatives"**

(73) Proprietor: **TOYO JOZO KABUSHIKI KAISHA**
**632-1 Mifuku Ohito-cho**
**Tagata-gun Shizuoka-ken(JP)**

(72) Inventor: **Yaso, Masao**
**848-1, Takyo, Ohito-cho**
**Tagata-gun, Shizuoka-ken(JP)**
Inventor: **Suzuki, Yukio**
**525-1, Mifuku, Ohito-cho**
**Tagata-gun, Shizuoka-ken(JP)**
Inventor: **Shibata, Kensuke**
**846-3, Nanjo, Nirayama-cho**
**Tagata-gun, Shizuoka-ken(JP)**
Inventor: **Mochizuki, Daisuke**
**711-2, Jike, Nirayama-cho**
**Tagata-gun, Shizuoka-ken(JP)**
Inventor: **Hayashi, Eiichi**
**7-32, Midori-cho**
**Shizuoka-ken, Shizuoka-shi(JP)**

(74) Representative: **Myerscough, Philip Boyd et al**
**J.A.Kemp & Co. 14, South Square Gray's Inn**
**London, WC1R 5EU(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to novel 2-substituted alkoxy-3-substituted pyrazines, which are useful as pharmaceuticals for treating circulatory and metabolic disorders. The compounds of the invention are active platelet aggregation inhibiting, vasodilating and/or antilipoperoxide generating agents.

Recently, a significant number of compounds having platelet aggregation inhibiting activity have been reported. Of these, the only known compounds having a pyrazine or 5, 6, 7, 8 - tetrahydro quinoxaline ring in the basic structure are tetramethyl pyrazine (16th Heterocyclic Chemistry Symposium (Osaka) pp.65-68 (1984) and 2-higher fatty acid acyloxymethyl pyrazine (JP-A-59-219269). Furthermore a compound of formula

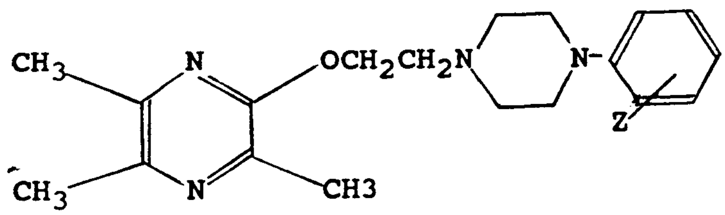

wherein Z is hydrogen or methyl, is known as an α-sympathetic nerve blocking agent (JP-A-48-21949). These compounds have almost no platelet aggregation inhibiting, vasodilating or antioxidant activity.

It is of great importance to discover high quality pharmaceuticals having stronger inhibitory activities for platelet aggregation, in order to treat effectively circulatory and metabolic disorders.

We have found that 2-substituted alkoxy-3-substituted pyrazines display inhibitory action on platelet aggregation, vasodilation activity and/or anti-lipoperoxide generation and have excellent pharmaceutical properties.

The present invention provides a compound which is a pyrazine derivative of formula

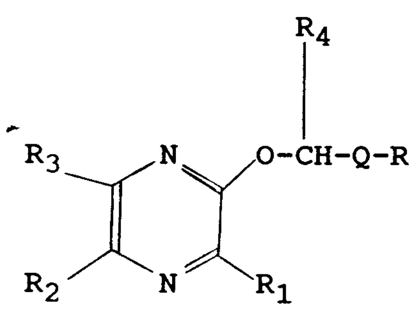

(1)

wherein:

Q is -CO- or $-CH_2-$, R is hydroxyl, $C_1$-$C_4$ alkoxy, a halogen, -NH-$C_1$-$C_4$ alkylene-OH, -NH-$C_1$-$C_4$ alkylene-arylthio,

$-NH-C_1-C_4$ alkylene-halogen, 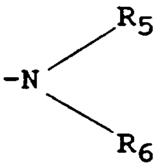 or $-NH-C_1-C_4$ alkylene 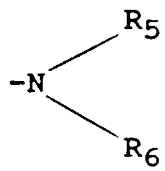, in which 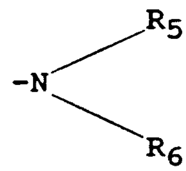 is di-$C_1$-$C_4$ alkylamino, morpholino, 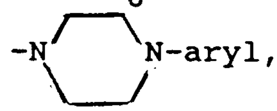,

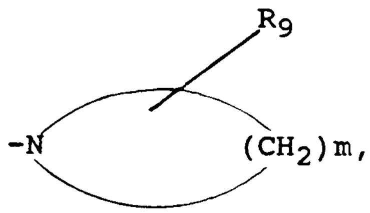 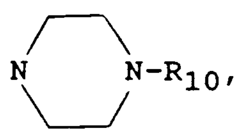 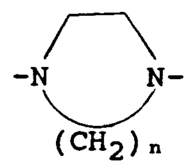

$C_1$-$C_4$ alkylene-aryl

or

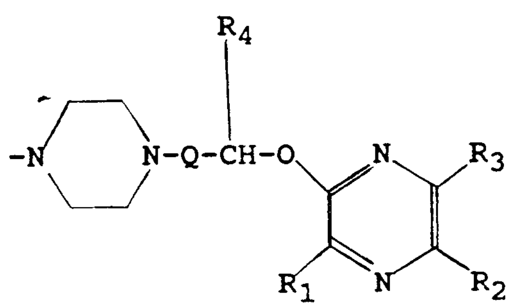

in which $R_9$ is hydrogen or $C_1$-$C_4$ alkyl, $R_{10}$ is hydrogen, $C_1$-$C_4$ alkyl, hydroxy -$C_1$-$C_4$ alkyl, hydroxy-$C_1$-$C_4$ alkoxy-$C_1$-$C_4$ alkyl or di(aryl) -$C_1$-$C_4$ alkyl, m is an integer of 4 to 6, n is 2 or 3, $R_1$ is -$C_1$-$C_{20}$ alkyl or aryl-$C_1$-$C_4$ alkyl, $R_2$ and $R_3$ are independently each $C_1$-$C_4$ alkyl, or together form a tetramethylene bridge, and $R_4$ is hydrogen, $C_1$-$C_4$ alkyl or aryl, wherein each aryl is phenyl optionally substituted by 1 to 3 halogens or nitro, $C_1$-$C_4$ alkyl or $C_1$-$C_4$ alkoxy groups, or a pharmaceutically acceptable salt thereof, with the proviso that when Q is $-CH_2-$, $R_1$, $R_2$ and $R_3$ are each methyl and $R_4$ is hydrogen, then R is not

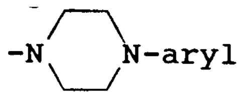

wherein "aryl" is 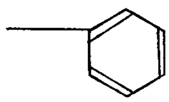 , 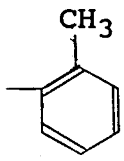 or 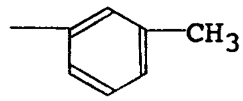 ,

The salts must be pharmacologically acceptable non-toxic salts. Examples of such salts are salts of an inorganic acid such as hydrochloric, sulfuric and phosphoric acid and salts of an organic acid such as acetic, propionic, butyric, glycolic, gluconic, malic, tartaric, succinic, mandelic, aspartic, glutamic, methanesulfonic and toluenesulfonic acid. Salts of other known acids can also be used.

The compound of formula (1) can be produced by the following processes:

Process A:

A compound of formula (1) where Q is -CO- and R is $C_1$-$C_4$ alkoxy (hereinafter designated as compound (1a)) can be produced by reacting a compound of the formula

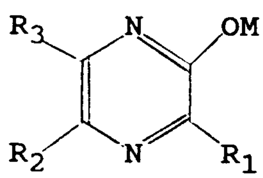

(3)

wherein M is alkaline metal, and $R_1$, $R_2$ and $R_3$ are as defined above in the compound of formula (1), with a $\alpha$-halogeno-carboxylate ester of formula

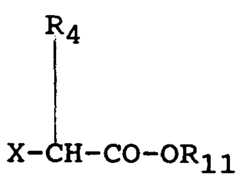

(4)

wherein X is a halogen, $R_{11}$ is $C_1$-$C_4$ alkyl and $R_4$ is as defined above in the compound of formula (1), in an organic solvent.

The compound of formula (3) can be obtained by dissolving a compound of formula

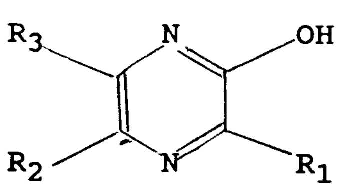

(2)

wherein $R_1$, $R_2$ and $R_3$ are as defined above, in methanol containing an alkaline metal methylate and distilling off methanol in vacuo.

The compound of formula (2) can be expressed as a tautomer of a compound of formula

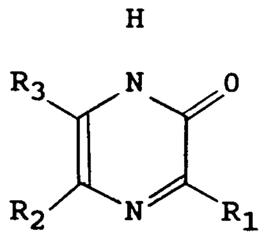

wherein $R_1$, $R_2$ and $R_3$ are as defined above.

In the compound of formula (2), $R_1$ is $-C_1-C_{20}$ alkyl or aryl-$C_1$-$C_4$ alkyl. "Alkyl", as used above, is defined as saturated or unsaturated $C_1$-$C_{20}$ alkyl, which may be branched or unbranched. Examples are methyl, ethyl, propyl, isopropyl, butyl, isobutyl, sec-butyl, t-butyl, pentyl, isopentyl, hexyl, heptyl, octyl, nonyl, decyl, undecyl, dodecyl, tridecyl, tetradecyl, pentadecyl or hexadecyl. "Optionally substituted phenyl", as used above, is defined as unsubstituted phenyl or phenyl substituted with $C_1$-$C_4$ alkyl, for example $C_{1-3}$ alkyl, halogen, nitro, or $C_1$-$C_4$ alkoxy. The aryl group in the aryl-$C_1$-$C_4$ alkyl, as used above, is defined as phenyl or phenyl substituted with 1 to 3 halogens or nitro, $C_1$-$C_4$ alkyl or $C_1$-$C_4$ alkoxy groups. Examples of the $C_1$-$C_4$ alkyl groups are methyl, 1-ethyl, 2-ethyl, 3-propyl and 1-propyl. Examples of the aryl-$C_1$-$C_4$ alkyl group are benzyl, p-chlorobenzyl, 2-phenylethyl and 1-phenylethyl.

In the compound of formula (2), $R_2$ and $R_3$ are independently each $C_1$-$C_4$ alkyl, or together form a tetramethylene bridge. The $C_1$-$C_4$ alkyl can be, for example, methyl, ethyl or propyl. Some compounds of formula (2) have been reported. These compounds can be produced by a process, or an improvement thereto, as disclosed in J. Am. Chem. Soc., 71:78-81 (1949) and ibid. 74:1580-1583 (1952). Novel derivatives can also be produced according to the methods described in the above references. The compound of formula (2) wherein $R_2$ and $R_3$ together form a tetramethylene bridge of formula

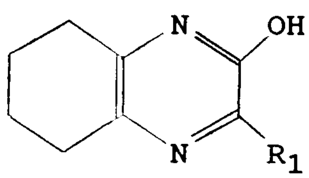

(2')

wherein $R_1$ is as defined above, i.e. a hexahydro quinoxaline derivative, is produced by reacting an $\alpha$-amino acidamide of formula

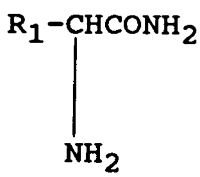

wherein $R_1$ is as defined above, or salt thereof, with 1, 2-cyclohexanedione in an alkaline medium.

Reaction of the $\alpha$-amino acidamide or salt thereof with 1, 2-cyclohexanedione proceeds in an organic solvent, for example a lower alcohol such as methanol or ethanol. The condensation reaction proceeds preferably at below 0 °C at first. However, when the reaction continues it can be carried out at room temperature. The reaction process can be checked by thin layer chromatography (TLC) or high performance liquid chromatography (HPLC) and is terminated at maximum production.

Isolation of the product of formula (2') can be performed by neutralizing the reaction mixture with an acid such as hydrochloric acid or sulfuric acid, thereafter extracting with water immiscible organic solvent such as chloroform under weakly alkaline conditions, and recrystallizing from an organic solvent such as acetone.

The thus obtained product of formula (2') can be purified, if necessary, by column chromatography using silica-gel, active alumina or an adsorption resin.

The group $R_4$ in the $\alpha$-halogenocarboxylate ester of formula (4) is hydrogen, $C_1$-$C_4$ alkyl or aryl.

Examples of $C_1$-$C_4$ alkyl are methyl, ethyl or propyl. The aryl is phenyl optionally substituted by 1 to 3 halogens or nitro, $C_1$-$C_4$ alkyl or $C_1$-$C_4$ alkoxy groups.

The group $R_{11}$ in the ester of formula (4) is a $C_1$-$C_4$ alkyl group such as methyl, ethyl or propyl. The group X is a halogen, such as chlorine, bromine or iodine.

The reaction of the compound of formula (3) with the $\alpha$-halogeno carboxylate ester of formula (4) is in general effected in an organic solvent such as dimethylformamide under heating. Isolation of the product of formula (1a) can be performed by distilling away the reaction solvent and extracting with a water-immiscible organic solvent such as benzene or chloroform.

Process B:

A compound of formula (1) wherein Q is -CO- and R is hydroxyl (hereinafter designated as compound (1b)) can be produced by de-esterifying a compound of formula (1a) by a known de-esterification procedure. For example, a compound of formula (1a) may be hydrolyzed by an alkaline hydroxide such as KOH or NaOH. In the case that unreacted compound of formula (1a) remains, after removing it by extracting with a water-immiscible organic solvent such as chloroform, the reaction mixture is neutralized with an acid and the precipitated product of formula (1b) is filtered or extracted with a water-immiscible organic solvent.

Process C:

A compound of formula (1) wherein Q is -CO- and R is -NH-$C_1$-$C_4$ alkylene-OH

or 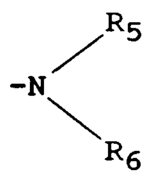

(hereinafter designated as compound (1c)) can be produced by reacting an amine of formula

H-R' (5)

wherein R' is -NH-$C_1$-$C_4$-alkylene-OH or

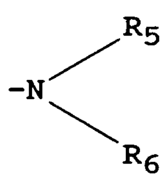

in which

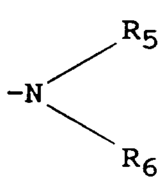

is di-$C_1$-$C_4$ alkylamino, morpholino,

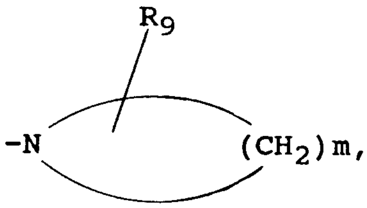, 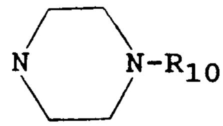 or

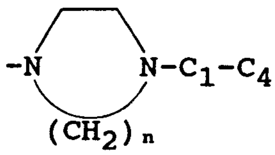

alkylene-aryl, and $R_9$, $R_{10}$ aryl, m and n are as defined above, with a compound of formula (1b) or a reactive derivative thereof suitable for N-acylation.

The $C_1$-$C_4$ alkylene group in R' in the above amine of formula (5) is optionally branched.

Examples of $C_1$-$C_4$ alkylene are methylene, ethylene, methylmethylene, propylene, 1-methylethylene, ethylmethylene, butylene, isobutylene or sec-butylene; $-(CH_2)_{1-3}$ is preferred.

The di-$C_1$-$C_4$ alkylamino group

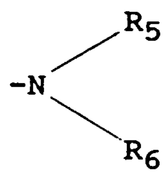

is for example dimethylamino, diethylamino or dipropylamino.

The ring 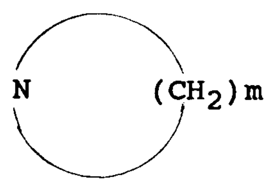

in the 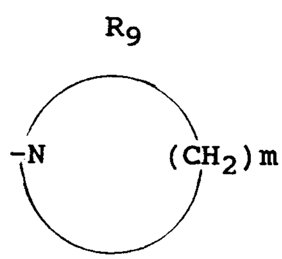 group,

wherein m is an integer of 4 to 6, is a pyrrolidine, piperidine or a hexamethyleneimine ring. $R_9$ is hydrogen or $C_1$-$C_4$ alkyl. The alkyl group is branched or unbranched $C_{1-4}$ alkyl. "Aryl" as used above means unsubstituted phenyl or phenyl substituted with 1 to 3 halogens or nitro, $C_1$-$C_4$ alkyl or $C_1$-$C_4$ alkoxy groups.

The group $R_{10}$ in the

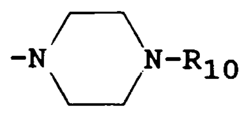 group

is hydrogen, $C_1$-$C_4$ alkyl, hydroxy-$C_1$-$C_4$ alkyl, hydroxy-$C_1$-$C_4$alkoxy-$C_1$-$C_4$ alkyl or diphenyl-$C_1$-$C_4$ alkyl. Examples of the above alkyl groups are branched or unbranched $C_{1-4}$ alkyl groups such as methyl, ethyl, propyl, isopropyl, butyl, isobutyl, sec-butyl and t-butyl. Examples of the above hydroxy-$C_1$-$C_4$ alkyl groups are hydroxymethyl, 2-hydroxyethyl and 3-hydroxypropyl. Examples of the hydroxy-$C_1$-$C_4$ alkoxy-$C_1$-$C_4$ alkyl groups are hydroxymethoxymethyl, (2-hydroxyethoxy) methyl, 2-(2-hydroxyethoxy) and 2-(hydroxymethoxy) ethyl. The phenyl group in the diphenyl-$C_1$-$C_4$ alkyl group is optionally substituted by 1-3 halogens or nitro, $C_1$-$C_4$ alkyl or $C_1$-$C_4$ alkoxy groups. Examples of the alkyl group are methyl and ethyl. An example of the diphenyl-$C_1$-$C_4$ alkyl group is diphenylmethyl.

The group 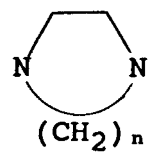 in the above 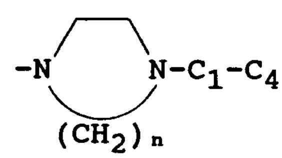

alkylene-aryl group wherein n is 2 or 3, is piperazine or homopiperazine. The $C_1$-$C_4$ alkylene bound to the above ring is defined hereinabove and is branched or unbranched.

Examples of the aryl group are unsubstituted phenyl or phenyl substituted with 1 to 3 halogens or nitro, $C_1$-$C_4$ alkyl or $C_1$-$C_4$ alkoxy groups, such as phenyl, tolyl, xylyl, p(m or o)-chlorophenyl, p(m or o)-fluorophenyl, p(m or o)-bromophenyl, 2,4(2,3-,3,4-,2,5-,3,5- or 2,6-)-dichlorophenyl, p(m or o)-nitrophenyl, p-(m or o)-methoxyphenyl and 2,4(2,3-,3,4-,2,5-,3,5-or 2,6)-dimethoxyphenyl.

Examples of the amine of formula (5) are dimethylamine, diethylamine, 2-hydroxyethylamine, cyclohexylamine, morpholine, pyrrolidine, piperidine, hexamethyleneimine, 4-methylpiperidine, 4-phenylpiperazine, piperazine, 4-methylpiperazine, 4-ethylpiperazine, 4-propylpiperazine, 4-butylpiperazine, 4-(2-hydroxyethyl)-piperazine, 4-(2-(2-hydroxyethoxy)ethyl)-piperazine, 4-benzyl-piperazine, 4-(o-chlorobenzyl)-piperazine, 4-(p-chlorobenzyl)-piperazine, 4-(2,4-dichlorobenzyl)-piperazine, 4-(p-nitrobenzyl)-piperazine, 4-(m-nitrobenzyl)-piperazine, 4-(o-nitrobenzyl)-piperazine, 4-(p-methoxybenzyl)-piperazine, 4-(methylbenzyl)-piperazine, 4-(diphenylmethyl)-piperazine, 4-benzyl-homopiperazine, 4-(p-methylbenzyl)-homopiperazine, 4-(p-chlorobenzyl)-homopiperazine, 4-(p-fluorobenzyl)-homopiperazine, 4-(p-nitrobenzyl)-homopiperazine and 4-(p-methoxybenzyl)-homopiperazine.

Reaction of the amine of formula (5) and the compound of formula (1b) or the reactive derivative thereof generally proceeds by the mixed anhydride method by reacting the compound of formula (1b) and the amine of formula (5) with pivaloyl chloride in the presence of a tertiary amine or tetrahydrofuran.

The compound of formula (1c) can be isolated by pouring the reaction mixture into dilute aqueous alkali and extracting with a water-immiscible organic solvent such as benzene or chloroform.

In the above reaction, when piperazine is used as the amine of formula (5), a compound of formula (1c) wherein

R is 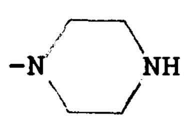 or

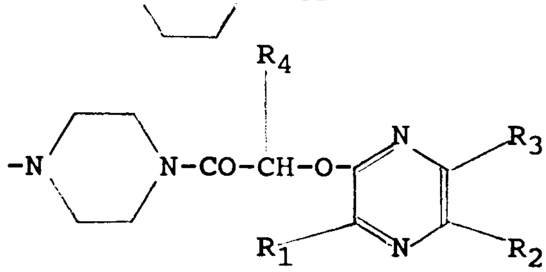

in which
$R_1$, $R_2$, $R_3$ and $R_4$ are as defined above is produced depending on the ratio of piperazine used.

Process D:

A compound of formula (1) wherein Q is -CO- and R is NH-$C_1$-$C_4$ alkylene-halogen (hereinafter designated as compound (1d)) can be produced by N-acylating a compound of formula

X-$C_1$-$C_4$ alkylene - $NH_2$ (6)

wherein X is a halogen and the $C_1$-$C_4$ alkylene is as defined above, with a compound of formula (1b) or a reactive derivative thereof.

The $C_1$-$C_4$ alkylene group in the amine of formula (6) is, as defined hereinbefore, a branched or unbranched $C_1$-$C_4$ alkylene group such as methylene, ethylene, methylmethylene, propylene, 1-methylethylene, ethylmethylene, butylene, isobutylene or sec-butylene; $-(CH_2)_{1-3}$ is preferred.

Examples of the amine of formula (6) are 2-chloroethylamine and 2-bromoethylamine.

Reaction of the amine of formula (6) with the compound of formula (1b) or the reactive derivative thereof is performed as mentioned in process C.

Process E:

A compound of formula (1) wherein Q is -CO- and R is

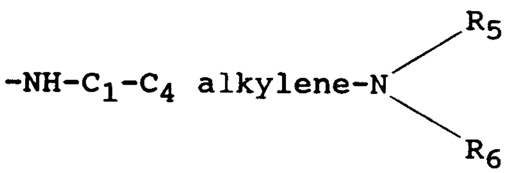

(hereinafter designated as compound (1e)) can be produced by aminating the compound of formula (1d) with an amine of formula

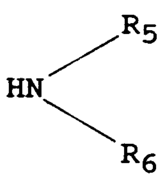

(7)

wherein

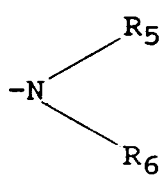

is defined above in formula (1).

The group

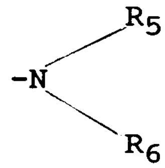

in the amine of formula (7) preferably has the same meaning of

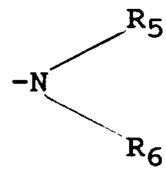

as defined in the amine of formula (5), and the aryl group in the

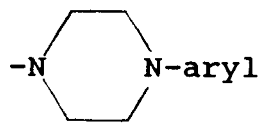

group means unsubstituted phenyl or phenyl substituted by 1 to 3 halogens or nitro, $C_1$-$C_4$ alkyl or $C_1$-$C_4$ alkoxy groups.

Examples of the amine of formula (7) are the amine of formula (5) and 4-phenylpiperazine, 4-(o-chlorophenyl)-piperazine, 4-(p-chlorophenyl)-piperazine, 4-(p-methoxyphenyl)-piperazine, 4-(m-methoxyphenyl)-piperazine and 4-(m-methoxyphenyl)-piperazine.

The above amination reaction can be effected in an inert organic solvent such as benzene in the presence of a tertiary organic amine such as triethylamine. Isolation of the product of formula (1e) can be performed by pouring the reaction mixture into diluted aqueous alkali and extracting with a water-immiscible organic solvent such as chloroform.

Process F:

A compound of formula (1) wherein Q is $-CH_2-$ and R is -NH-$C_1$-$C_4$ alkylene-arylthio (hereinafter designated as compound (1f)) can be produced by thioetherification of the compound of formula (1) wherein Q is $-CH_2-$ and R is -NH-$C_1$-$C_4$-alkylene-halogen with an aryl-thiol (8).

The aryl group in the aryl-thiol (8) means unsubstituted phenyl or phenyl substituted by 1 to 3 halogens or nitro, $C_1$-$C_4$ alkyl or $C_1$-$C_4$ alkoxy groups, such as thiophenol.

The thioetherification can be effected in a reaction solvent such as dimethylformamide in the presence of an alkali such as potassium carbonate or sodium carbonate. Isolation of the compound of formula (1f) can be performed by removing the reaction solvent and extracting with a water-immiscible organic solvent such as chloroform in a dilute aqueous alkali.

Process G:

A compound of formula (1) wherein Q is $-CH_2-$ and R is a hydroxyl group (hereinafter designated as compound (1g)) can be produced by reducing the compound of formula (1a) with an alkaline borohydride in the presence of a $C_1$-$C_4$ alcohol under heating.

Isolation of the product of formula (1g) can be performed by removing organic solvent from the reaction medium, adding water to the residue and extracting with a water-immiscible organic solvent such as chloroform.

Process H:

A compound of formula (1) wherein Q is $-CH_2$ and R is a halogen (hereinafter designated as compound

(1h)) can be produced by halogenating the compound of formula (1g) with a halogenating agent in a solvent.

The halogenating agent may be any known halogenating agent. Conventional chlorination reagents such as $SOCl_2$, $PCl_5$ and $POCl_3$ can be used. The halogenation reaction can be effected, in general, in an inert organic solvent such as chloroform. Reaction proceeds at room temperature. Isolation of the product of formula (1h) can be performed by adding a water-immiscible organic solvent such as chloroform, washing with dilute aqueous alkali, dehydrating the organic layer and removing the solvent therefrom.

The compound of formula (1h) can be used without purification, as by silica-gel column chromatography, to prepare a compound of formula (1j) below.

Process J:

A compound of formula (1) wherein Q is $-CH_2-$ and R is

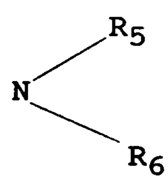

(hereinafter designated as compound (1j)) can be produced by aminating the compound of formula (1h) with an amine of formula (5) in a solvent under heating.

The above amination reaction can be effected in an organic solvent such as benzene.

In the above reaction, co-generated acid can be removed by an acid binder, for example a known tertiary organic amine such as triethylamine.

The product of formula (1j) can be isolated by pouring the reaction mixture into dilute aqueous alkali and extracting with a water immiscible organic solvent such as benzene or chloroform.

The thus-obtained compound of formula (1) is purified, if required, by column chromatography using silica-gel, activated alumina or an adsorption resin with an elution solvent such as chloroform-methanol or benzene-ethyl acetate.

A compound of formula (1) is generally produced in the form of free base, but it can also be produced in the form of a conventional salt thereof. For example, a hydrochloride can be prepared by dissolving a compound of formula (1) in a lower alcohol such as methanol or ethanol, adding a slightly molar excess amount of hydrochloric acid, and isolating the precipitated material, or if not precipitated, by adding ether therein to precipitate. The molar ratio of hydrochloric acid used may be different according to the specific compound of formula (1).

Examples of the compound of formula (1) of the present invention are set forth in Tables 1 and 2:

TABLE 1

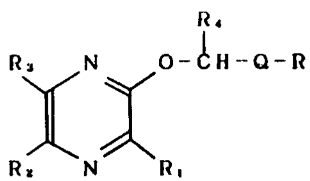

Me ; methyl
Et ; ethyl
Pro ; propyl
Bu ; butyl
Ph ; benzene ring
( ): position of substituent

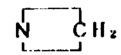 ; piperidine ring

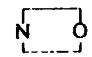 ; morpholine ring

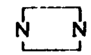 ; piperazine ring

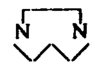 ; homopiperazine ring

| Compound No. | $R_1$ | $R_2$ | $R_3$ | $R_4$ | Q | R |
|---|---|---|---|---|---|---|
| 034 | Me | Me | Me | H | –CO– | –OMe |
| 035 | Me | Me | Me | Me | –CO– | –OMe |
| 036 | Me | Me | Me | Ph | –CO– | –OMe |
| 037 | Me | Me | Me | H | –CO– | –OH |
| 038 | Me | Me | Me | Me | –CO– | –OH |
| 039 | Me | Me | Me | Ph | –CO– | –OH |
| 066 | Me | Me | Me | H | –CO– | 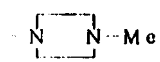 |
| 067 | Me | Me | Me | H | –CO– | 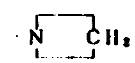 |
| 068 | Me | Me | Me | H | –CO– | 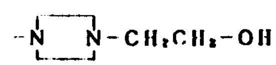 |
| 069 | Me | Me | Me | H | –CO– | –NH–$CH_2CH_2$–OH |
| 070 | Me | Me | Me | H | –CO– | 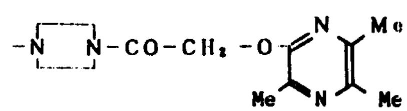 |

| | | | | | | |
|---|---|---|---|---|---|---|
| 071 | Me | Me | Me | H | $-CO-$ | $-NH-CH_2CH_2-NMe_2$ |
| 072 | Me | Me | Me | H | $-CO-$ | $-NH-CH_2CH_2-S-Ph$ |
| 073 | Me | Me | Me | H | $-CO-$ | $-NH-CH_2CH_2-$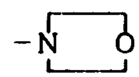 |
| 074 | Me | Me | Me | H | $-CO-$ | $-NH-CH_2CH_2-$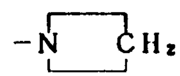 |

| Compound No. | $R_1$ | $R_2$ | $R_3$ | $R_4$ | Q | R |
|---|---|---|---|---|---|---|
| 075 | Me | Me | Me | H | -CO- | -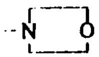 |
| 076 | Me | Me | Me | H | -CO- | $-NH-CH_2CH_2-$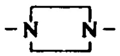$-CH_2CH_2-OH$ |
| 103 | Me | Me | Me | H | -CO- | $-NH-CH_2CH_2-$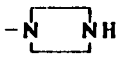 |
| 105 | Me | Me | Me | H | -CO- | $-NH-CH_2CH_2-$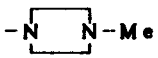-Me |
| 135 | Me | Me | Me | H | -CO | $-NH-CH_2CH_2-$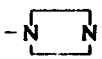$-CH_2Ph-Cl$ (p) |
| 136 | Me | Me | Me | H | -CO | $-NH-CH_2CH_2-$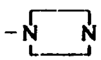$-CH_2Ph$ |
| 137 | Me | Me | Me | H | -CO | $-NH-CH_2CH_2-$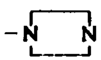$-CH_2Ph-Cl$ (o) |
| 341 | Et | Me | Me | Ph | -CO | $-NH-CH_2CH_2-$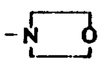 |
| 342 | Et | Me | Me | Ph | -CO | $-NH-CH_2CH_2-$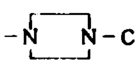$-CH_2Ph$ |
| 343 | Et | Me | Me | Ph | -CO | $-NH-CH_2CH_2-$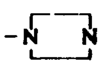$-CH_2Ph-Cl$ (p) |
| 344 | Et | Me | Me | Ph | -CO- | $-NH-CH_2CH_2-$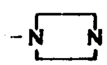$-CH_2Ph-NO_2$ (p) |
| 345 | Et | Me | Me | Ph | -CO- | $-NH-CH_2CH_2-$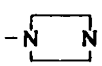$-CH_2Ph-Cl_2$ (2, 4) |

| | | | | | | |
|---|---|---|---|---|---|---|
| 346 | Et | Me | Me | Ph | -CO- | $-NH-CH_2CH_2-$ 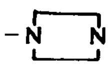 $-Ph$ |
| 347 | Et | Me | Me | Ph | -CO- | $-NH-CH_2CH_2-$ 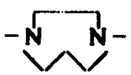 $-CH_2Ph-F$ (p) |
| 348 | Et | Me | Me | Ph | -CO- | $-NH-CH_2CH_2-$ 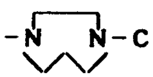 $-CH_2Ph-Cl$ (p) |
| 376 | $-CH_2Ph$ | Et | Et | H | -CO- | $-NH-CH_2CH_2-$ 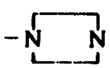 $-CH_2Ph$ |

| Compound No. | $R_1$ | $R_2$ | $R_3$ | $R_4$ | Q | R |
|---|---|---|---|---|---|---|
| 377 | $-CH_2Ph$ | Et | Et | H | -CO- | 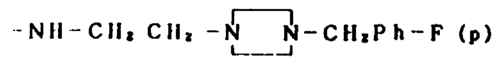 |
| 379 | 〃 | Et | Et | H | -CO- | 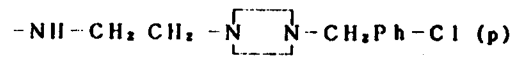 |
| 381 | 〃 | Et | Et | H | -CO- | 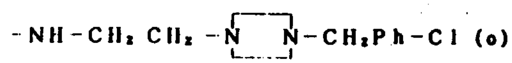 |
| 382 | 〃 | Et | Et | H | -CO- | 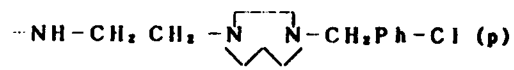 |
| 491 | Et | Me | Me | H | $-CH_2-$ | 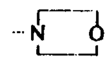 |
| 492 | Et | Me | Me | H | $-CH_2-$ | 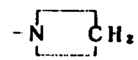 |
| 493 | Et | Me | Me | H | $-CH_2-$ | 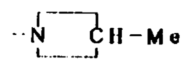 |
| 494 | Et | Me | Me | H | $-CH_2-$ | 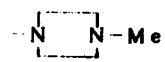 |
| 495 | Et | Me | Me | H | $-CH_2-$ | 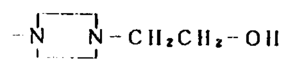 |
| 496 | Et | Me | Me | H | $-CH_2-$ | 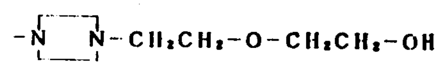 |
| 497 | Et | Me | Me | H | $-CH_2-$ | 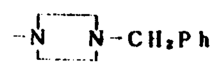 |
| 498 | Et | Me | Me | H | $-CH_2-$ | 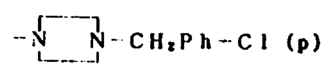 |

| | | | | | | |
|---|---|---|---|---|---|---|
| 499 | Et | Me | Me | H | $-CH_2-$ | 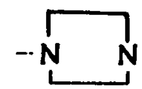 $-CH_2Ph-OMe$ (p) |
| 500 | Et | Me | Me | H | $-CH_2-$ | 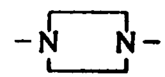 $-CH_2Ph-F$ (p) |
| 501 | Et | Me | Me | H | $-CH_2-$ | 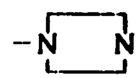 $-CH_2Ph-NO_2$ (p) |
| 502 | Et | Me | Me | H | $-CH_2-$ | 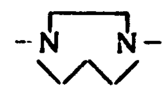 $-CH_2Ph-NO_2$ (p) |

| Compound No. | $R_1$ | $R_2$ | $R_3$ | $R_4$ | Q | R |
|---|---|---|---|---|---|---|
| 503 | Et | Me | Me | H | $-CH_2-$ | 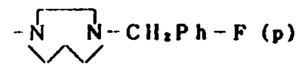 |
| 504 | Et | Me | Me | H | $-CH_2-$ | 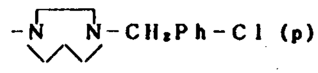 |
| 511 | $-CH_2Ph$ | Me | Me | H | $-CH_2-$ | 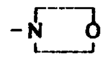 |
| 512 | 〃 | Me | Me | H | $-CH_2-$ | 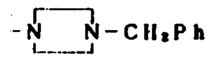 |
| 516 | 〃 | Me | Me | H | $-CH_2-$ | 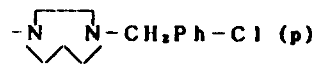 |
| 536 | Me | Me | Me | H | $-CH_2-$ | 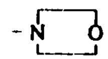 |
| 537 | Me | Me | Me | H | $-CH_2-$ | 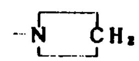 |
| 538 | Me | Me | Me | H | $-CH_2-$ | 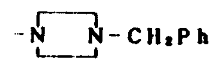 |
| 539 | Me | Me | Me | H | $-CH_2-$ | 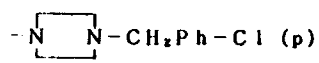 |
| 540 | Me | Me | Me | H | $-CH_2-$ | 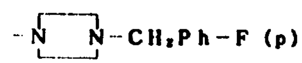 |
| 541 | Me | Me | Me | H | $-CH_2-$ | 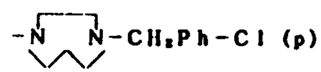 |
| 544 | iso-Pro | Me | Me | H | $-CH_2-$ | 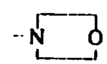 |

| | | | | | | |
|---|---|---|---|---|---|---|
| 546 | 〃 | Me | Me | H | $-CH_2-$ | 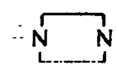 $-CH_2Ph-Cl$ (p) |
| 547 | 〃 | Me | Me | H | $-CH_2-$ | 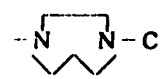 $-CH_2Ph-Cl$ (p) |
| 550 | 〃 | Et | Et | H | $-CH_2-$ | 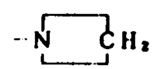 |
| 551 | 〃 | Et | Et | H | $-CH_2-$ | 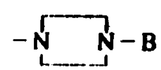 $-Bu$ |

| Compound No. | $R_1$ | $R_2$ | $R_3$ | $R_4$ | Q | R |
|---|---|---|---|---|---|---|
| 552 | iso-Pro | Et | Et | H | $-CH_2-$ | − 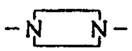 $-CH_2Ph$ |
| 553 | 〃 | Et | Et | H | $-CH_2-$ | − 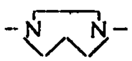 $-CH_2Ph-OMe$ (p) |
| 556 | iso-Bu | Et | Et | H | $-CH_2-$ | − 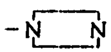 $-CH_2Ph$ |
| 558 | 〃 | Et | Et | H | $-CH_2-$ | − 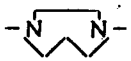 $-CH_2Ph-OMe$ (p) |
| 648 | Me | Me | Me | H | -CO- | $-NH-CH_2CH_2-Br$ |
| 649 | Me | Me | Me | H | -CO- | $-NH-CH_2CH_2-Cl$ |
| ~~650~~ | ~~Me~~ | ~~Me~~ | ~~Me~~ | ~~H~~ | ~~-CO-~~ | ~~$-NH-CH_2CH_2-C_6H_{11}$~~ |
| 651 | Et | Me | Me | Ph | -CO- | -OMe |
| 652 | Et | Me | Me | Ph | -CO- | -OH |
| 653 | Et | Me | Me | Ph | -CO- | $-NH-CH_2CH_2-Cl$ |
| 654 | $-CH_2Ph$ | Et | Et | H | -CO- | -OMe |
| 655 | 〃 | Et | Et | H | -CO- | -OH |
| 656 | 〃 | Et | Et | H | -CO- | $-NH-CH_2CH_2-Cl$ |
| 657 | 〃 | Et | Et | H | -CO- | $-NH-CH_2CH_2-$ 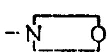 |
| 658 | 〃 | Et | Et | H | -CO- | $-NH-CH_2CH_2-$ 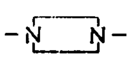 -Ph |
| 662 | Et | Me | Me | H | -CO- | -OMe |
| 663 | $-CH_2Ph$ | Me | Me | H | -CO- | -OMe |
| 664 | iso-Pro | Me | Me | H | -CO- | -OMe |
| 665 | 〃 | Et | Et | H | -CO- | -OMe |

| Compound No. | $R_1$ | $R_2$ | $R_3$ | $R_4$ | Q | R |
|---|---|---|---|---|---|---|
| 666 | iso-Pro | Et | Et | H | -CO- | -OMe |
| 679 | Me | Me | Me | H | $-CH_2-$ | -OH |
| 680 | Et | Me | Me | H | $-CH_2-$ | -OH |
| 681 | $-CH_2Ph$ | Me | Me | H | $-CH_2-$ | -OH |
| 682 | iso-Pro | Me | Me | H | $-CH_2-$ | -OH |
| 683 | 〃 | Et | Et | H | $-CH_2-$ | -OH |
| 684 | iso-Bu | Et | Et | H | $-CH_2-$ | -OH |
| 697 | iso-Pro | Me | Me | H | $-CH_2-$ | 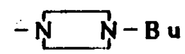 |
| 810 | Me | Me | Me | H | $-CH_2-$ | 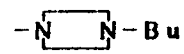 |
| 812 | Et | Me | Me | H | $-CH_2-$ | 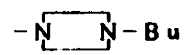 |
| 814 | iso-Pro | Me | Me | H | $-CH_2-$ | 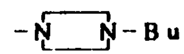 |
| 822 | Me | Me | Me | H | $-CH_2-$ | 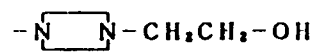 |
| 825 | iso-Pro | Me | Me | H | $-CH_2-$ | 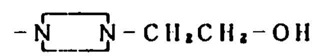 |
| 826 | iso-Pro | Et | Et | H | $-CH_2-$ | 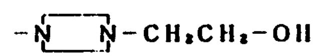 |
| 834 | iso-Pro | Me | Me | H | $-CH_2-$ | 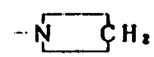 |
| 842 | Me | Me | Me | H | $-CH_2-$ | $-NEt_2$ |
| 844 | Et | Me | Me | H | $-CH_2-$ | $-NEt_2$ |
| 846 | iso-Pro | Me | Me | H | $-CH_2-$ | $-NEt_2$ |
| 848 | iso-Pro | Et | Et | H | $-CH_2-$ | $-NEt_2$ |

TABLE 2

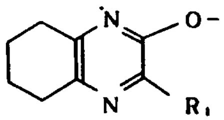O-CH₂-Q-R

Me ; methyl
Et ; ethyl
Pro ; propyl
Bu ; butyl
Ph ; benzene ring

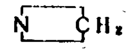 ; piperidine ring
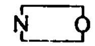 ; morpholine ring
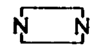 ; piperazine ring
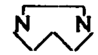 ; homopiperazine ring

( ) : position of substituent

| Compound No. | $R_1$ | Q | R |
|---|---|---|---|
| 368 | $-CH_2Ph$ | -CO- | $-NH-CH_2CH_2-$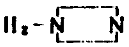$-CH_2Ph$ |
| 369 | 〃 | -CO- | $-NH-CH_2CH_2-$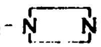$-CH_2Ph-OMe$ (p) |
| 370 | 〃 | -CO- | $-NH-CH_2CH_2-$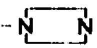$-CH_2Ph-Cl$ (p) |
| 371 | 〃 | -CO- | $-NH-CH_2CH_2-$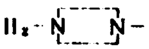$-Ph-Cl$ (p) |
| 372 | 〃 | -CO- | $-NH-CH_2CH_2-$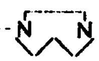$-CH_2Ph-F$ (p) |
| 373 | 〃 | -CO- | $-NH-CH_2CH_2-$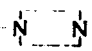$-Ph$ |
| 517 | Bu | $-CH_2-$ | -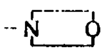 |
| 518 | Bu | $-CH_2-$ | -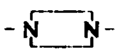$-CH_2Ph$ |
| 519 | Bu | $-CH_2-$ | -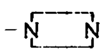$-CH_2Ph-Cl$ (p) |
| 520 | Bu | $-CH_2-$ | -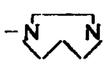$-CH_2Ph-Cl$ (p) |
| 521 | Bu | $-CH_2-$ | -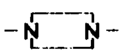$-Ph$ |
| 522 | Bu | $-CH_2-$ | -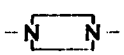$-Ph-OMe$ (o) |
| 559 | $-(CH_2)_4CH_3$ | $-CH_2-$ | -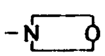 |

| Compound No. | $R_1$ | Q | R |
|---|---|---|---|
| 560 | $-(CH_2)_4CH_3$ | $-CH_2-$ | 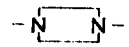 $-Bu$ |
| 561 | 〃 | $-CH_2-$ | 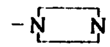 $-CH_2Ph$ |
| 562 | 〃 | $-CH_2-$ | 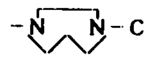 $-CH_2Ph-F$ (p) |
| 563 | 〃 | $-CH_2-$ | 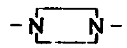 $-Ph-OMe$ (o) |
| 564 | $-(CH_2)_5CH_3$ | $-CH_2-$ | 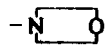 |
| 565 | 〃 | $-CH_2-$ | 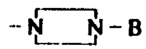 $-Bu$ |
| 567 | 〃 | $-CH_2-$ | 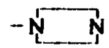 $-CH_2Ph$ |
| 568 | 〃 | $-CH_2-$ | 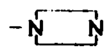 $-CH_2Ph-Cl$ (p) |
| 569 | 〃 | $-CH_2-$ | 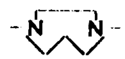 $-CH_2Ph-F$ (p) |
| 570 | 〃 | $-CH_2-$ | 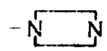 $-Ph-OMe$ (o) |
| 571 | $-(CH_2)_6CH_3$ | $-CH_2-$ | 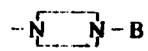 $-Bu$ |
| 572 | $-(CH_2)_7CH_3$ | $-CH_2-$ | 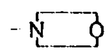 |
| 573 | 〃 | $-CH_2-$ | 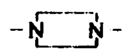 $-Bu$ |
| 574 | 〃 | $-CH_2-$ | 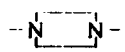 $-CH_2Ph$ |
| 575 | 〃 | $-CH_2-$ | 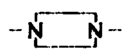 $-CH_2Ph-Cl$ (p) |
| 576 | 〃 | $-CH_2-$ | 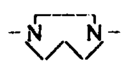 $-CH_2Ph-F$ (p) |

| Compound No. | $R_1$ | Q | R |
|---|---|---|---|
| 577 | $-(CH_2)_7CH_3$ | $-CH_2-$ | - 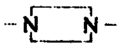 -Ph |
| 578 | 〃 | $-CH_2-$ | - 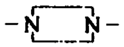 -Ph-OMe (p) |
| 579 | $-(CH_2)_8CH_3$ | $-CH_2-$ | - 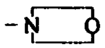 |
| 580 | 〃 | $-CH_2-$ | - 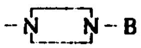 -Bu |
| 581 | 〃 | $-CH_2-$ | - 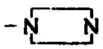 $-CH_2Ph$ |
| 582 | 〃 | $-CH_2-$ | - 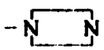 $-CH_2Ph-OMe$ (p) |
| 583 | 〃 | $-CH_2-$ | - 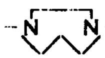 $-CH_2Ph-Cl$ (p) |
| 584 | 〃 | $-CH_2-$ | - 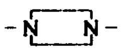 -Ph-Cl (p) |
| 585 | $-(CH_2)_9CH_3$ | $-CH_2-$ | - 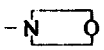 |
| 586 | 〃 | $-CH_2-$ | - 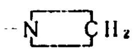 |
| 587 | 〃 | $-CH_2-$ | - 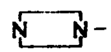 -Bu |
| 588 | 〃 | $-CH_2-$ | - 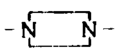 $-CH_2CH_2-OH$ |
| 589 | 〃 | $-CH_2-$ | - 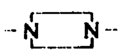 $-CH_2Ph$ |
| 590 | 〃 | $-CH_2-$ | - 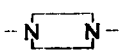 $-CH_2Ph-Cl$ (p) |
| 591 | 〃 | $-CH_2-$ | - 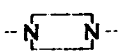 $-CHPh_2$ |
| 592 | 〃 | $-CH_2-$ | - 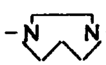 $-CH_2Ph-F$ (p) |

| Compound No. | | Q | R |
|---|---|---|---|
| 593 | $-(CH_2)_9CH_3$ | $-CH_2-$ | 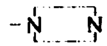 Ph-OMe (p) |
| 594 | $-(CH_2)_{11}CH_3$ | $-CH_2-$ | 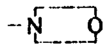 |
| 595 | 〃 | $-CH_2-$ | 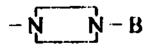-Bu |
| 596 | 〃 | $-CH_2-$ | 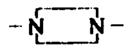$-CH_2Ph$ |
| 597 | 〃 | $-CH_2-$ | 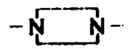$-CH_2Ph-OMe$ (p) |
| 598 | 〃 | $-CH_2-$ | 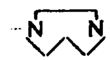$-CH_2Ph-F$ (p) |
| 599 | 〃 | $-CH_2-$ | 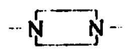-Ph-OMe (o) |
| 600 | $-(CH_2)_{13}CH_3$ | $-CH_2-$ | 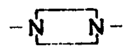-Bu |
| 601 | 〃 | $-CH_2-$ | 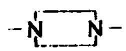$-CH_2Ph$ |
| 602 | 〃 | $-CH_2-$ | 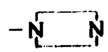$-CH_2Ph-F$ (p) |
| 603 | 〃 | $-CH_2-$ | 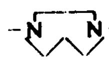$-CH_2Ph-Cl$ (p) |
| 604 | 〃 | $-CH_2-$ | 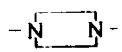$-CHPh_2$ |
| 605 | 〃 | $-CH_2-$ | 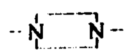-Ph-Cl (o) |
| 606 | $-(CH_2)_{15}CH_3$ | $-CH_2-$ | 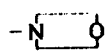 |
| 607 | 〃 | $-CH_2-$ | 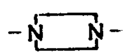-Bu |
| 608 | 〃 | $-CH_2-$ | 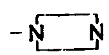$-CH_2Ph$ |

| Compound No. | $R_1$ | Q | R |
|---|---|---|---|
| 609 | $-(CH_2)_{15}CH_3$ | $-CH_2-$ | - 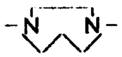 $-CH_2Ph-OMe$ (p) |
| 610 | 〃 | $-CH_2-$ | - 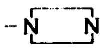 $-CH_2Ph-OMe$ (p) |
| 659 | $-CH_2Ph$ | $-CO-$ | $-OMe$ |
| 660 | 〃 | $-CO-$ | $-OH$ |
| 661 | 〃 | $-CO-$ | $-NH-CH_2CH_2-Cl$ |
| 667 | Bu | $-CO-$ | $-OMe$ |
| 668 | $-(CH_2)_4CH_3$ | $-CO-$ | $-OMe$ |
| 669 | $-(CH_2)_5CH_3$ | $-CO-$ | $-OMe$ |
| 670 | $-(CH_2)_6CH_3$ | $-CO-$ | $-OMe$ |
| 671 | $-(CH_2)_7CH_3$ | $-CO-$ | $-OMe$ |
| 672 | $-(CH_2)_8CH_3$ | $-CO-$ | $-OMe$ |
| 673 | $-(CH_2)_9CH_3$ | $-CO-$ | $-OMe$ |
| 674 | $-(CH_2)_{11}CH_3$ | $-CO-$ | $-OMe$ |
| 675 | $-(CH_2)_{13}CH_3$ | $-CO-$ | $-OMe$ |
| 676 | $-(CH_2)_{15}CH_3$ | $-CO-$ | $-OMe$ |
| 677 | Pro | $-CO-$ | $-OMe$ |
| 678 | sec-Bu | $-CO-$ | $-OMe$ |
| 685 | Bu | $-CH_2-$ | $-OH$ |
| 686 | $-(CH_2)_4CH_3$ | $-CH_2-$ | $-OH$ |
| 687 | $-(CH_2)_5CH_3$ | $-CH_2-$ | $-OH$ |

| Compound No. | $R_1$ | Q | R |
|---|---|---|---|
| 688 | $-(CH_2)_6CH_3$ | $-CH_2-$ | $-OH$ |
| 689 | $-(CH_2)_7CH_3$ | $-CH_2-$ | $-OH$ |
| 690 | $-(CH_2)_8CH_3$ | $-CH_2-$ | $-OH$ |
| 691 | $-(CH_2)_9CH_3$ | $-CH_2-$ | $-OH$ |
| 692 | $-(CH_2)_{11}CH_3$ | $-CH_2-$ | $-OH$ |
| 693 | $-(CH_2)_{13}CH_3$ | $-CH_2-$ | $-OH$ |
| 694 | $-(CH_2)_{15}CH_3$ | $-CH_2-$ | $-OH$ |
| 695 | Pro | $-CH_2-$ | $-OH$ |
| 696 | sec-Bu | $-CH_2-$ | $-OH$ |
| 817 | Bu | $-CH_2-$ | 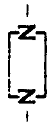 -Bu |
| 830 | $-(CH_2)_7CH_3$ | $-CH_2-$ | 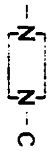 $-CH_2CH_2-OH$ |
| 837 | Bu | $-CH_2-$ | 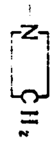 |
| 839 | $-(CH_2)_9CH_3$ | $-CH_2-$ | 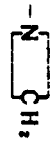 |
| 850 | Bu | $-CH_2-$ | $-NEt_2$ |
| 851 | $-(CH_2)_7CH_3$ | $-CH_2-$ | $-NEt_2$ |

The pharmacological activity of the compounds of formula (1) is illustrated below. All the compounds of formula (1) used in the pharmacological tests were in the form of the hydrochloride salt thereof.

1. Platelet aggregation inhibition:

A sample solution containing a compound of formula (1) (final concentration 500 or 100μM) is added to rabbit platelet plasma to which was added 10% by volume of a 3.8% sodium citrate solution. The mixture is incubated at 37°C for 3 minutes. A platelet activation factor (PAF, final concentration 10-50μg/ml) or collagen (final concentration 2.5μg/ml) is added thereto as an aggregating agent, and platelet aggregation activity is measured with an aggrigometer. The results of assays for PAF-induced aggregation are shown in Table 3, and those for collageninduced aggregation are shown in Table 4. These tables show the strong platelet aggregation inhibitory activity of the compounds of formula (1) of the present invention.

Table 3 Platelet aggregation inhibitory action on PAF induced aggregation

| Compound No. | concentration (μM) | inhibition ratio (%) |
|---|---|---|
| 560 | 100 | 97 |
| 561 | 100 | 56 |

| | | |
|---|---|---|
| 562 | 100 | 45 |
| 565 | 100 | 94 |
| 567 | 100 | 58 |
| 570 | 100 | 40 |
| 571 | 100 | 88 |
| 573 | 100 | 86 |
| 580 | 100 | 94 |
| 587 | 100 | 91 |
| 588 | 100 | 64 |
| 595 | 100 | 85 |
| 600 | 100 | 52 |
| 067 | 300 | 53 |
| 070 | 500 | 48 |
| 135 | 500 | 77 |
| 136 | 100 | 50 |
| 137 | 100 | 76 |
| 341 | 100 | 42 |
| 342 | 100 | 43 |
| 344 | 100 | 44 |
| 347 | 100 | 59 |
| 348 | 100 | 70 |
| 502 | 100 | 81 |
| 503 | 100 | 42 |
| 504 | 100 | 60 |
| 541 | 100 | 44 |
| 550 | 100 | 56 |

| | | |
|---|---|---|
| 551 | 100 | 97 |
| 552 | 100 | 78 |
| 553 | 100 | 82 |
| 556 | 100 | 51 |

Table 4

| Platelet aggregation inhibitory action on collagen induced aggregation | | |
|---|---|---|
| compound No. | concentration ( μ M ) | inhibition ratio ( % ) |
| 573 | 100 | 58 |
| 580 | 100 | 80 |
| 582 | 100 | 58 |
| 585 | 100 | 41 |
| 587 | 100 | 79 |
| 588 | 100 | 78 |
| 547 | 100 | 56 |
| 550 | 100 | 85 |
| 556 | 100 | 40 |

2. Vasodilation activity:

A dog, pretreated with morphine (1.5 mg/kg, sc) is anesthesized with urethane (450 mg/kg, iv) and α-chloralose (45 mg/kg, iv), and immobilized in the dosal positions. Right femoral arterial blood is introduced into a left femoral artery via a perfusion pump, and Sterling's resistance is connected to the exosomatic circulatory system to perfuse blood to a left back limb at constant pressure. The perfusion pressure is set with a valve at slightly higher than that of the average blood pressure of the animal. A sample (100 μg) dissolved in physiological saline solution is administered to a right femoral artery, and changes in blood flow are measured. Vasodilation activity is measured as a relative activity, by defining as 100% the increased rate of blood flow when 30 μg papaverin is administered intra-arterially. The result are shown in Table 5, where it will be seen that the compounds of formula (1) of the present invention have strong vasodilation activity.

Table 5

| Vasodilation activity | |
|---|---|
| compound No. | vasodilation activity ( % ) |
| 075 | 89 |
| 135 | 242 |
| 136 | 204 |
| 137 | 216 |

3. Antioxidant activity:

Antioxidant activity is determined according to the method of Stocks et al. [Clin. Sci. Mol. Med., 47: 215

(1974)]. Rat cerebrum is added to ice-cooled 40 mM phosphate saline buffer solution (PBS)(pH 7.4, 4 ml buffer per 1 g cerebrum ), homogenized and centrifuged (1000 x g, 4° C, 10 min.) to obtain a supernatant solution. The supernatant solution is diluted five fold with the above ice-cooled PBS solution, and to a 0.9 ml aliquot thereof is added a sample containing a compound of formula (1) (0.1 ml, final concentration 100μM) dissolved in ethanol. The resultant mixture is incubated at 37° C for 15 minutes, 35% perchloric acid (0.2ml) is added, and the mixture is ice-cooled to stop the reaction and centrifuged (1300 x g, 4° C, 10 min.). 0.5 ml thiobarbituric acid (5g/l of 50% acetic acid) is added to the supernatant solution (1 ml), so as to measure its absorbancy at 532 nm. The amount of lipoperoxide thus generated is expressed as an amount of malondialdehyde. The results are shown in Table 6, where it will be seen that the compounds of formula (1) of the present invention inhibit lipoperoxide generation.

**Table 6** Antoxidant activity

| compound No. | inhibition ratio ( % ) |
|---|---|
| 371 | 49 |
| 372 | 64 |
| 373 | 49 |
| 520 | 83 |
| 562 | 65 |
| 568 | 61 |
| 569 | 81 |

| | |
|---|---|
| 570 | 65 |
| 573 | 56 |
| 574 | 71 |
| 575 | 79 |
| 576 | 85 |
| 577 | 71 |
| 578 | 88 |
| 580 | 77 |
| 581 | 71 |
| 582 | 82 |
| 583 | 82 |
| 584 | 65 |
| 585 | 62 |
| 586 | 68 |
| 587 | 65 |
| 588 | 65 |
| 589 | 74 |
| 590 | 74 |
| 591 | 59 |
| 592 | 85 |
| 593 | 73 |
| 594 | 58 |
| 595 | 70 |
| 596 | 73 |
| 597 | 76 |
| 598 | 88 |

| | |
|---|---|
| 599 | 70 |
| 600 | 61 |
| 601 | 67 |
| 602 | 64 |
| 603 | 76 |
| 607 | 52 |
| 608 | 55 |
| 609 | 61 |
| 344 | 46 |
| 345 | 54 |
| 347 | 51 |
| 348 | 62 |
| 379 | 50 |
| 382 | 83 |
| 502 | 69 |
| 516 | 75 |

As explained hereinabove, a compound of formula (1) of the present invention or a salt thereof inhibits platelet aggregation, has vasodilating activity, and/or inhibits lipoperoxide generation, and is useful in pharmaceutical form for treating circulatory and metabolic disorders.

## EXAMPLES

The following Examples further illustrate the present invention.

In the Examples, the Rf value of silica-gel thin layer chromatography (TLC) is either specified by or measured using the following carrier and developing solvent:

Carrier: silica-gel, Kieselgel 60 $F_{254}$ Art 5715 (Merck)

Developer: chloroform-methanol (20:1)

Physical properties (NMR, Mass, CI, Rf on TLC) of the compounds of formula (1) obtained in the following Examples are shown in Tables 25-27.

Symbols for chemical structure in Tables 7-19 have the same meanings as in Table 1, and the symbols in Tables 20-24 have the same meanings as in Table 2.

Example 1

Methyl (3,5,6-trimethylpyrazine-2-yl oxy) acetate (compound 034):

Metallic sodium (1.29g, 56mM) was dissolved in anhydrous methanol (120 ml). 2-hydroxy-3, 5, 6-trimethylpyrazine (7.74g, 56 mM) was dissolved therein, and methanol was distilled off in vacuo to obtain sodium, 3, 5, 6-trimethyl-2-pyrazinolate which was suspended in dimethylformamide (150 ml). Methyl

chloroacetate (10.85g, 0.10M) was added thereto and stirred at 95-100 °C for 3 hours. Solvent was removed in vacuo. Dilute aqueous sodium carbonate was added to the residue, which was then extracted twice with chloroform (200 ml), and the extract was dried with anhydrous magnesium sulfate and concentrated in vacuo. The residue was charged on a column of silica-gel (Wako Pure Chem. Co., C-200, 170 g) and eluted with benzene and benzene-ethyl acetate (10 : 1) successively to obtain the product. (8.23 g, Yield: 70.0%)

Example 2
Methyl α-(3, 5, 6-trimethylpyrazine-2-yl oxy)-propionate (compound 035):

Methyl α-chloropropionate (12.25 g, 0.10 mM) has added to sodium 3, 5, 6-trimethyl-2-pyrazinolate (8.00 g, 50 mM) suspended in dimethylformamide (100 ml) and stirred at 95 100 °C for 7 hours. Solvent was removed in vacuo. Dilute aqueous sodium carbonate has added to the residue, which was then extracted once with chloroform (200 ml) and twice with chloroform (100 ml), and the extract was dried with anhydrous magnesium sulfate and concentrated in Vacuo. The residue was charged on a column of silica-gel (Wako Pure Chem. Co., C-200, 160 g) and eluted with benzene and benzene-ethyl acetate (10 : 1) successively to obtain the product. (8.39 g, Yield: 74.9%)

Example 3
Methyl α-(3, 5, 6-trimethylpyrazine-2-yl oxy)-phenyl acetate (compound 036):

Methyl α-bromophenyl acetate (23.0 g, 0.10 nM) was added to sodium 3,5,6-trimethyl-2-pyrazinolate (8.00 g, 50 mM) suspended in dimethylformamide (150 ml) and stirred at 100 °C for 5 hours. The reaction mixture was treated in the same way as in Example 2 to obtain the product. (10.97g, Yield: 76.7%)

Example 4
(3,5,6-trimethylpyrazine-2-yl oxy)-acetate (compound 037):

Aqueous 1N-NaOH (80 ml) was added to compound 034 (8.40 g, 40 mM) and stirred for 30 minutes. 1N-HCl (80 ml) was added to the reaction mixture and the precipitated crystals were collected by filtration to obtain the product. (3.15 g) Filtrate was dried in vacuo and the residue was extracted with hot ethanol, then the extract was dried in vacuo to obtain the product. (4.09 g) (Total: 7.24 g Yield: 92.3%)

Example 5
α-(3,5,6-trimethylpyrazine-2-yl oxy)-propionate (compound 038):

Aqueous 1N-NaOH (80 ml) was added to compound 035 (7.81 g, 35 mM) and stirred for 1 hour. The reaction mixture was extracted with chloroform to remove unreacted compound. 1N-HCl (70 ml) was added to an aqueous layer and concentrated in vacuo to precipitate crystals, which were collected by filtration, washed with water completely and dried to obtain the product. (6.66 g, Yield: 90.6%)

Example 6
α-(3, 5, 6-trimethylpyrazine-2-yl oxy)-phenyl acetate (compound 039):

Aqueous 1N-NaOH (75 ml) was added to a compound 036 (10.80 g, 37.7 mM) dissolved in methanol (75 ml) and stirred for 1.5 hour. Methanol was distilled off in vacuo and the aqueous layer was washed with chloroform. 1N-HCl (75 ml) was added to the aqueous layer which was extracted with chloroform. The extract was dried with anhydrous magnesium sulfate and concentrated in vacuo to obtain the product as white crystals. (9.89 g, Yield: 96.4%)

Examples 7-12:
2-(substituted carbonylmethoxy)-3,5,6-trimethylpyrazine:

Triethylamine (0.70 ml, 5 mM) was added to compound 037 (0.98 g, 5 mM) dissolved in tetrahydrofuran. Pivaloyl chloride (0.16 g, 5 mM) was added dropwise at -5 °C and stirred for 30 minutes under ice-cooling. A tetrahydrofuran solution of a base (5 mM) was added dropwise to the reaction mixture and stirred at room temperature for 4 hours. The reaction mixture was concentrated in vacuo and the residue was dried with chloroform, then concentrated in vacuo. The residue was charged on a column of silica-gel (C-20, 60 g) and eluted with chloroform and chloroform-methanol (200 : 1) successively to obtain

the compounds in Table 7.

Table 7 identifies the product, yields in weight and percentage, the base and its amount used.

Example 13
N-(2-bromoethyl)-(3,5,6-trimethylpyrazine-2-yl oxy)- acetamide (compound 648):

Triethylamine (7.0 ml, 50 mM) was added to compound 037 (9.80 g, 50 mM) dissolved in tetrahydrofuran (100 ml) and Chloroform (100 ml) in solution with bromoethylamine hydrobromide (10.30 g, 50 mM) and triethylamine (7.7 ml, 55 mM) was added dropwise at -5° C, then stirred for 4 hours under ice-cooling. The reaction mixture was concentrated in vacuo, the residue was dissolved in chloroform and washed with dilute aqueous potassium carbonate. The aqueous layer was extracted with chloroform. The organic layer was combined, dried with anhydrous magnesium sulfate, and concentrated in vacuo. The residue was charged on a column of silica-gel (chlodil, 250 g), and eluted successively with chloroform and chloroform-methanol (500:1) to obtain the product (6.92 g)

Example 14
N-(2-chlorethyl)-(3,5,6-trimethylpyrazine-2-yl oxy)-acetamide (compound 649):

Triethylamine (3.50 ml, 25 mM) was added to compound 037 (4.90 g, 25 mM) dissolved in tetrahydrofuran (50 ml) and pivaloylchloride (3.05 g, 25 mM) was added dropwise at -5° C, then stirred for 30 minutes. Chloroform (50 ml) in solution with 2-chloroethylamine hydrochloride (2.90 g, 25 mM) and triethylamine (3.9 ml, 28 mM) was added dropwise thereto, then stirred for 4 hours with the reaction mixture slowly reaching room temperature. The reaction mixture was concentrated in vacuo, the residue was dissolved in chloroform and washed with dilute aqueous potassium carbonate. The aqueous layer was extracted with chloroform. The organic layer was combined, dried with anhydrous magnesium sulfate, and concentrated in vacuo. The residue was charged on a column of silica-gel (C-200, 40 g), and eluted successively with chloroform and chloroform-methanol (20:1) to obtain the product (5.66 g, Yield: 7.5%)

Examples 15-20
N-(2-substituted ethyl)-(3,5,6-trimethylpyrazine-2-yl oxy)-acetamide:

Benzene (20 ml) in solution with compound 648 (0.91 g, 3.0 mM), triethylamine (0.84 ml) and a base was refluxed. The reaction mixture was washed with dilute aqueous potassium carbonate, and the aqueous layer was extracted with chloroform. Organic layers (benzene and chloroform) were combined, dried with anhydrous magnesium sulfate, and concentrated in vacuo. The residue was charged on a column of silica-gel (C-200, 65 g) and eluted successively with chloroform and chloroform-methanol to obtain the compounds in Table 8.

Table 8 shows the reflux time, yield in weight and percentage, the base and its amount used and the ratio of chloroform-methanol mixture used in each of the above examples.

Example 21
N-(2-phenylthioethyl)-(3,5,6-trimethylpyrazine-2-yl oxy)-acetamide (compound 072):

Thiophenol (0.60 g, 5.4 mM) and potassium carbonate (0.75 g, 5.4 mM) were added to compound 648 (0.91 g, 3.0 mM) dissolved in dimethylformamide (10 ml) and stirred for 2 hours at room temperature. The reaction mixture was concentrated in vacuo, the residue was dissolved in chloroform and washed with dilute aqueous potassium carbonate. The aqueous layer was further extracted with chloroform. The combined organic layer was chromatographed by eluting successively with benzene and benzene-ethyl acetate (10:1) to obtain the product (0.35 g, Yield: 35.2%)

Examples 22-24
N-(2-substituted ethyl)-(3,5,6-trimethylpyrazine-2-yl oxy)-acetamide:

Benzene (25 ml) in solution with compound 649 (0.98 g, 4 mM), triethylamine (1.14 ml) and base was refluxed. The reaction mixture was washed with dilute aqueous potassium carbonate, and the aqueous layer was further extracted with chloroform. Organic layers (benzene and chloroform) were combined, dried with anhydrous magnesium sulfate, and concentrated in vacuo. The residue was charged on a column of silica-gel (C-200, 80 g) eluted successively with chloroform and chloroform-methanol to obtain the compounds in

Table 9.

Table 9 shows the base and its amount used, the reflux time, the ratio of chloroform-methanol mixture used, the products and the yield in weight and percentage, in each of the above examples.

Example 25

Methyl α-(3-ethyl-5,6-dimethylpyrazine-2-yl oxy)-phenyl acetate (compound 651):

A solution (12.5 ml) of 4N-$CH_3ONa$/methanol was added to a solution of 2-hydroxy-3-ethyl-5,6-dimethylpyrazine (7.60 g, 50 mM) in anhydrous methanol (100 ml), and methanol was distilled off in vacuo. The residue was dissolved in dimethylformamide (150 ml). Methyl α-bromophenyl acetate (11.5 g, 50 mM) was added thereto, and stirred at 100 °C for 5 hours. The reaction mixture was concentrated in vacuo. The residue was dissolved in chloroform, and washed with dilute aqueous potassium carbonate. The aqueous layer was further extracted twice with chloroform. The organic layer was combined, dried with anhydrous magnesium sulfate, and concentrated in vacuo. The residue was charged on a column of silica-gel (C-200, 170 g) and eluted successively with benzene and benzene-ethyl acetate (50:1) to obtain the product. (10.42 g, Yield: 69.5%).

Example 26

α-(3-ethyl-5,6-dimethylpyrazine-2-yl oxy)-phenyl acetate (compound 652):

Aqueous 2N-NaOH (25.7 ml) was added to compound 651 (7.72 g, 25.7 mM) dissolved in methanol (30 ml) and stirred for 4.5 hours at room temperature. The reaction mixture, to which 2N-HCl (25.7 ml) was added, was ice-cooled to precipitate the crystals. The thus precipitated crystals were collected by filtration, washed with water and dried to obtain the product. (6.91 g, Yield: 94.0%)

Example 27

N-(2-chlorethyl)-α-(3-ethyl-5,6-dimethylpyrazine-2-yl oxy)-phenyl acetamide (compound 653):

Triethylamine (3.7 ml, 26.6 mM) was added to compound 652 (7.50 g, 26.2 mM) dissolved in tetrahydrofuran (44 ml) and pivaloylchloride (3.21 g, 26.3 mM) was added dropwise at -5 °C, then stirred for 30 minutes. Chloroform (26 ml) in solution with 2-chloroethylamine hydrochloride (3.05 g, 26.3 mM) and triethylamine (3.7 ml) was added dropwise thereto under cooling at 0~-5 °C, then stirred for 1 hour under ice-cooling and for 4 hours at room temperature. Chloroform was added to the reaction mixture which was washed with dilute aqueous potassium carbonate. The aqueous layer was further extracted twice with chloroform. The organic layer was combined, dried with anhydrous magnesium sulfate, and concentrated in vacuo. The residue was charged on a column of silica-gel (c-200, 170 g), and eluted successively with benzene, benzene-ethyl acetate (40:1) and benzene-ethyl acetate (10:1) to obtain the product (6.46 g, Yield: 70.4%)

Examples 28-35

N-(2-substituted ethyl)-α-(3-ethyl-5,6-dimethylpyrazine-2-yl oxy)-phenyl acetamide:

Base (8.0 mM) was added to benzene (30 ml) in solution with compound 653 (1.39 g, 4.0 mM) and triethylamine (1.12 ml) and refluxed overnight. The reaction mixture was washed with dilute aqueous potassium carbonate, and the aqueous layer was extracted three times with chloroform. Organic layers (benzene and chloroform) were combined, dried with anhydrous magnesium sulfate, and concentrated in vacuo. The residue was charged on a column of silica-gel (C-200, 65 g) and eluted successively with chloroform and chloroform-methanol (100 : 1) to obtain the compounds in Table 10.

Table 10 shows the base and its amount used, the compounds and their yield in weight and percentage in each of the above examples.

Example 36

Methyl (3-benzyl-5,6-diethylpyrazine-2-yl oxy)-acetate (compound 654):

A solution of 4N-$CH_3ONa$/methanol (10.0 ml, 40mM) was added to a solution of 2-hydroxy-3-benzyl-5,6-diethylpyrazine (9.68 g, 40 mM) in anhydrous methanol (120 ml), and methanol was distilled off in vacuo. The residue was dissolved in dimethylformamide (150 ml). Methyl chloroacetate (4.34 g, 40 mM) was added thereto, and stirred at 100 °C for 5 hours. The reaction mixture was concentrated in vacuo, dilute aqueous

potassium carbonate was added to the residue and the aqueous layer was extracted three times with chloroform. The extract was dried with anhydrous magnesium sulfate, and concentrated in vacuo. The residue was charged on a column of silica-gel (C-200, 175 g) and eluted successively with benzene and benzene-ethyl acetate (100 : 1) to obtain the product. (11.41 g, Yield: 90.8%)

Example 37
(3-benzyl-5,6-diethylpyrazine-2-yl oxy)-acetate (Compound 655):

Aqueous 2N-NaOH (34 ml) was added to compound 654 (10.68 g, 34.0 mM) dissolved in methanol (34 ml) and stirred for 3 hours. 2N-HCl (34 ml) was added to the reaction mixture and the precipitated crystals were collected by filtration, washed completely with water and dried to obtain the product. (8.86 g, Yield: 86.9%)

Example 38
N-(2-chloroethyl)-(3-benzyl-5,6-diethylpyrazine-2-yl oxy)-acetamide (compound 656)

Pivaloylchloride (3.66 g, 30 mM) was added dropwise at -5 °C to compound 655 (8.62 g, 28.7 mM) and triethylamine (4.2 ml, 30 mM) dissolved in tetrahydrofuran (50 ml), then stirred for 30 minutes. Chloroform (30 ml) in solution with 2-fluoroethylamine hydrochloride (3.48 g) and triethylamine (4.2 ml) was added dropwise thereto under cooling at 0~-5 °C then stirred for 1 hour under ice-cooling and 4 hours at room temperature. Dilute aqueous potassium carbonate was added to the reaction mixture and extracted three times with chloroform. The extract was dried with anhydrous magnesium sulfate, and concentrated in vacuo. The residue was charged on a column of silica-gel (C-200, 175 g) and eluted successively with benzene and benzene-ethyl acetate (5 : 1) to obtain the product. (9.21 g, Yield: 88.8%)

Examples 39-45
N-(2-substituted ethyl)-(3-benzyl-5,6-dimethylpyrazine-2-yl oxy)-acetamide

Benzene (30 ml) in solution with compound 656 (1.27 g, 3.5 mM), triethylamine (0.98 ml, 7 mM) and a base (7 mM) was refluxed. Dilute aqueous potassium carbonate was added to the reaction mixture and extracted three times with chloroform. The organic layer was dried with anhydrous magnesium sulfate and concentrated in vacuo. The residue was charged on a column of silica-gel (C-200, 80 g) and eluted successively with chloroform and chloroform-methanol (100 : 1) to obtain the compounds in Table 11.

Table 11 shows the base and its amount used, the reflux time and the product and yield in weight and percentage in each of the above examples.

Examples 46-50
Methyl (3,5,6-trialkylpyrazine-2-yl oxy) acetate:

Metallic sodium (1.15 g) was dissolved in anhydrous methanol (100 ml). The starting material, 2-hydroxy-3,5,6-trialkylpyrazine, (50 mM) was dissolved therein, and methanol was distilled off in vacuo to obtain the sodium salt thereof. Dimethylformamide (100 ml) and methyl chloroacetate (5.43 g, 50 mM) were added thereto, then extracted three times with chloroform. The extract was dried with anhydrous magnesium sulfate and concentrated in vacuo. The residue was charged on a column of silica-gel (C-200) and eluted successively with benzene and benzeneethyl acetate (20 : 1) to obtain the compounds in Table 12.

Table 12 shows the starting compound and its amount used, the reaction time, amount of silica-gel used and the product and yield in weight and percentage in each of the above examples.

Examples 51-56
2-(2-hydroxyethoxy)-3,5,6-trialkylpyrazine:

The starting compound was dissolved in methanol. 6-8 equivalents of $NaBH_4$ were added several portionwise under reflux. The reaction mixture was concentrated in vacuo, water was added thereto, then extracted three times with chloroform. The extract was dried with anhydrous magnesium sulfate and concentrated in vacuo. The residue was charged on a column of silica-gel (C-200) and eluted successively with benzene and benzene-ethyl acetate (10 : 1) to obtain the compounds in Table 13.

Table 13 shows the starting compound and its amount used, amount of $NaBH_4$ used, reflux time, amount of silica-gel used and the product and yield in weight and percentage in each of the above

examples.

Examples 57-65
2-(2-substituted ethoxy)-3,5,6-trimethylpyrazine:

Thionylchloride (0.56 ml, 1.3 equivalent) was added dropwise under ice-cooling to compound 679 (1.10 g, 6.0 mM) dissolved in chloroform and stirred at room temperature for 2.5 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.68 ml, 12 mM) and a base (12 mM) were added to the residue, and the mixture was refluxed. A dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted successively with chloroform and chloroform-methanol (200 : 1) to obtain the compounds in Table 14.

Table 14 shows the base and its amount used, the reflux time, and the product and yield in weight and percentage in each of the above examples.

Examples 66-71
2-(2-substituted ethoxy)-3-isopropyl-5,6-diethylpyrazine:

Thionylchloride (0.47 ml, 1.3 equivalent) was added dropwise under ice-cooling to compound 683 (1.19 g, 5.0 mM) dissolved in chloroform (5 ml) and stirred at room temperature for 4 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.40 ml, 10 mM) and a base (10 mM) were added to the residue, and the mixture was refluxed. Dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted successively with chloroform and chloroform-methanol (200 : 1) to obtain the compounds in Table 15.

Table 15 shows the base and its amount used, the reflux time, and the product and yield in weight and percentage in each of the above examples.

Examples 72-87
2-(2-substituted ethoxy)-3-ethyl-5,6,-dimethylpyrazine:

Thionylchloride (0.43 ml, 1.3 equivalent) was added dropwise under ice-cooling to compound 680 (0.98 g, 5.0 mM) dissolved in chloroform (5 ml) and stirred at room temperature for 3 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.40 ml, 10 mM) and a base (10 mM) were added to the residue, and the mixture was refluxed. Dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted successively with chloroform and chloroform-methanol to obtain the compounds in Table 16.

Table 16 shows the base and its amount used, the reflux time, ratio of chloroform-methanol in column chromatography, and the product and yield in weight and percentage in each of the above examples.

Examples 88-90
2-(2-substituted ethoxy)-3-benzyl-5,6-dimethylpyrazine:

Thionylchloride (0.36 ml, 1.3 equivalent) was added dropwise under ice-cooling to compound 681 (1.04 g, 4.0 mM) dissolved in chloroform (5 ml) and stirred at room temperature for 3 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.40 ml, 10 mM) and a base (8.0 mM) were added to the residue, and the mixture was refluxed. Dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted

successively with chloroform and chloroform-methanol to obtain the compounds in Table 17.

Table 17 shows the base and its amount used, the reflux time, ratio of chloroform-methanol in column chromatography, and the product and yield in weight and percentage in each of the above examples.

Examples 91-98

2-(2-substituted ethoxy)-3-isopropyl-5,6-dimethylpyrazine:

Thionylchloride (0.47 ml, 1.3 equivalent) was added dropwise under ice-cooling to compound 682 (1.05 g, 4.0 mM) dissolved in chloroform (5 ml) and stirred at room temperature for 3.5 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.40 ml, 10 mM) and a base (10 mM) were added to the residue, and the mixture was refluxed. Dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted successively with chloroform and chloroform-methanol to obtain the compounds in Table 18.

Table 18 shows the base and its amount used, the reflux time, ratio of chloroform-methanol in column chromatography, and the product and yield in weight and percentage in each of the above examples.

Examples 99-100

2-(2-substituted ethoxy)-3-isobutyl-5,6-diethylpyrazine:

Thionylchloride (0.47 ml, 1.3 equivalent) was added dropwise under ice-cooling to compound 684 (1.26 g, 5.0 mM) dissolved in chloroform (5 ml) and stirred at room temperature for 4 hours. Dilute aqueous potassium carbonate was added to the reaction mixture and extracted three times with chloroform. The extract, which was dried with anhydrous sodium sulfate, was concentrated in vacuo. Benzene (30 ml), triethylamine (1.40 ml, 10 mM) and a base (10 mM) were added to the residue, and the mixture was refluxed. Dilute aqueous potassium carbonate was added to the reaction mixture, and the mixture was extracted three times with chloroform. The extract was dried with anhydrous magnesium sulfate and concentrated in vacuo. The residue was charged on a column of silica-gel (C-200, 75 g) and eluted successively with chloroform and chloroform-methanol to obtain the compounds in Table 19.

Table 19 shows the base and its amount used, the reflux time, and the product and yield in weight and percentage in each of the above examples.

Comparative Example 1

2-hydroxy-3-benzyl-5,6,7,8-tetrahydroquinoxaline:

A methanol (30 ml) solution of cyclohexane-1,2-dione (13.44 g, 0.12 M) was added to phenylalanineamide hydrochloride (20.05 g, 0.1 M) dissolved in methanol (200 ml) under cooling to below -30 °C, and aqueous 12.5 N-NaOH (20 ml) was added dropwise thereto. The reaction mixture was stirred at below -30 °C for 30 minutes, and further stirred at room temperature for 3 hours. Conc. hydrochloric acid (25 ml) was added to the reaction mixture, and sodium bicarbonate (15 g) was added after 10 minutes stirring, the solvent then being distilled off in vacuo. The residue, to which was added water, was extracted three times with chloroform, and the extract was dried with anhydrous magnesium sulfate and concentrated in vacuo. The residue was recrystallized from acetone to obtain the product. (19.7 g. Yield: 82.1%)

Comparative Example 2

2-hydroxy-3-(2-phenylethyl)-5,6,7,8-tetrahydroquinoxaline:

A methanol (20 ml) solution of cyclohexane-1,2-dione (5.38 g) was added to α-amino-2-phenylacetic acidamide hydrochloride (8.58 g, 40 mM) dissolved in methanol (100 ml) under cooling to below -30 °C, and aqueous 12.5 N-NaOH was added dropwise thereto. The reaction mixture was stirred at below -30 °C for 30 minutes, and the mixture was then stirred at room temperature for 6 hours. Conc. hydrochloric acid (8 ml) was added to the reaction mixture, and sodium bicarbonate (6.0 g) was added after 10 minutes stirring, the solvent then being distilled off in vacuo. The residue, to which was added water, was extracted three times with chloroform, dried with anhydrous magnesium sulfate and concentrated in vacuo. The residue was recrystallised from acetone to obtain the product. (6.79 g. Yield: 66.8%)

Comparative Example 3
2-hydroxy-3-methyl-5,6,7,8-tetrahydroquinoxaline:

A methanol (20 ml) solution of cyclohexane-1,2-dione (6.72 g) was added to alanine (4.4 g 50 mM) dissolved in methanol (100 ml) under cooling to below -30 °C, and aqueous 12.5 N-NaOH (5 ml) was added dropwise thereto. The reaction mixture was stirred at below -30 °C for 30 minutes, and the mixture was then stirred at room temperature for 3 hours. Conc. hydrochloric acid (8 ml) was added to the reaction mixture, and sodium bicarbonate (5 g) was added after 10 minutes stirring, the solvent then being distilled off in vacuo. The residue, to which was added water, was extracted three times with chloroform, dried with anhydrous magnesium sulfate and concentrated in vacuo. The residue was recrystallized from acetone to obtain the product as pale yellow crystals. (6.50 g, Yield: 79.0%)

Comparative Example 4
2-hydroxy-3-ethyl-5,6,7,8-tetrahydroquinoxaline:

A methanol (20 ml) solution of cyclohexane-1,2-dione (6.72 g, 60 mM) was added to a methanol solution (100 ml) of α-aminobutylamide (5.1 g, 50 mM) under cooling to below -30 °C, and aqueous 12.5 N-NaOH (5 ml) was added dropwise thereto. The reaction mixture was stirred at below -30 °C for 30 minutes and the mixture was then stirred at room temperature for 3 hours. Conc. hydrochloric acid (6.25 ml) was added to the reaction and sodium bicarbonate (5 g) was added after 10 minutes stirring, the solvent being distilled off in vacuo. The residue, to which was added water, was extracted three times with chloroform, dried with anhydrous magnesium sulfate and concentrated in vacuo. The residue was recrystallized from acetone to obtain colorless crystals. (5.30 g, Yield: 60.0%)

Comparative Examples 5-18:
2-hydroxy-3-alkyl-5,6,7,8-tetrahydroquinoxaline:

Cyclohexane-1,2-dione (6.72 g 60 mM) was added all at once to amino acidamide hydrochloride (50 mM) dissolved in methanol (100 ml) under cooling to below -30 °C, and aqueous 12 N-NaOH (5 ml) was added dropwise thereto. The reaction mixture was stirred for 30 minutes under cooling to below -30 °C, and the mixture was then stirred at room temperature for 5 hours. Conc. hydrochloric acid (12.5 ml) was added to the reaction mixture and sodium bicarbonate (7.5 g) was added after 10 minutes, whereafter the solvent was distilled off in vacuo. The residue, to which was added water, was extracted three times with chloroform, dried with anhydrous sodium sulfate and concentrated in vacuo. The residue was recrystallized from acetone to obtain the product shown in Table 20.

Table 20 shows the acid amide hydrochloride and its amount used and the product and yield in weight and percentage in each of the above comparative examples.

Example 101
Methyl (3-benzyl-5,6,7,8-tetrahydroquinoxaline-2-yl oxy)-acetate (compound 656):

2-hydroxy-3-benzyl-5,6,7,8-tetrahydroquinoxaline (12.0 g, 50 mM) was dissolved in a solution of metallic sodium (1.15 g, 50 mM) in anhydrous methanol (200 ml), whereafter methanol was distilled off in vacuo to obtain the sodium salt thereof, which was suspended in dimethylformamide (100 ml). Methyl chloroacetate (5.43 g, 50 mM) was added thereto and stirred at 100 °C for 3 hours. The reaction mixture was concentrated in vacuo. Dilute aqueous sodium carbonate was added to the residue, which was then extracted three times with chloroform, and the extract was dried with anhydrous sodium sulfate and concentrated in vacuo. The residue was charged on a column of silica-gel (C-200, 180 g) and eluted successively with benzene and benzene-ethyl acetate (10 : 1) to obtain the product (14.12 g, Yield 90.5%)

Example 102
(3-benzyl-5,6,7,8-tetrahydroquinoxaline-2-yl oxy)-acetate (compound 660):

Aqueous 2N-NaOH (30 ml) was added to compound 656 (9.42 g, 30.2 mM) dissolved in methanol (30 ml) and stirred for 3 hours at room temperature. 2N-HCl (30 ml) was added to the reaction mixture under ice-cooling and the precipitated crystals were collected by filtration, washed with water and dried to obtain the product. (8.10 g) Filtrate was concentrated in vacuo and the residue was extracted with hot ethanol, then the extract was concentrated in vacuo. The residue was dissolved in dilute aqueous NaOH, was neutralized

with 1 N-HCl and the precipitated crystals were collected by filtration, washed with water, then dried to obtain the product. (0.32 g) (Total: 8.42 g, Yield: 93.6%)

Example 103
N-(2-chloroethyl)-(3-benzyl-5,6,7,8-tetrahydroquinoxaline-2-yl oxy)-acetamide (compound 661):

Pivaloylchloride (3.05 g, 25 mM) was added dropwise at -5 °C to compound 660 (7.45 g, 25 mM) and triethylamine (3.5 ml, 25 mM) dissolved in tetrahydrofuran (45 ml), then stirred for 30 minutes. Chloroform (25 ml) in solution with 2-chloroethylamine hydrochloride (2.90 g) and triethylamine (3.5 ml) was added dropwise thereto, then stirred for 1 hour under ice-cooling and for 4 hours at room temperature. Dilute aqueous potassium carbonate was added to the reaction mixture and extracted twice with chloroform. The extract was dried with anhydrous sodium sulfate, and concentrated in vacuo. The residue was charged on a column of silica-gel (C-200, 175 g) and eluted successively with benzene-ethyl acetate to obtain the product. (7.142 g, Yield: 79.5%)

Examples 104-109
N-(2-substituted ethyl)-(3-benzyl-5,6,7,8-tetrahydroquinoxaline-2-yl oxy)-acetamide:

Benzene (30 ml) in solution with compound 661 (1.08 g, 3.0 mM), triethylamine (0.84 ml) and base (6.0 mM) was refluxed. Dilute aqueous potassium carbonate was added to the reaction mixture, and the aqueous layer was extracted three times with chloroform. The organic layer was dried with anhydrous sodium sulfate, and concentrated in vacuo. The residue was charged on a column of silica-gel (C-200, 80 g) and eluted successively with benzene and benzene-ethyl acetate (10 : 1) to obtain the compounds in Table 21.

Table 21 shows the base and its amount used, the reflux time, the products and the yield in weight and percentage, in each of the above examples.

Examples 110-121
Methyl (3-alkyl-5,6,7,8-tetrahydroquinoxaline-2-yl oxy)-acetate:

Metallic sodium (1.15 g) was dissolved in anhydrous methanol (100 ml). 2-hydroxy-3-alkyl-5,6,7,8-tetrahydroquinoxaline (50 mM) was dissolved therein, and methanol was distilled off in vacuo to obtain the sodium salt. Dimethylformamide (100 ml) and methyl chloroacetate (5.43 g, 50 mM) were added thereto and stirred at 100 °C. The reaction mixture was concentrated in vacuo, and dilute aqueous sodium carbonate was added to the residue, which was then extracted three times with chloroform, and the extract was dried with anhydrous sodium sulfate and concentrated in vacuo. The residue was charged on a column of silica-gel (C-200) and eluted successively with benzene and benzene-ethyl acetate (20 : 1) to obtain the compound in Table 22.

Table 22 shows the starting compound and its amount used, the reaction time, amount of silica-gel used, the products and the yield in weight and percentage in each of the above examples.

Examples 122-133
2-(2-hydroxyethoxy)-3-alkyl-5,6,7,8-tetrahydroquinoxaline:

The starting compound was dissolved in methanol. 6-8 equivalents of $NaBH_4$ were added several portionwise under reflux. The reaction mixture was concentrated in vacuo, water was added thereto, then extracted three times with chloroform. The extract was dried with anhydrous sodium sulfate and concentrated in vacuo. The residue was charged on a column of silica-gel (C-200) and eluted successively with benzene and benzene-ethyl acetate (10 : 1) to obtain the compounds in Table 23.

Table 23 shows the starting compound and its amount used, amount of methanol used, amount of $NaBH_4$ used, reflux time, amount of silica-gel used and the product and yield in weight and percentage in each of the above examples.

Examples 134-185
2-(2-substituted ethoxy)-3-alkyl-5,6,7,8-tetrahydroquinoxaline:

Thionylchloride (1.3 equivalent) was added dropwise under ice-cooling to 2-(2-hydroxyethoxy)-3-alkyl-5,6,7,8-tetrahydroquinoxaline (3.5 mM) dissolved in chloroform and stirred at room temperature for 2.5 hours to chlorinate the compound. The reaction mixture was poured into dilute aqueous potassium carbonate and

extracted three times with chloroform. The extract, which was dried with anhydrous sodium sulfate, was concentrated in vacuo. Benzene (30 ml), triethylamine (2 equivalents) and a base (2 equivalents) were added to the residue, and the mixture was refluxed. The reaction mixture was poured into dilute aqueous potassium carbonate, and the mixture was extracted three times with chloroform. The extract was dried with anhydrous sodium sulfate and concentrated in vacuo. The residue was charged on a column of silica-gel (C-200, 75 g) and eluted successively with chloroform and chloroform-methanol (200 : 1) to obtain the compounds in Table 24.

Table 24 shows the starting compound and its amount used, chlorination reaction time, the base and its amount used, the reflux time, and the product and yield in weight and percentage in each of the above examples.

TABLE 7 2-(substituted carbonylmethoxy)-3,5,6-trimethylpyrazine

| Example | Base name | Base used (g) | Product | Yield (g) | Yield (%) |
|---|---|---|---|---|---|
| 7 | 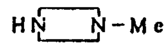 | 0.50 | 066 | 1.12 | 80.5 |
| 8 | 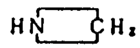 | 0.43 | 067 | 1.10 | 83.6 |
| 9 | 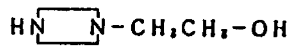 | 0.66 | 068 | 1.23 | 79.8 |
| 10 | $H_2N-CH_2CH_2-OH$ | 0.32 | 069 | 0.88 | 73.6 |
| 11 | 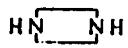 | 0.43 | 070 | 0.91 | 82.0 |
| 12 | 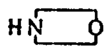 | 0.44 | 075 | 1.10 | 83.0 |

product is shown by No. of compound

TABLE 8 N-(2-substituted ethyl)-(3,5,6-trimethylpyrazine-2-yloxy)-acetamide

| Example | Base name | Base used (g) | reaction (time) | solvent ratio (hr.) | product | Yield (g) | Yield (%) |
|---|---|---|---|---|---|---|---|
| 15 | 40%HN $(CH_3)_2$ aqueous solution | 8.68 | 1 | 50:1 | 071 | 0.22 | 27.6 |
| 16 | 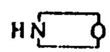 | 0.53 (6mM) | 1.5 | 100:1 | 073 | 0.41 | 44.4 |
| 17 | 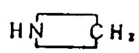 | 0.43 (5mM) | 1 | 20:1 | 074 | 0.55 | 59.9 |
| 18 | 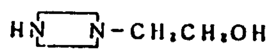 | 0.78 (6mM) | 1.5 | 50:1 | 076 | 0.58 | 55.0 |
| 19 | 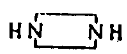 | 3.44 (40mM) | 1.5 | 10:1 | 103 | 0.94 | 30.0 |
| 20 | 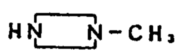 | 0.60 (6mM) | 1 | 50:1 | 105 | 0.27 | 28.0 |

product is shown by No. of compound

TABLE 9 N-(2-substituted ethyl)-(3,5,6-trimethylpyrazine-2-yloxy)-acetamide

| Example | BASE name | BASE used (g) | reaction time (hr.) | solvent ratio | Product | Yield (g) | Yield (%) |
|---|---|---|---|---|---|---|---|
| 22 | 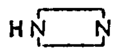-$CH_2$Ph-Cl (p) | 1.68 (8mM) | 8 | 100:1 | 135 | 0.50 | 30.5 |
| 23 | 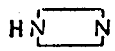-$CH_2$Ph | 1.41 (8mM) | 6 | 50:1 | 136 | 0.43 | 27.1 |
| 24 | 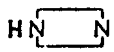-$CH_2$Ph-Cl (o) | 1.68 (8mM) | 8 | 100:1 | 137 | 0.50 | 30.5 |

product is shown by No. of compound

TABLE 10 N-(2-substituted ethyl)-α-(3-ethyl-5,6-dimethylpyrazine-2-yloxy)-phenylacetamide

| Example | BASE name | BASE used (g) | product | Yield (g) | Yield (%) |
|---|---|---|---|---|---|
| 28 | 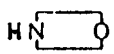 | 0.70 | 341 | 0.94 | 59.0 |
| 29 | 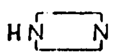-$CH_2$Ph | 1.41 | 342. | 1.48 | 76.0 |
| 30 | 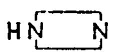-$CH_2$Ph-Cl (p) | 1.69 | 343 | 1.27 | 60.9 |
| 31 | 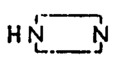-$CH_2$Ph-$NO_2$ (p) | 1.77 | 344 | 1.32 | 62.0 |
| 32 | 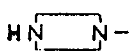-$CH_2$Ph-$Cl_2$ (2, 4) | 1.96 | 345 | 0.91 | 41.0 |
| 33 | 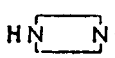-Ph | 1.30 | 346 | 1.20 | 63.4 |
| 34 | 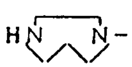-$CH_2$Ph-F (p) | 1.67 | 347 | 1.28 | 61.5 |
| 35 | 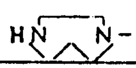-$CH_2$Ph-Cl (p) | 1.80 | 348 | 1.77 | 82.6 |

product is shown by No. of compound

TABLE 11 N-(2-substituted ethyl)-(3-benzyl 5,6-dimethylpyrazine-2-yloxy)-acetamide

| Example | BASE | | reaction (day) | product | Yield (g) | Yield (%) |
|---|---|---|---|---|---|---|
| | name | used (g) | | | | |
| 39 | 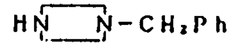 | 1.24 | 2 | 376 | 1.54 | 87.8 |
| 40 | 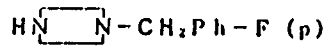 | 1.48 | 2 | 377 | 1.48 | 81.5 |
| 41 | 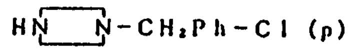 | 1.48 | 2 | 379 | 1.17 | 62.4 |
| 42 | 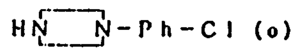 | 1.38 | 1 | 381 | 1.29 | 70.7 |
| 43 | 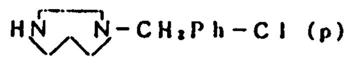 | 1.58 | 2 | 382 | 1.38 | 71.8 |
| 44 | 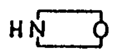 | 0.61 | 2 | 657 | 0.73 | 50.6 |
| 45 | 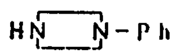 | 1.14 | 1 | 658 | 1.15 | 67.6 |

product is shown by No. of compound

TABLE 12 Methyl (3,5,6-trialkylpyrazine-2-yloxy)-acetate

| Example | starting material | | | | reaction time (hr.) | silica-gel (g) | product | Yield (g) | Yield (%) |
|---|---|---|---|---|---|---|---|---|---|
| | R₃ N OH / R₂ N R₁ | | | used (g) | | | | | |
| | $R_1$ | $R_2$ | $R_3$ | | | | | | |
| 46 | Et | Me | Me | 7.60 | 2.5 | 160 | 662 | 9.62 | 85.9 |
| 47 | $-CH_2Ph$ | Me | Me | 10.70 | 2.5 | 210 | 663 | 12.03 | 84.1 |
| 48 | iso-Pro | Me | Me | 8.3 | 2.5 | 160 | 664 | 9.92 | 83.4 |
| 49 | 〃 | Et | Et | 9.70 | 2.5 | 180 | 665 | 12.77 | 96.0 |
| 50 | iso-Bu | Et | Et | 10.40 | 3 | 230 | 666 | 12.86 | 91.9 |

product is shown by No. of compound

TABLE 13 2-(2-hydroxyethoxy)-3,5,6-trialkylpyrazine

| Example | starting material | | methanol (ml) | $NaBH_4$ (g) | reaction time (hr.) | silica-gel (g) | product | Yield (g) | Yield (%) |
|---|---|---|---|---|---|---|---|---|---|
| | name | used g (mM) | | | | | | | |
| 51 | 034 | 17.00 (80.95) | 200 | 18.6 | 3.0 | 200 | 679 | 12.84 | 87.2 |
| 52 | 662 | 9.62 (42.94) | 200 | 9.9 | 4.0 | 120 | 680 | 7.69 | 91.3 |
| 53 | 663 | 8.75 (30.6) | 150 | 9.4 | 2.5 | 160 | 681 | 7.22 | 91.5 |
| 54 | 664 | 19.67 (82.65) | 200 | 25.2 | 3.0 | 200 | 682 | 15.51 | 89.4 |
| 55 | 665 | 9.18 (34.5) | 150 | 10.5 | 4.0 | 200 | 683 | 7.48 | 91.1 |
| 56 | 666 | 7.32 (26.14) | 100 | 8.0 | 3.5 | 160 | 684 | 5.94 | 90.1 |

product is shown by No. of compound

TABLE 14 2-(2-substituted ethoxy)-3,5,6-trimethylpyrazine

| Example | BASE | | reaction time (hr.) | product | Yield (g) | Yield (%) |
|---|---|---|---|---|---|---|
| | name | used (g) | | | | |
| 57 | 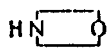 | 1.05 | 2.5 | 536 | 0.95 | 63.1 |
| 58 | 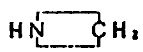 | 1.10 | 2.5 | 537 | 1.03 | 68.9 |
| 59 | 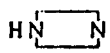-$CH_2Ph$ | 2.02 | 3.0 | 538 | 1.31 | 64.2 |
| 60 | 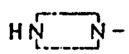-$CH_2Ph$-Cl (p) | 2.11 | 2.5 | 539 | 1.62 | 72.1 |
| 61 | 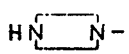-$CH_2Ph$-F (p) | 1.94 | 2.0 | 540 | 1.59 | 74.0 |
| 62 | 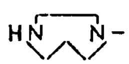-$CH_2Ph$-Cl (p) | 2.25 | 2.5 | 541 | 1.56 | 66.9 |
| 63 | 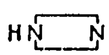-Bu | 1.70 | 5.0 | 810 | 1.28 | 70.0 |
| 64 | 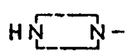-$CH_2CH_2$-OH | 3.90 | 5.0 | 822 | 1.56 | 94.5 |
| 65 | $HNEt_2$ | 0.88 | 5.0 | 842 | 0.51 | 35.7 |

product is shown by No. of compound

TABLE 15 2-(2-substituted ethoxy)-3-isopropyl-5,6-diethylpyrazine

| Example | BASE name | BASE used (g) | reaction time (hr.) | product | Yield (g) | Yield (%) |
|---|---|---|---|---|---|---|
| 66 | 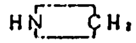 | 0.85 | 3.0 | 550 | 0.83 | 54.4 |
| 67 | 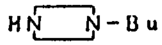 | 1.42 | 2.5 | 551 | 0.93 | 51.4 |
| 68 | 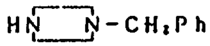 | 1.76 | 3.0 | 552 | 1.05 | 52.8 |
| 69 | 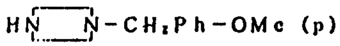 | 2.20 | 3.0 | 553 | 1.01 | 46.0 |
| 70 | 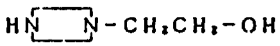 | 1.30 | 5.0 | 826 | 1.15 | 66.2 |
| 71 | $HNEt_2$ | 0.73 | 6.5 | 848 | 0.36 | 25.2 |

product is shown by No. of compound

TABLE 16 2-(2-substituted ethoxy)-3-ethyl-5,6-dimethylpyrazine

| Example | BASE name | BASE used (g) | reaction time (hr.) | solvent ratio | product | Yield (g) | Yield (%) |
|---|---|---|---|---|---|---|---|
| 72 | 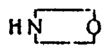 | 0.87 | 2.5 | 200:1 | 491 | 0.66 | 49.8 |
| 73 | 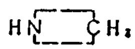 | 0.85 | 2.0 | 200:1 | 492 | 0.79 | 60.1 |
| 74 | 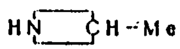 | 1.00 | 2.0 | 200:1 | 493 | 0.67 | 48.4 |
| 75 | 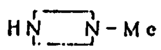 | 1.00 | 2.0 | 30:1 | 494 | 0.84 | 44.6 |
| 76 | 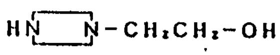 | 1.20 | 2.0 | 100:1 | 495 | 0.73 | 47.4 |
| 77 | 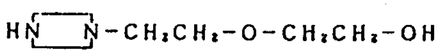 | 1.74 | 2.0 | 10:1<br>50:1 | 496 | 1.13 | 64.2 |
| 78 | 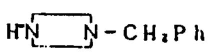 | 1.68 | 2.0 | 200:1 | 497 | 1.15 | 65.0 |

| Example | BASE name | BASE used (g) | reaction time (hr.) | solvent ratio | product | Yield (g) | Yeild (%) |
|---|---|---|---|---|---|---|---|
| 79 | 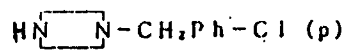 | 2. 11 | 2. 0 | 200:1 | 498 | 1. 10 | 56. 6 |
| 80 | 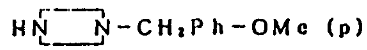 | 2. 06 | 2. 0 | 200:1 | 499 | 1. 17 | 60. 9 |
| 81 | 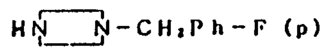 | 1. 94 | 2. 0 | 200:1 | 500 | 1. 17 | 62. 9 |
| 82 | 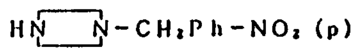 | 2. 21 | 2. 0 | 200:1 | 501 | 1. 40 | 70. 2 |
| 83 | 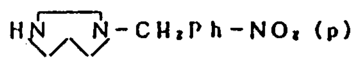 | 2. 35 | 2. 0 | 200:1 | 502 | 0. 62 | 30. 0 |
| 84 | 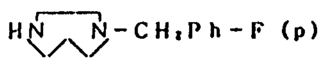 | 2. 08 | 2. 0 | 200:1 | 503 | 1. 00 | 51. 8 |
| 85 | 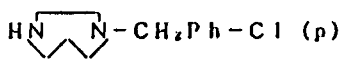 | 2. 25 | 2. 0 | 200:1 | 504 | 1. 14 | 56. 6 |
| 86 | 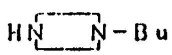 | 1. 42 | 2. 0 | 200:1 | 812 | 0. 67 | 42. 0 |
| 87 | $HNEt_2$ | 0. 73 | 3. 0 | 200:1 | 844 | 0. 23 | 18. 8 |

product is shown by No. of compound

TABLE 17 2-(2-substituted ethoxy)-3-benzyl-5,6-dimethylpyrazine

| Example | BASE | | reaction time (hr.) | solvent ratio | product | Yield (g) | Yeild (%) |
|---|---|---|---|---|---|---|---|
| | name | used (g) | | | | | |
| 88 | 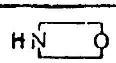 | 0. 70 | 2. 0 | 200:1 | 511 | 0. 57 | 43. 6 |
| 89 | 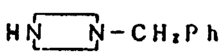 | 1. 04 | 2. 0 | 200:1 | 512 | 1. 04 | 62. 5 |
| 90 | 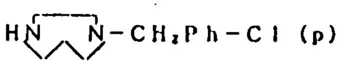 | 1. 80 | 2. 0 | 200:1 | 516 | 1. 20 | 64. 6 |

product is shown by No. of compound

TABLE 18 2-(2-substituted ethoxy)-3-isopropyl-5,6-dimethylpyrazine

| Example | BASE name | BASE used (g) | reaction time (hr.) | solvent ratio | product | Yield (g) | Yield (%) |
|---|---|---|---|---|---|---|---|
| 91 | 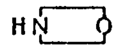 | 0. 87 | 2. 5 | 200 : 1 | 544 | 0. 89 | 63. 8 |
| 92 | 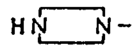 $-CH_2Ph-Cl$ (p) | 2. 11 | 2. 5 | 200 : 1 | 546 | 1. 57 | 80. 8 |
| 93 | 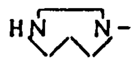 $-CH_2Ph-Cl$ (p) | 2. 25 | 2. 5 | 200 : 1 | 547 | 1. 42 | 68. 2 |
| 94 | 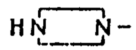 -Bu | 1. 43 | 3. 0 | 200 : 1 | 697 | 0. 94 | 56. 3 |
| 95 | 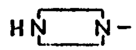 -Bu | 1. 43 | 2. 0 | 200 : 1 | 814 | 0. 77 | 46. 0 |
| 96 | 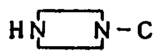 $-CH_2CH_2-OH$ | 1. 30 | 2. 5 | 200 : 1 | 825 | 1. 24 | 77. 0 |
| 97 | 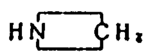 | 0. 85 | 5. 0 | 200 : 1 | 834 | 1. 17 | 68. 3 |
| 98 | $HNEt_2$ | 0. 73 | 18. 0 | 200 : 1 | 846 | 0. 12 | 11. 2 |

product is shown by No. of compound

TABLE 19 2-(2-substituted ethoxy)-3-isobutyl-5,6-diethylpyrazine

| Example | BASE name | BASE used (g) | reaction time (hr.) | product | Yield (g) | Yield (%) |
|---|---|---|---|---|---|---|
| 99 | 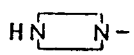 $-CH_2Ph$ | 1. 76 | 3. 5 | 556 | 0. 45 | 21. 9 |
| 100 | 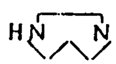 $-CH_2Ph-OMe$ (p) | 2. 20 | 4. 0 | 558 | 0. 77 | 33. 9 |

product is shown by No. of compound

TABLE 20 2-hydroxy-3-alkyl-5, 6, 7, 8 tetrahydroquinoxaline

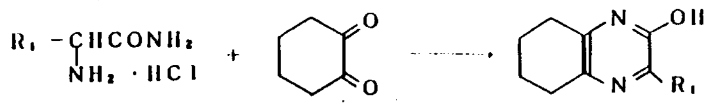

| Reference example | amino acidamide HCℓ used (g) | Product | | |
|---|---|---|---|---|
| | | $R_1$ | Yield (g) | Yield (%) |
| 5 | 7. 63 | Pro | 4. 75 | 50. 0 |
| 6 | 7. 63 | iso-Pro | 4. 95 | 51. 6 |
| 7 | 8. 32 | Bu | 7. 49 | 73. 0 |
| 8 | 8. 32 | iso-Bu | 6. 58 | 64. 0 |
| 9 | 8. 32 | sec-Bu | 6. 58 | 64. 0 |
| 10 | 9. 04 | $-(CH_2)_4CH_3$ | 9. 03 | 82. 0 |
| 11 | 9. 74 | $-(CH_2)_5CH_3$ | 7. 81 | 66. 7 |
| 12 | 10. 44 | $-(CH_2)_6CH_3$ | 9. 33 | 75. 0 |
| 13 | 11. 14 | $-(CH_2)_7CH_3$ | 11. 67 | 89. 1 |
| 14 | 11. 83 | $-(CH_2)_8CH_3$ | 10. 50 | 72. 4 |
| 15 | 12. 55 | $-(CH_2)_9CH_3$ | 12. 73 | 92. 2 |
| 16 | 13. 95 | $-(CH_2)_{11}CH_3$ | 14. 00 | 88. 1 |
| 17 | 15. 35 | $-(CH_2)_{13}CH_3$ | 16. 58 | 95. 8 |
| 18 | 16. 75 | $-(CH_2)_{15}CH_3$ | 18. 55 | 99. 2 |

TABLE 21 N-(2-substituted ethyl)-(3-benzyl 5, 6, 7, 8-tetrahydroquinoxaline-2-yloxy)-acetamide

| Example | BASE | | reaction time (day) | Product | | |
|---|---|---|---|---|---|---|
| | name | used (g) | | No. of comp | Yield (g) | Yield (%) |
| 104 | 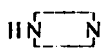 $-CH_2Ph$ | 1. 06 | 1 | 368 | 1. 00 | 66. 8 |
| 105 | 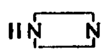 $-CH_2Ph-OMe$ (p) | 1. 24 | 4 | 369 | 1. 38 | 87. 0 |
| 106 | 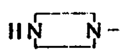 $-CH_2Ph-Cl$ (p) | 1. 27 | 2 | 370 | 1. 07 | 67. 0 |
| 107 | 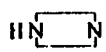 $-Ph-Cl$ (o) | 1. 18 | 3 | 371 | 1. 36 | 87. 3 |

| | | | | | | |
|---|---|---|---|---|---|---|
| 108 | 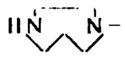-CH$_2$Ph-F (p) | 1.25 | 3 | 372 | 1.21 | 76.0 |
| 109 | 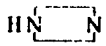-Ph | 0.73 | 2 | 373 | 0.73 | 50.2 |

TABLE 22 methyl(3-alkyl-5,6,7,8-tetrahydroquinoxaline 2-yloxy)-acetate

| Example | starting compound N OH / N R$_1$ — R$_1$ | starting compound used (g) | reaction time (hr.) | silica-gel (g) | Product: No. of compound | Product: Yield (g) | Product: Yield (%) |
|---|---|---|---|---|---|---|---|
| 110 | Bu | 10.30 | 3 | 210 | 667 | 11.54 | 83.2 |
| 111 | $-(CH_2)_4CH_3$ | 10.10 | 3 | 210 | 668 | 11.64 | 79.7 |
| 112 | $-(CH_2)_5CH_3$ | 11.70 | 2.5 | 210 | 669 | 12.24 | 80.0 |
| 113 | $-(CH_2)_6CH_3$ | 12.40 | 2.5 | 210 | 670 | 13.35 | 83.5 |
| 114 | $-(CH_2)_7CH_3$ | 13.10 | 3 | 200 | 671 | 13.02 | 78.0 |
| 115 | $-(CH_2)_8CH_3$ | 13.80 | 3 | 240 | 672 | 13.71 | 78.8 |
| 116 | $-(CH_2)_9CH_3$ | 14.50 | 2.5 | 210 | 673 | 13.61 | 75.2 |
| 117 | $-(CH_2)_{11}CH_3$ | 15.90 | 3 | 280 | 674 | 16.00 | 82.1 |
| 118 | $-(CH_2)_{13}CH_3$ | 17.30 | 3 | 280 | 675 | 16.33 | 78.1 |
| 119 | $-(CH_2)_{15}CH_3$ | 18.70 | 2.5 | 340 | 676 | 18.74 | 84.0 |
| 120 | Pro | 9.60 | 3 | 220 | 677 | 10.29 | 78.0 |
| 121 | sec-Bu | 10.30 | 2.5 | 260 | 678 | 11.73 | 84.4 |

TABLE 23 2-(2-hydroxyethoxy)-3-alkyl-5,6,7,8-tetrahydroquinoxaline

| Example | starting compound: No. of comp | starting compound: used g (mM) | methanol (ml) | $NaBH_4$ (g) | reaction time (hr.) | silica-gel (g) | Product: No. of comp | Product: Yield (g) | Product: Yield (%) |
|---|---|---|---|---|---|---|---|---|---|
| 122 | 667 | 12.40 (44.6) | 200 | 10.2 | 2.5 | 200 | 685 | 10.63 | 95.3 |
| 123 | 668 | 9.07 (31.1) | 200 | 7.2 | 3.0 | 200 | 686 | 7.12 | 86.7 |

| | | | | | | | | | |
|---|---|---|---|---|---|---|---|---|---|
| 124 | 669 | 9.55 (31.2) | 200 | 9.6 | 2.5 | 200 | 687 | 7.95 | 91.7 |
| 125 | 670 | 9.70 (30.3) | 200 | 9.3 | 3.0 | 200 | 688 | 8.05 | 91.0 |
| 126 | 671 | 11.10 (33.2) | 300 | 7.8 | 3.0 | 250 | 689 | 9.31 | 91.6 |
| 127 | 672 | 9.50 (27.3) | 250 | 9.4 | 3.0 | 250 | 690 | 8.20 | 93.9 |
| 128 | 673 | 10.80 (29.8) | 250 | 9.1 | 2.5 | 250 | 691 | 9.15 | 92.0 |
| 129 | 674 | 9.54 (24.46) | 250 | 7.5 | 3.0 | 200 | 692 | 7.94 | 89.7 |
| 130 | 675 | 9.56 (22.9) | 250 | 6.9 | 3.0 | 200 | 693 | 8.63 | 96.6 |
| 131 | 676 | 9.17 (32.9) | 250 | 6.2 | 3.0 | 200 | 694 | 7.27 | 84.6 |
| 132 | 677 | 7.78 (29.47) | 200 | 9.0 | 3.0 | 200 | 695 | 6.60 | 95.0 |
| 133 | 678 | 7.50 (27.0) | 200 | 8.4 | 2.5 | 170 | 696 | 5.77 | 85.5 |

TABLE 24 2-(2-substituted ethoxy-3-alkyl 5,6,7,8-tetrahydroquinoxaline

| Exampl | starting compound | | chlorination time(hr) | BASE | | reflux time (hr.) | product | | |
|---|---|---|---|---|---|---|---|---|---|
| | No.of comp | used g | | name | used g | | No.of comp | Yield(g) | Yield(%) |
| 134 | 685 | 1.25 (5mM) | 3.0 | 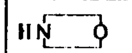 | 0.87 (10mM) | 2.0 | 517 | 1.38 | 86.5 |
| 135 | 685 | 1.00 (4mM) | 3.0 | 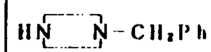 | 1.42 (8mM) | 2.0 | 518 | 1.23 | 75.4 |
| 136 | 685 | 1.00 (4mM) | 3.0 | 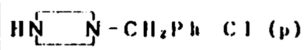 | 1.69 (8mM) | 2.0 | 519 | 0.89 | 50.3 |
| 137 | 685 | 1.00 (4mM) | 3.0 | 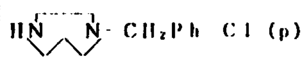 | 1.80 (8mM) | 2.5 | 520 | 1.00 | 54.8 |
| 138 | 686 | 1.32 (5mM) | 3.5 | 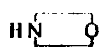 | 0.87 (10mM) | 3.0 | 559 | 0.92 | 55.3 |
| 139 | 686 | 1.32 (5mM) | 3.5 | 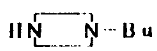 | 1.42 (10mM) | 2.5 | 560 | 1.01 | 52.1 |
| 140 | 686 | 1.32 (5mM) | 3.5 | 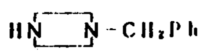 | 1.76 (10mM) | 3.0 | 561 | 1.27 | 60.2 |
| 141 | 686 | 1.32 (5mM) | 3.5 | 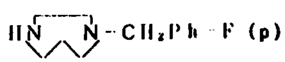 | 2.08 (10mM) | 2.5 | 562 | 1.24 | 54.6 |
| 142 | 687 | 1.39 (5mM) | 3.5 | 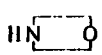 | 0.87 (10mM) | 3.0 | 564 | 0.99 | 57.1 |

| | | | | | | | | | |
|---|---|---|---|---|---|---|---|---|---|
| 143 | 687 | 1. 39 (5mM) | 3. 5 | 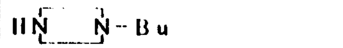 | 1. 42 (10mM) | 3. 0 | 565 | 1. 11 | 55. 2 |
| 144 | 687 | 1. 39 (5mM) | 3. 5 | 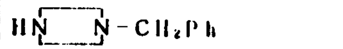 | 1. 76 (10mM) | 2. 5 | 567 | 1. 25 | 57. 3 |
| 145 | 687 | 1. 39 (5mM) | 3. 5 | 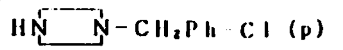 | 2. 10 (10mM) | 2. 5 | 568 | 1. 62 | 68. 9 |
| 146 | 687 | 1. 39 (5mM) | 3. 5 | 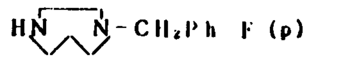 | 2. 08 (10mM) | 2. 5 | 569 | 1. 29 | 55. 4 |
| 147 | 688 | 1. 17 (4mM) | 3. 0 | 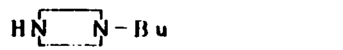 | 1. 14 (8mM) | 2. 5 | 571 | 1. 01 | 60. 7 |
| 148 | 689 | 1. 53 (5mM) | 3. 0 | 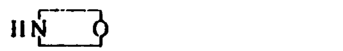 | 0. 87 (10mM) | 2. 0 | 572 | 1. 06 | 56. 5 |
| 149 | 689 | 1. 23 (4mM) | 3. 0 | 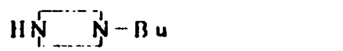 | 1. 14 (8mM) | 2. 0 | 573 | 0. 87 | 50. 6 |
| 150 | 689 | 1. 23 (4mM) | 3. 0 | 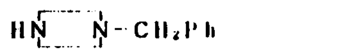 | 1. 42 (8mM) | 2. 0 | 574 | 0. 95 | 52. 9 |
| 151 | 689 | 1. 23 (4mM) | 3. 0 | 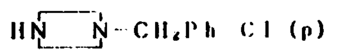 | 1. 68 (8mM) | 2. 0 | 575 | 0. 65 | 32. 6 |
| 152 | 689 | 1. 23 (4mM) | 3. 0 | 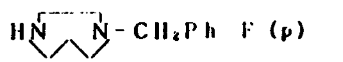 | 1. 67 (8mM) | 2. 5 | 576 | 0. 57 | 28. 7 |
| 153 | 690 | 1. 60 (5mM) | 3. 5 | 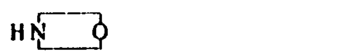 | 0. 87 (10mM) | 2. 0 | 579 | 0. 63 | 32. 4 |
| 154 | 690 | 1. 28 (4mM) | 3. 5 | 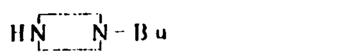 | 1. 14 (8mM) | 2. 5 | 580 | 0. 54 | 30. 4 |
| 155 | 690 | 1. 28 (4mM) | 3. 5 | 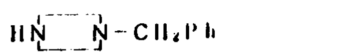 | 1. 41 (8mM) | 2. 0 | 581 | 0. 84 | 43. 9 |
| 156 | 690 | 1. 28 (4mM) | 3. 5 | 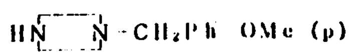 | 1. 65 (8mM) | 2. 0 | 582 | 1. 01 | 47. 8 |
| 157 | 690 | 1. 28 (4mM) | 3. 5 | 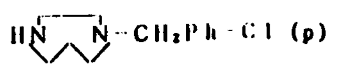 | 1. 80 (8mM) | 2. 5 | 583 | 0. 78 | 37. 0 |
| 158 | 691 | 1. 67 (5mM) | 3. 0 | 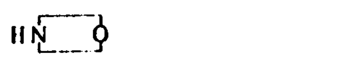 | 0. 87 (10mM) | 2. 0 | 585 | 0. 79 | 39. 2 |
| 159 | 691 | 1. 34 (4mM) | 3. 0 | 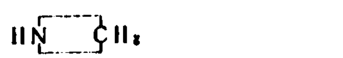 | 0. 68 (8mM) | 2. 5 | 586 | 0. 65 | 40. 5 |

| | | | | | | | | | |
|---|---|---|---|---|---|---|---|---|---|
| 160 | 691 | 1. 34 (4mM) | 3. 0 | 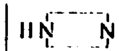 -Bu | 1. 14 (8mM) | 2. 5 | 587 | 0. 77 | 42. 0 |
| 161 | 691 | 1. 34 (4mM) | 3. 0 | 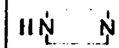 $-CH_2CH_2-OH$ | 1. 04 (8mM) | 2. 5 | 588 | 0. 58 | 32. 5 |
| 162 | 691 | 1. 34 (4mM) | 3. 0 | 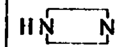 $-CH_2Ph$ | 1. 41 (8mM) | 2. 5 | 589 | 0. 93 | 47. 3 |
| 163 | 691 | 1. 34 (4mM) | 3. 0 | 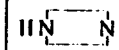 $-CH_2Ph-Cl$ (p) | 1. 68 (8mM) | 2. 5 | 590 | 1. 07 | 50. 8 |
| 164 | 691 | 1. 34 (4mM) | 3. 0 | 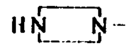 $-CHPh_2$ | 2. 01 (8mM) | 2. 5 | 591 | 1. 17 | 51. 5 |
| 165 | 691 | 1. 34 (4mM) | 3. 0 | 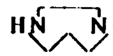 $-CH_2Ph$ F (p) | 1. 67 (8mM) | 2. 0 | 592 | 0. 70 | 33. 4 |
| 166 | 692 | 1. 09 (3mM) | 4. 5 | 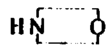 | 0. 53 (6mM) | 3. 0 | 594 | 0. 64 | 49. 5 |
| 167 | 692 | 1. 09 (3mM) | 5. 0 | 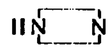 -Bu | 0. 86 (6mM) | 2. 5 | 595 | 0. 84 | 57. 6 |
| 168 | 692 | 1. 09 (3mM) | 5. 0 | 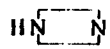 $-CH_2Ph$ | 1. 06 (6mM) | 2. 5 | 596 | 0. 92 | 59. 0 |
| 169 | 692 | 1. 09 (3mM) | 5. 0 | 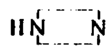 $-CH_2Ph$ OMe (p) | 1. 24 (6mM) | 2. 5 | 597 | 0. 93 | 56. 4 |
| 170 | 692 | 1. 09 (3mM) | 4. 5 | 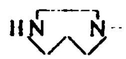 $-CH_2Ph$ F (p) | 1. 25 (6mM) | 2. 5 | 598 | 0. 83 | 50. 1 |
| 171 | 693 | 1. 17 (3mM) | 5. 0 | 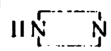 Bu | 0. 86 (6mM) | 2. 5 | 600 | 0. 74 | 48. 0 |
| 172 | 693 | 1. 17 (3mM) | 5. 0 | 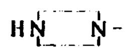 $-CH_2Ph$ | 1. 06 (6mM) | 2. 5 | 601 | 0. 84 | 51. 1 |
| 173 | 693 | 1. 17 (3mM) | 5. 0 | 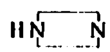 $-CH_2Ph$ F (p) | 1. 17 (6mM) | 2. 5 | 602 | 0. 93 | 54. 8 |
| 174 | 693 | 1. 17 (3mM) | 5. 0 | 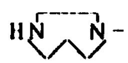 $-CH_2Ph-Cl$ (p) | 1. 35 (6mM) | 2. 5 | 603 | 0. 92 | 51. 4 |
| 175 | 693 | 1. 17 (3mM) | 5. 0 | 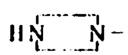 $-CHPh_2$ | 1. 51 (6mM) | 2. 5 | 604 | 0. 65 | 34. 7 |
| 176 | 694 | 1. 26 (3mM) | 3. 5 | 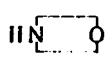 | 0. 53 (6mM) | 2. 5 | 606 | 0. 58 | 39. 7 |

| | | | | | | | | | |
|---|---|---|---|---|---|---|---|---|---|
| 177 | 694 | 1. 26 (3mM) | 4. 5 | 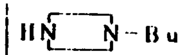 | 0. 86 (6mM) | 2. 5 | 607 | 0. 86 | 52. 9 |
| 178 | 694 | 1. 26 (3mM) | 5. 0 | 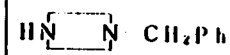 | 1. 06 (6mM) | 2. 0 | 608 | 0. 88 | 50. 9 |
| 179 | 694 | 1. 26 (3mM) | 4. 5 | 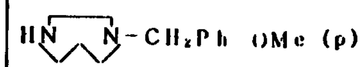 | 1. 32 (6mM) | 2. 5 | 609 | 0. 95 | 51. 1 |
| 180 | 685 | 1. 25 (5mM) | 6. 0 | 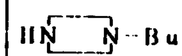 | 1. 42 (10mM) | 5. 0 | 817 | 1. 10 | 59. 0 |
| 181 | 689 | 0. 77 (2. 5 mM) | 5. 5 | 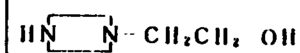 | 1. 60 (12. 3 mM) | 5. 0 | 830 | 0. 45 | 43. 1 |
| 182 | 685 | 1. 18 (4. 7 mM) | 6. 5 | 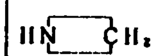 | 0. 85 (10mM) | 5. 0 | 837 | 0. 89 | 56. 3 |
| 183 | 689 | 0. 77 (2. 5 mM) | 6. 0 | 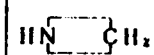 | 0. 43 (5mM) | 6. 0 | 839 | 0. 61 | 65. 1 |
| 184 | 685 | 1. 25 (5mM) | 5. 0 | $HNEt_2$ | 1. 46 (20mM) | 24 | 850 | 0. 38 | 25. 0 |
| 185 | 689 | 0. 77 (2. 5 mM) | 7. 0 | $HNEt_2$ | 0. 73 (10mM) | 24 | 851 | 0. 47 | 52. 1 |

TABLE 25 NMR spectrum, Mass spectrum and Rf value on silica gel layer chromatography

| Comp. No. | NMR ($CDCl_3$、inner standard TMS) ppm | MASS | Rf |
|---|---|---|---|
| 034 | 2.34 (3H, s)、2.40 (3H, s)、2.46 (3H, s)、3.76 (3H, s)、4.89 (2H, s) | 211 | |
| 035 | 1.53 (3H, d, J=6.9)、2.28 (3H, s)、2.31 (3H×2, s)、3.65 (3H, s)、5.17 (1H, q, J=6.9) | 225 | |
| 036 | 2.36 (3H, s)、2.41 (3H, s)、2.51 (3H, s)、3.70 (3H, s)、6.11 (1H, s)、7.3-7.6 (5H, m) | 287 | |
| 037 | 2.30 (3H, s)、2.35 (6H, s)、4.83 (2H, s) | 197 | |
| 038 | 1.66 (2H, d, J=7.1)、2.32 (3H, s)、2.33 (3H, s)、2.45 (3H, s)、5.27 (1H, q, J=7.1) | 211 | |
| 039 | 2.33 (3H, s)、2.35 (3H, s)、2.38 (3H, s)、6.05 (1H, s)、7.3-7.6 (5H, m) | 273 | |
| 066 | 2.32 (3H, s)、2.35 (3H, s)、2.39 (3H, s)、2.46 (3H, s)、2.3-2.5 (4H, m) | 279, 179 | |
| 067 | 1.5-1.7 (3H×2), 2.35 (3H, s)、2.39 (3H, s)、2.47 (3H, s)、3.3-3.7 (4H, m)、4.99 (2H, s) | 263, 179 | |
| 068 | 2.35 (3H, s)、2.39 (3H, s)、2.46 (3H, s)、2.2-2.7 (6H, m)、3.4-3.7 (4H, m)、4.99 (2H, s) | 309, 179 | |
| 069 | 2.37 (3H, s)、2.41 (3H, s)、2.45 (3H, s)、3.3-3.6 (2H, m)、3.75 (2H, t, J≒4.7)、4.84 (2H, s)、6.86 (1H, bs) | 240, 179 | |
| 070 | 2.35 (3H×2, s)、2.40 (3H×2, s)、2.47 (3H×2, s)、3.6 (8H, bs)、5.02 (4H, s) | 443, 180<br>179 | |
| 071 | 2.21 (3H×2, s)、2.38 (3H, s)、2.42 (3H, s)、2.47 (3H, s)、3.1-3.5 (4H, m)、4.83 (2H, s)、7.0 (1H, bs) | 267 | |
| 072 | 2.37 (3H, s)、2.42 (3H, s)、2.48 (3H, s)、3.07 (2H, t, J≒6.1)、3.54 (2H, t, J≒6.1)、79 (2H, s)、6.8 (1H, bs)、7.0-7.4 (5H, m) | 332, 179 | |

| | | |
|---|---|---|
| 073 | 2.38 (3H, s), 2.42 (3H, s), 2.51 (3H, s), 2.3-2.6 (6H, m), 3.42 (2H, q, J≒5.7), 3.5-3.8 (4H, m), 4.84 (2H, s) | 309, 240, 179 |
| 074 | 1.3-1.7 (6H, m), 2.38 (3H, s), 2.42 (3H, s), 2.50 (3H, s), 2.2-2.5 (6H, m), 3.41 (2H, sext, J≒5.6), 3.76 (1H, t, J≒5.6), 4.84 (2H, s) | 307, 240, 179 |
| 075 | 2.36 (3H, s), 2.40 (3H, s), 2.47 (3H, s), 3.4-3.8 (8H, m), 4.99 (2H, s) | 266, 179 |
| 076 | 2.42 (3H, s), 2.46 (3H, s), 2.50 (3H, s), 2.3-2.6 (12H, m), 3.40 (2H, q, J≒6), 3.62 (2H, t, J≒5.2), 4.84 (2H, s) | 352 |
| 103 | 2.38 (3H, s), 2.42 (3H, s), 2.51 (3H, s), 2.3-2.6 (6H, m), 2.7-2.9 (4H, m), 3.41 (2H, q, J≒5.5), 4.84 (2H, s), 7.0 (1H, bs) | 308 |
| 105 | 2.26 (3H, s), 2.38 (3H, s), 2.43 (3H, s), 2.51 (3H, s), 3.2-3.5 (4H, m), 4.84 (2H, s), 7.0 (1H, bs) | 322, 113 |
| 135 | 2.37 (3H, s), 2.43 (3H, s), 2.51 (3H, s), 2.2-2.6 (10H, m), 3.2-3.5 (2H, m), 3.42 (2H, s), 4.83 (2H, s), 7.26 (4H, s) | 434, 432, 223, 125 |
| 136 | 2.26 (3H, s), 2.38 (3H, s), 2.50 (3H, s), 2.2-2.6 (10H, m), 3.2-3.5 (2H, m), 3.48 (2H, s), 4.83 (2H, s), 7.30 (5H, s) | 398, 304, 189 |
| 137 | 2.38 (3H, s), 2.44 (3H, s), 2.46 (3H, s), 2.3-2.6 (10H, m), 3.2-3.5 (2H, m), 3.60 (2H, s), 4.84 (2H, s), 6.9-7.5 (4H, m) | 434, 432, 223, 125 |
| 341 | 1.33 (3H, t, J=7.4), 1.74 (2H, br, s), 2.35 (3H, s), 2.40 (3H, s), 2.3-2.6 (4H, m), 2.92 (2H, q, J=7.4), 3.2-3.5 (2H, m), 3.5-3.7 (4H, m), 6.44 (1H, s), 7.0 (1H, br, s), 7.2-7.7 (5H, m) | 399 |
| 342 | 1.33 (3H, t, J=7.4), 1.77 (4H, br, s), 2.34 (3H, s), 2.40 (3H, s), 2.3-2.6 (6H, m), 2.91 (2H, q, J=7.4), 3.2-3.5 (2H, m), 3.49 (2H, s), 6.42 (1H, s), 7.0-7.7 (11H, m) | 488, 189 |
| 343 | 1.33 (3H, t, J=7.4), 1.6 (4H, br, s), 2.34 (3H, s), 2.40 (3H, s), 2.2-2.8 (6H, m), 2.92 (2H, q, J≒7.4), 3.2-3.5 (2H, m), 3.43 (2H, s), 6.42 (1H, s), 7.26 (1H, s), 7.2-7.6 (5H, m) | 524, 522, 223 |

| | | | |
|---|---|---|---|
| 344 | 1.34 (3H, t, J=7.4)、1.66 (4H, br. s)、2.35 (3H, s)、2.40 (3H, s)、2.2-2.6 (6H, m)、2.92 (2H, q, J=7.4)、3.2 3.5 (2H, m)、3.56 (2H, s)、6.43 (1H, s)、7.0 (1H, br. s)、7.2-7.7 (7H, m)、8.18 (2H, d, J=8.8) | 533, 234 | |
| 345 | 1.34 (3H, t, J=7.4)、1.90 (2H, br. s)、2.34 (3H, s)、2.41 (3H, s)、2.2-2.6 (8H, m)、2.92 (2H, q, J=7.4)、3.2 3.5 (2H, m)、3.55 (2H, s)、6.43 (1H, s)、7.0-7.7 (9H, m) | 560, 558 556, 258 256 | |
| 346 | 1.34 (3H, t, J=7.4)、1.73 (2H, br. s)、2.35 (3H, s)、2.38 (3H, s)、2.3-2.6 (6H, m)、2.88 (2H, q, J=7.4)、3.0 3.2 (2H, m)、3.3-3.5 (2H, m)、6.45 (1H, s)、6.7-7.7 (10H, m) | 474, 330 175 | |
| 347 | 1.31 (3H, t, J=7.4)、1.6-2.1 (4H, m)、2.34 (3H, s)、2.40 (3H, s)、2.2-2.8 (8H, m)、2.90 (2H, q, J=7.4)、3.2 3.5 (2H, m)、3.54 (2H, s)、6.43 (1H, s)、6.8-7.7 (10H, m) | 521, 520 221 | |
| 348 | 1.31 (3H, t, J=7.4)、1.6-2.1 (2H, m)、2.34 (3H, s)、2.40 (3H, s)、2.4-2.8 (12H, m)、3.1-3.5 (2H, m)、3.54 (2H, s)、6.43 (1H, s)、7.26 (4H, s)、7.2-7.6 (5H, m) | 538, 536 239, 237 | |
| 376 | 1.23 (3H×2, t, J≒7.6)、1.4-2.2 (8H, m)、2.2 3.6 (10H, m)、4.21 (4H, s)、4.83 (2H, s)、6.51 (1H, br. s)、7.0 7.4 (10H, m) | 487, 174 | 0.44 |
| 377 | 1.22 (3H, t, J≒7.6)、1.26 (3H, t, J-7.6)、1.6-2.0 (2H, m)、2.2-2.5 (8H, m)、2.69 (2H, q, J≒7.6)、2.77 (2H, q, J≒7.6)、3.1-3.6 (2H, m)、3.36 (2H, s)、4.18 (2H, s)、4.81 (2H, s)、6.4 (1H, br. s)、6.8-7.4 (9H, m) | 520, 207 | 0.38 |
| 379 | 1.22 (3H, t, J≒7.6)、1.26 (3H, t, J-7.6)、1.6-2.0 (2H, m)、2.2-3.0 (14H, m)、3.0-3.6 (2H, m)、3.36 (2H, s)、4.18 (2H, s)、4.81 (2H, s)、6.4 (1H, br. s)、7.0-7.4 (9H, m) | 538, 536 225, 223 | 0.40 |
| 381 | 1.23 (3H×2, t, J≒7.6)、1.6-1.8 (2H, m)、2.2-3.0 (14H, m)、3.0-3.5 (2H, m)、4.19 (2H, s)、4.84 (2H, s)、6.51 (1H, br. s)、6.8-7.4 (9H, m) | 524, 522 | 0.62 |

| | | | |
|---|---|---|---|
| 382 | 1.22 (3H, t, J≒7.6)、1.25 (3H×2, t, J≒7.6)、1.4-2.0 (2H, m)、2.2-3.0 (14H, m)、3.0-3.4 (2H, m)、3.50 (2H, s)、4.07 (2H, s)、4.81 (2H, s)、6.4 (1H, br, s)、7.0-7.4 (9H, m) | 552, 550: 343, 237 | 0.37 |
| 491 | 1.23 (3H, t, J≒7.6)、2.32 (3H×2)、2.3-3.0 (8H, m)、3.6-3.8 (4H, m)、4.0-4.3 (2H, m) | 266, 114 | 0.33 |
| 492 | 1.23 (3H, t, J≒7.4)、1.3-2.0 (6H, m)、2.31 (3H, s)、2.33 (3H, s)、2.5-3.0 (8H, m)、4.2-4.5 (2H, m) | 264, 179 | 0.28 |
| 493 | 0.9-1.2 (3H, m)、1.23 (3H, t, J≒7.4)、1.3-2.0 (5H, m)、2.31 (3H, s)、2.33 (3H, s)、2.5-3.0 (8H, m)、4.2-4.5 (2H, m) | 278, 111 | 0.29 |
| 494 | 1.23 (3H, t, J≒7.4)、2.30 (3H×2, s)、2.31 (3H, s)、2.3-3.0 (12H, m)、4.0-4.3 (2H, m) | 279, 113 | 0.15 |
| 495 | 1.22 (3H, t, J≒7.4)、2.31 (3H×2, s)、2.3-3.0 (14H, m)、3.62 (2H, t, J=5.4)、4.0-4.3 (2H, m) | 309, 143 | 0.06 |
| 496 | 1.23 (3H, t, J≒7.4)、2.31 (3H×2, s)、2.3-3.0 (14H, m)、3.5-3.8 (6H, m)、4.0-4.3 (3H, m) | 353, 187 | 0.11 |
| 497 | 1.22 (3H, t, J≒7.4)、2.31 (3H×2, s)、2.3-3.0 (12H, m)、3.52 (2H, s)、4.0-4.3 (2H, m)、7.30 (5H, s) | 355, 189 | 0.38 |
| 498 | 1.22 (3H, t, J≒7.4)、2.31 (3H×2, s)、2.3-3.0 (12H, m)、3.48 (2H, s)、4.0-4.3 (2H, m)、7.29 (4H, s) | 391, 389 223 | 0.36 |
| 499 | 1.22 (3H, t, J≒7.4)、2.31 (3H×2, s)、2.3-3.0 (12H, m)、3.46 (2H, s)、3.79 (3H, s)、4.0-4.3 (2H, m)、6.84 (2H, d, J=8.6)、7.22 (2H, d, J=8.6) | 385, 219 | 0.33 |
| 500 | 1.22 (3H, t, J≒7.4)、2.31 (3H×2, s)、2.3-3.0 (12H, m)、3.47 (2H, s)、4.0-4.3 (2H, m)、6.8-7.4 (4H, m) | 373, 207 | 0.35 |
| 501 | 1.22 (3H, t, J≒7.4)、2.31 (3H, s)、2.33 (3H, s)、2.3-3.0 (12H, m)、3.59 (2H, s)、4.0-4.2 (2H, m)、7.50 (2H, d, J=8.8)、8.17 (2H, d, J= | 400, 234 | 0.39 |

| | | | |
|---|---|---|---|
| | 8. 8) | | |
| 502 | 1. 23 (3H, t, J≒7. 4)、1. 6−2. 0 (2H, m)、2. 31 (3H, s)、2. 33 (3H, s)、2. 5−3. 0 (12H, m)、3. 72 (2H, s)、4. 0−4. 3 (2H, m)、7. 51 (2H, d, J≒8. 8)、8. 19 (2H, d, J≒8. 8) | 414, 248 | 0. 23 |
| 503 | 1. 23 (3H, t, J≒7. 4)、1. 6−2. 0 (2H, m)、2. 31 (3H×2, s)、2. 5−3. 0 (12H, m)、3. 60 (2H, s)、4. 0−4. 3 (2H, m)、6. 8 7. 4 (4H, m) | 387 | 0. 20 |
| 504 | 1. 22 (3H, t, J≒7. 4)、1. 6−2. 0 (2H, m)、2. 31 (3H×2, s)、2. 5−3. 0 (12H, m)、7. 27 (4H, s) | 405, 403 237 | 0. 23 |
| 511 | 2. 31 (3H×2, s)、2. 4−2. 7 (6H, m)、3. 6 3. 8 (4H, m)、4. 0−4. 3 (2H, s)、7. 1−7. 5 (5H, m) | 328, 114 | 0. 41 |
| 512 | 2. 30 (3H×2, s)、2. 3−2. 7 (10H, m)、3. 51 (2H, s)、4. 0−4. 2 (2H, m)、4. 08 (2H, s)、7. 1−7. 5 (10H, m) | 417, 202 189 | 0. 37 |
| 516 | 1. 6−2. 0 (2H, m)、2. 30 (3H×2, s)、2. 5 3. 0 (10H, m)、3. 58 (2H, s)、3. 9−4. 2 (2H, m)、4. 08 (2H, s)、7. 1−7. 5 (10H, m) | 467, 465 251, 237 | 0. 24 |
| 536 | 2. 30 (3H, s)、2. 34 (3H, s)、2. 41 (3H, s)、2. 4 2. 7 (6H, m)、3. 6−3. 8 (4H, m)、4. 0−4. 3 (2H, m) | 252, 101 | 0. 30 |
| 537 | 1. 2−1. 8 (6H, m)、2. 30 (3H, s)、2. 36 (3H, s)、2. 41 (3H, s)、2. 4−2. 8 (6H, m)、4. 1−4. 3 (2H, m) | 250, 111 | 0. 20 |
| 538 | 2. 29 (3H, s)、2. 32 (3H, s)、2. 40 (3H, s)、2. 3 2. 8 (10H, m)、3. 52 (2H, s)、4. 0−4. 3 (2H, m)、7. 30 (5H, s) | 341, 189 | 0. 27 |
| 539 | 2. 30 (3H, s)、2. 32 (3H, s)、2. 41 (3H, s)、2. 3 2. 8 (10H, m)、3. 47 (2H, s)、4. 0−4. 3 (2H, m)、7. 26 (4H, s) | 377, 375 223 | 0. 27 |
| 540 | 2. 30 (3H, s)、2. 33 (3H, s)、2. 41 (3H, s)、2. 4−2. 8 (10H, m)、3. 48 (2H, s)、4. 0−4. 3 (2H, m)、6. 8−7. 4 (4H, m) | 359, 220 207 | 0. 20 |
| 541 | 1. 6−2. 0 (2H, m)、2. 30 (3H, s)、2. 32 (3H, s)、2. 41 (3H, s)、2. 5−3. 0 (10H, m)、3. 60 (2H, s)、4. 0−4. 3 (2H, m)、7. 26 (4H, s) | 391, 389 237, 165 | 0. 17 |

| | | | |
|---|---|---|---|
| 544 | 1.20 (3H×2, d, J=6.8)、2.32 (3H×2, s)、2.4 2.8 (6H, m)、3.42 (1H, sept, J=6.8)、3.5−3.8 (4H, m)、4.0 4.3 (2H, m) | 280, 114 | 0.33 |
| 546 | 1.20 (3H×2, d, J=6.8)、2.30 (3H×2, s)、2.2−2.8 (10H, m)、3.42 (1H, sept, J=6.8)、3.47 (2H, s)、3.9−4.2 (2H, m)、7.27 (4H, s) | 405, 403 | 0.33 |
| 547 | 1.20 (3H×2, d, J=6.8)、1.6−2.0 (2H, m)、2.30 (3H×2, s)、2.5−3.0 (10H, m)、3.42 (1H, sept, J=6.8)、3.60 (2H, s)、3.9−4.2 (2H, m)、7.27 (4H, s) | 419, 417 237 | 0.23 |
| 550 | 1.0−1.3 (12H, m)、1.3−1.8 (6H, m)、2.3 2.8 (10H, m)、3.42 (1H, sept, J=6.8)、4.0−4.3 (2H, m) | 306, 112 | 0.30 |
| 551 | 0.91 (3H, t, J≒6)、1.20 (3H×2, d, J 6.8)、1.21 (3H×2, d, J=7.6)、1.2−1.6 (4H, m)、2.3−2.8 (16H, m)、3.42 (1H, sept, J=6.8)、4.0−4.2 (2H, m) | 363, 155 | 0.25 |
| 552 | 1.20 (3H×2, d, J=6.8)、1.21 (3H×2, d, J 7.6)、2.3−2.8 (14H, m)、3.42 (1H, sept, J=6.8)、3.51 (2H, s)、4.0 4.2 (2H, m)、7.30 (5H, s) | 397, 189 | 0.35 |
| 553 | 1.20 (3H×2, d, J=6.8)、1.21 (3H×2, d, J 7.6)、1.6−2.0 (2H, m)、2.4−3.0 (14H, m)、3.42 (1H, sept, J=6.8)、3.59 (2H, s)、3.80 (3H, s)、3.9−4.2 (2H, m)、6.84 (2H, d, J=8.8)、7.24 (2H, d, J=8.8) | 441, 233 | 0.21 |
| 556 | 0.94 (3H×2, d, J=6.7)、1.20 (3H, t, J 7.6)、1.21 (3H, t, J=7.6)、2.0−2.4 (1H, m)、2.4−3.0 (16H, m)、3.53 (2H, s)、4.0−4.3 (2H, m)、7.30 (5H, s) | 411, 189 | 0.32 |
| 558 | 0.94 (3H×2, d, J=6.7)、1.20 (3H, t, J 7.6)、1.21 (3H, t, J=7.6)、1.6−2.0 (2H, m)、2.0−2.4 (1H, m)、2.4 3.0 (16H, m)、3.60 (2H, s)、3.80 (3H, s)、3.9−4.2 (2H, m)、6.84 (2H, d, J 8.6)、7.25 (2H, d, J=8.6) | 455, 246 121 | 0.17 |
| 648 | 2.32 (3H, s)、2.35 (3H×2, s)、3.3−3.5 (2H, m)、4.2−4.4 (2H, m)、4.99 (2H, s)、8.0 (1H, br, s) | 304, 302 222, 179 | |
| 649 | 2.38 (3H, s)、2.43 (3H, s)、2.48 (3H, s)、3.6 3.8 (4H, m)、4.85 (2H, | 260, 258 | |

| | | | |
|---|---|---|---|
| | s)、6.8 (1H, br. s) | | |
| 650 | 0.7−2.0 (10H, m)、2.1−2.5 (1H, m)、2.37 (3H, s)、2.42 (3H, s)、2.48 (3H, s)、2.81 (2H, t, J=5.8)、3.2−3.5 (3H, m)、4.84 (2H, s)、7.1 (1H, br. s) | 321 | |
| 651 | 1.29 (3H, t, J≒7)、2.36 (3H, s)、2.41 (3H, s)、2.87 (2H, q, J≒7)、3.69 (3H, s)、6.14 (1H, s)、7.3−7.7 (5H, m) | 301, 121 | |
| 652 | 1.22 (3H, t, J≒7.6)、2.31 (3H, s)、2.37 (3H, s)、2.83 (2H, q, J≒7.6)、5.65 (1H, br. s)、6.14 (1H, s)、7.2−7.6 (5H, m) | 287 | |
| 653 | 1.22 (3H/2, t, J≒7.4)、1.27 (3H/2, t, J≒7.4)、2.72 (1H, q, J=7.4)、2.80 (1H, q, J≒7.4)、3.2−3.5 (4H, m)、4.17 (2H, s)、4.81 (2H, s)、6.9 (1H, br. s)、7.0−7.4 (5H, m) | 364, 362<br>241, 146 | |
| 654 | 1.19 (3H, t, J=5.7)、1.23 (3H, t, J=5.7)、2.65 (2H, q, J=5.7)、2.73 (2H, q, J=5.7)、3.73 (3H, s)、4.16 (2H, s)、4.85 (2H, s)、7.0−7.5 (5H, m) | 315 | |
| 655 | 1.19 (3H, t, J=7.6)、1.21 (3H, t, J=7.6)、2.65 (2H, q, J=7.6)、2.75 (2H, q, J=7.6)、4.16 (2H, s)、4.88 (2H, s)、6.98 (1H, br. s)、7.0−7.4 (5H, m) | 301 | |
| 656 | 1.22 (3H, t, J=7.4)、1.27 (3H, t, J=7.4)、2.72 (2H, q, J=7.4)、2.80 (2H, q, J=7.4)、3.2−3.5 (4H, m)、4.17 (2H, s)、4.81 (2H, s)、6.91 (1H, br. s)、7.0−7.4 (5H, m) | 364, 362<br>241, 146 | |
| 657 | 1.23 (3H, t, J≒7.6)、1.26 (3H, t, J≒7.6)、1.6−1.8 (2H, m)、2.34 (2H×2, J≒4.7)、2.70 (2H, q, J≒7.6)、2.78 (2H, q, J≒7.6)、3.0−3.4 (2H, m)、3.4−3.6 (4H, m)、4.18 (2H, s)、4.83 (2H, s)、6.31 (1H, br. s)、7.0−7.4 (5H, m) | 413, 102 | 0.50 |
| 658 | 1.23 (3H, t, J=7.6)、1.4−2.0 (2H, m)、2.2−3.6 (10H, m)、4.21 (2H, s)、4.83 (2H, s)、6.51 (1H, br. s)、70.−7.4 (10H, m) | 487, 174 | 0.44 |

| | | | |
|---|---|---|---|
| 662 | 1.23 (3H, t, J≒7.6)、2.39 (3H×2, s)、2.83 (2H, q, J≒7.4)、3.75 (3H, s) 4.88 (2H, s) | 225 | |
| 663 | 2.33 (3H, s)、2.41 (3H, s)、3.73 (3H, s)、4.15 (2H, s)、4.87 (2H, s)、7.0−7.4 (5H, m) | 287 | |
| 664 | 1.27 (3H×2, d, J=6.8)、2.32 (3H, s)、2.40 (3H, s)、3.34 (1H, sept, J=6.8)、3.75 (3H, s)、4.90 (2H, s) | 239 | |
| 665 | 1.23 (3H, t, J=7.6)、1.24 (3H, t, J=7.6)、1.28 (3H×2, d, J=6.8)、2.65 (2H, q, J=7.6)、2.73 (2H, q, J=7.6)、3.34 (1H, sept, J=6.8)、3.74 (3H, s)、4.87 (2H, s) | 267 | 0.89 |
| 666 | 0.94 (3H×2, d, J=6.7)、1.20 (3H, t, J≒7.3)、1.21 (3H, t, J≒7.3)、2.18 (1H, sept, J=7.3)、2.5−2.9 (6H, m)、3.74 (3H, s)、4.86 (2H, s) | 281 | 0.68 |
| 679 | 2.38 (3H, s)、2.41 (3H×2, s)、3.64 (1H, t, J≒5.5)、3.8−4.1 (3H, m)、4.4−4.6 (2H, m) | 183 | 0.59 |
| 680 | 1.23 (3H, t, J≒7.4)、2.32 (3H×2, s)、2.78 (2H, q, J≒7.4)、3.8−4.1 (3H, m)、4.4−4.6 (2H, m) | 197 | 0.64 |
| 681 | 2.38 (3H, s)、2.45 (3H, s)、2.6−2.8 (1H, br, m)、3.7−3.9 (2H, m)、4.11 (2H, s)、4.3−4.5 (2H, m)、7.1−7.4 (5H, m) | 259 | 0.91 |
| 682 | 1.24 (3H×2, d, J≒6.8)、2.37 (3H, s)、2.42 (3H, s)、3.29 (1H, sept, J≒6.8)、3.6−4.0 (2H, m)、4.3−4.5 (2H, m) | 211 | 0.57 |
| 683 | 1.1−1.4 (12H, m)、2.70 (2H, q, J=7.6)、3.30 (1H, sept, J=6.8)、3.8−4.1 (3H, m)、4.4−4.6 (2H, m) | 239 | 0.63 |
| 684 | 0.93 (3H×2, d, J≒6.7)、1.20 (3H, t, J≒7.3)、1.26 (3H, t, J≒7.3)、2.13 (1H, sept, J≒7.3)、2.6−2.9 (6H, m)、3.67 (1H, t, J≒5.3)、3.8−4.1 (3H, m)、4.4−4.6 (2H, m) | 253 | 0.71 |
| 697 | 0.91 (3H, t, J≒6)、1.20 (3H×2, d, J=6.8)、1.2−1.6 (4H, m)、2.2−2.8 (12H, m)、2.31 (3H×2, q)、3.43 (1H, sept, J=6.8)、4.0−4.3 (2H, m) | 345 | 0.18 |

| | | | |
|---|---|---|---|
| 810 | 0. 91 (3H. t)、1. 1−1. 6 (4H. m)、2. 30 (3H. s)、2. 34 (3H. s)、2. 41 (3H. s)、4. 15 (2H. t)、2. 3−2. 7 (12H. m) | 307 | 0. 26 |
| 812 | 0. 91 (3H. t)、1. 23 (3H. t)、1. 1−1. 6 (4H. m)、2. 31 (6H. s)、2. 3−2. 9 (14H. m)、4. 14 (2H. t) | 321 | 0. 19 |
| 814 | 0. 91 (3H. t)、1. 20 (6H. t)、1. 1−1. 7 (4H. m)、2. 31 (6H. s)、2. 3−2. 9 (12H. m)、3. 42 (1H. sept)、4. 14 (2H. t) | 335 | 0. 22 |
| 822 | 2. 30 (3H. s)、2. 33 (3H. s)、2. 41 (3H. s)、2. 3−2. 7 (12H. m)、3. 62 (2H. t)、4. 15 (3H. t) | 295 | 0. 02 |
| 825 | 1. 20 (6H. d)、2. 31 (6H. s)、2. 1−2. 9 (12H. m)、3. 42 (1H. sept)、3. 62 (2H. t)、4. 14 (2H. t) | 323 | 0. 06 |
| 826 | 1. 21 (6H. d)、1. 21 (6H. t)、2. 0−2. 9 (12H. m)、3. 42 (1H. sept)、3. 62 (2H. t)、4. 13 (2H. t) | 351 | 0. 04 |
| 834 | 1. 20 (6H. d)、1. 3−1. 8 (6H. m)、2. 31 (3H. s)、2. 34 (3H. s)、2. 4−2. 7 (6H. m)、3. 42 (1H. sept)、4. 21 (2H. t) | 278 | 0. 22 |
| 842 | 1. 04 (6H. t)、2. 30 (3H. s)、2. 35 (3H. s)、2. 41 (3H. s)、2. 5−2. 8 (6H. m)、4. 11 (2H. t) | 238 | 0. 63 |
| 844 | 1. 04 (6H. t)、1. 23 (3H. t)、2. 31 (3H. s)、2. 34 (3H. s)、2. 3−2. 9 (8H. m)、4. 11 (2H. t) | 252 | 0. 46 |
| 846 | 1. 09 (6H. t)、1. 20 (6H. d)、2. 31 (3H. s)、2. 34 (3H. s)、2. 4−2. 9 (6H. m)、3. 43 (1H. sept)、4. 14 (2H. t) | 266 | 0. 63 |
| 848 | 0. 99−1. 27 (18H. m)、2. 4−2. 9 (10H. m)、3. 44 (1H. sept)、4. 13 (2H. t) | 294 | 0. 27 |

TABLE 2C physical properties of 2-hydroxy-3-substituted-5, 6, 7, 8-tetrahydroquinoxakine

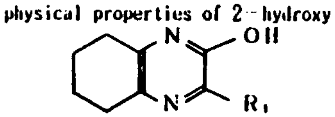

| $R_1$ | NMR ($CDCl_3$ 中, inner standard TMS) δ ppm | MASS |
|---|---|---|
| Me | 1. 78-1. 84 (4H, m), 2. 42 (3H, s), 2. 66 (4H, m), 13. 05 (1H, br, s) | 165 |
| Et | 1. 25 (3H, t), 1. 7-1. 9 (4H, m), 2. 5-2. 8 (4H, m), 2. 80 (2H, q), 13. 06 (1H, br, s) | 179 |
| Pro | 1. 00 (4H, t), 1. 55 (6H, m), 2. 67-2. 83 (6H, m), 13. 09 (1H, br, s) | 193 |
| iso-Pro | 1. 24 (3H×2, d×2, J=6. 8), 1. 7-2. 0 (4H, m), 2. 5-2. 8 (4H, m), 3. 41 (1H, sept, J=6. 8), 12. 9 (1H, br, s) | 193 |
| Bu | 0. 94 (3H, t), 1. 24-2. 00 (8H, m), 2. 55-2. 85 (6H, m), 12. 99 (1H, br, s) | 207 |
| iso-Bu | 0. 96 (3H×2, d), 1. 6-2. 0 (4H, m), 2. 0-2. 4 (1H, m), 2. 5-2. 8 (6H, m) | 207 |
| sec-Bu | 0. 96 (3H×2, d×2, J=6. 7), 1. 6-2. 2 (5H, m), 2. 5-2. 8 (6H, m) | 207 |
| $-(CH_2)_4CH_3$ | 0. 90 (3H, t, J=6. 3), 1. 2-1. 5 (4H, m), 1. 5-1. 9 (6H, m), 2. 5-2. 9 (6H, m) | 221, 164 |
| $-(CH_2)_5CH_3$ | 0. 88 (3H, t, J=7. 9), 1. 1-1. 5 (6H, m), 1. 5-2. 0 (6H, m), 2. 4-2. 9 (6H, m) | 235 |
| $-(CH_2)_6CH_3$ | 0. 88 (3H, t, J≒7. 4), 1. 1-1. 5 (8H, m), 1. 5-2. 0 (6H, m), 2. 5-2. 9 (6H, m) | 249, 164 |
| $-(CH_2)_7CH_3$ | 0. 88 (3H, t, J=7. 5), 1. 1-1. 5 (10H, m), 1. 5-2. 0 (6H, m), 2. 5-2. 9 (6H, m) | 263 |
| $-(CH_2)_8CH_3$ | 0. 88 (3H, t, J=6. 2), 1. 1-1. 5 (12H, m), 1. 5-2. 0 (6H, m), 2. 5-2. 8 (6H, m) | 277, 164 |
| $-(CH_2)_9CH_3$ | 0. 88 (3H, t, J≒6), 1. 2-2. 0 (20H, m), 2. 5-2. 9 (6H, m), 12. 7 (1H, br, s) | 291 |
| $-(CH_2)_{11}CH_3$ | 0. 88 (3H, t, J≒6), 1. 1-1. 9 (24H, m), 2. 4-2. 8 (6H, m) | 319, 164 |
| $-(CH_2)_{13}CH_3$ | 0. 88 (3H, t, J≒6), 1. 1-1. 9 (28H, m), 2. 5-2. 8 (6H, m) | 347, 164 |
| $-(CH_2)_{15}CH_3$ | 0. 88 (3H, t, J≒6), 1. 1-1. 9 (32H, m), 2. 5-2. 8 (6H, m) | 375, 164 |
| $-CH_2Ph$ | 1. 6-2. 0 (4H, m), 2. 4-2. 7 (4H, m), 4. 02 (2H, s), 7. 0-7. 4 (4H, m) | 241 |
| $-CH_2CH_2Ph$ | 1. 6-2. 0 (4H, m), 2. 5-2. 8 (4H, m), 3. 06 (2H×2, s), 7. 1-7. 3 (5H, s), 12. 9 (1H, br, s) | 255 |

TABLE 27 physical properties of a compound of the present invention

| Comp. No. | NMR ($CDCl_3$, inner standard TMS) δ ppm | MASS | Rf |
|---|---|---|---|
| 368 | 1.7−2.0 (4H, m), 2.2−3.0 (14H, m), 3.0−3.6 (2H, m), 3.40 (2H, s), 4.17 (2H, s), 4.79 (2H, s), 6.4 (1H, br, s), 7.0−7.4 (10H, m) | 500, 189 | 0.45 |
| 369 | 1.6−2.0 (4H, m), 2.1−3.0 (14H, m), 3.0−3.6 (2H, m), 3.34 (2H, s), 3.79 (3H, s), 4.17 (2H, s), 4.79 (2H, s), 6.4 (1H, br, s), 6.7−7.4 (9H, m) | 530, 219 | 0.43 |
| 370 | 1.6−2.0 (4H, m), 2.1−3.0 (14H, m), 3.0−3.6 (2H, m), 3.35 (2H, s), 4.18 (2H, s), 4.79 (2H, s), 6.3 (1H, br, s), 7.0−7.4 (9H, m) | 535, 533 223 | 0.44 |
| 371 | 1.6−2.0 (4H, m), 2.3−3.0 (10H, m), 3.0−3.4 (6H, m), 4.19 (2H, s), 4.82 (2H, s), 6.4 (1H, br, s), 6.7−7.4 (9H, m) | 522, 520 209 | 0.48 |
| 372 | 1.5−2.1 (6H, m), 2.3−3.0 (14H, m), 3.0−3.4 (2H, m), 3.51 (2H, s), 4.17 (2H, s), 4.79 (2H, s), 6.3 (1H, br, s), 6.9−7.4 (9H, m) | 532, 221 | 0.38 |
| 373 | 1.6−2.1 (2H, m), 2.2−3.0 (10H, m), 3.0−3.4 (4H, m), 4.15 (2H, s), 4.81 (2H, s), 6.3 (1H, br, s), 6.6−7.4 (10H, m) | 486, 342 175 | 0.49 |
| 517 | 0.93 (3H, t, J≒6.6), 1.2−2.0 (8H, m), 2.4−3.0 (12H, m), 3.6−3.8 (4H, m), 3.9−4.2 (2H, m) | 320, 102 | 0.42 |
| 518 | 0.93 (3H, t, J≒6.6), 1.2−2.0 (8H, m), 2.4−3.0 (16H, m), 3.53 (2H, s), 3.9−4.2 (2H, m), 7.30 (5H, s) | 409, 189 | 0.41 |
| 519 | 0.93 (3H, t, J≒6.6), 1.2−2.0 (8H, m), 2.4−3.0 (16H, m), 3.47 (2H, s), 3.9−4.2 (2H, m), 7.27 (4H, s) | 445, 443 227 | 0.43 |
| 520 | 0.93 (3H, t, J≒6.6), 1.2−2.0 (10H, m), 2.4−3.0 (16H, m), 3.60 (2H, s), 3.9−4.2 (2H, m), 7.27 (4H, s) | 459, 457 237 | 0.29 |
| 521 | 0.93 (3H, t, J≒6.6), 1.2−2.0 (8H, m), 2.4−3.0 (12H, m), 3.0−3.3 (4H, m), 4.0−4.3 (2H, m), 6.7−7.4 (5H, m) | 395, 175 | 0.61 |

| | | | |
|---|---|---|---|
| 522 | 0. 93 (3H. t. J≒6. 6)、1. 2−2. 0 (8H. m)、2. 5−3. 0 (12H. m)、3. 2−3. 2 (4H. m)、3. 86 (3H. s)、4. 0−4. 3 (2H. m)、6. 7−7. 1 (4H. m) | 425. 205 | 0. 69 |
| 559 | 0. 89 (3H. t. J≒6)、1. 2−2. 0 (10H. m)、2. 4−2. 9 (12H. m)、3. 6−3. 8 (4H. m)、3. 9−4. 2 (2H. m) | 334. 102 | 0. 44 |
| 560 | 0. 89 (3H. t. J≒6)、0. 91 (3H. t. J≒6)、1. 2−2. 0 (14H. m)、2. 2−2. 9 (18H. m)、3. 9−4. 2 (2H. m) | 389. 155 | 0. 24 |
| 561 | 0. 89 (3H. t. J≒6)、1. 1−2. 0 (10H. m)、2. 4−2. 9 (16H. m)、3. 53 (2H. s)、3. 9−4. 2 (2H. m)、7. 30 (5H. s) | 423. 189 | 0. 31 |
| 562 | 0. 89 (3H. t. J≒6)、1. 1−2. 0 (12H. m)、2. 4−2. 9 (16H. m)、3. 61 (2H. s)、3. 9−4. 2 (2H. m)、6. 8−8. 4 (4H. m) | 455. 234<br>221 | 0. 19 |
| 563 | 0. 89 (3H. t. J≒6)、1. 2−2. 0 (10H. m)、2. 5−2. 9 (12H. m)、3. 0−3. 2 (4H. m)、3. 86 (3H. s)、4. 0−4. 3 (2H. m)、6. 7−7. 1 (4H. m) | | |
| 564 | 0. 87 (3H. t. J≒6)、1. 1−2. 0 (12H. m)、2. 4−2. 9 (12H. m)、3. 6−3. 8 (4H. m)、3. 9−4. 2 (2H. m) | 348. 101 | 0. 32 |
| 565 | 0. 87 (3H. t. J≒6)、0. 91 (3H. t. J≒6)、1. 1−2. 0 (16H. m)、2. 1−2. 9 (18H. m)、3. 9−4. 2 (2H. m) | 403. 155 | 0. 20 |
| 567 | 0. 87 (3H. t. J≒6)、1. 1−2. 0 (12H. m)、2. 3−2. 9 (16H. m)、3. 52 (2H. s)、2. 9−4. 2 (2H. m)、7. 30 (5H. s) | 437. 189 | 0. 28 |
| 568 | 0. 87 (3H. t. J≒6)、1. 1−2. 0 (12H. m)、2. 4−2. 9 (16H. m)、3. 52 (2H. s)、3. 9−4. 2 (2H. m)、7. 30 (4H. s) | 473. 471<br>236. 223 | 0. 31 |
| 569 | 0. 87 (3H. t. J≒6)、1. 1−2. 0 (14H. m)、2. 4−3. 0 (16H. m)、3. 60 (2H. s)、3. 9−4. 2 (2H. m)、6. 8−7. 4 (4H. m) | 469. 234<br>221 | 0. 20 |
| 570 | 0. 87 (3H. t. J≒6)、1. 1−2. 0 (12H. m)、2. 5−3. 0 (12H. m)、2. 9−3. 2 (4H. 3. 86 (3H. s)、4. 0−4. 3 (2H. m)、6. 7−7. 1 (4H. m) | 453. 218<br>205 | 0. 53 |
| 571 | 0. 7−1. 0 (6H. m)、1. 1−2. 0 (18H. m)、2. 2−2. 9 (18H. m)、3. 9−4. 2 (2H. m) | 417. 155 | 0. 33 |

| | | | |
|---|---|---|---|
| 572 | 0.87 (3H, t, J≈6)、1.1−2.0 (16H, m)、2.4−2.9 (12H, m)、3.6−3.8 (4H, m)、3.9−4.2 (2H, m) | 376 | 0.58 |
| 573 | 0.7−1.0 (6H, m)、1.1−2.0 (20H, m)、2.2−2.9 (18H, m)、3.9−4.2 (2H, m) | 431, 155 | 0.27 |
| 574 | 0.87 (3H, t, J≈6)、1.1−2.0 (16H, m)、2.4−2.9 (16H, m)、3.53 (2H, s)、3.9−4.2 (2H, m)、7.30 (5H, s) | 465, 189 | 0.52 |
| 575 | 0.87 (3H, t, J≈6)、1.1−2.0 (16H, m)、2.3−2.9 (16H, m)、3.9−4.2 (2H, m)、7.26 (4H, s) | 501, 499, 223 | 0.43 |
| 576 | 0.87 (3H, t, J≈6)、1.1−2.0 (18H, m)、2.5−2.9 (16H, m)、3.59 (2H, s)、3.9−4.2 (2H, m)、6.8−7.4 (4H, m) | 497, 235, 221 | 0.24 |
| 577 | 0.88 (3H, t, J≈6)、1.1−2.0 (16H, m)、2.5−2.9 (12H, m)、3.0−3.3 (4H, m)、4.0−4.3 (2H, m)、6.7−7.4 (5H, m) | 450, 189, 175 | 0.67 |
| 578 | 0.87 (3H, t, J≈6)、1.1−2.0 (16H, m)、2.5−2.9 (12H, m)、3.0−3.2 (4H, m)、3.77 (3H, s)、4.0−4.2 (2H, m)、6.86 (4H, s) | 480, 218, 205 | 0.55 |
| 579 | 0.87 (3H, t, J≈6)、1.0−2.0 (18H, m)、2.4−2.9 (12H, m)、3.5−3.8 (4H, m)、3.9−4.2 (2H, m) | 390, 102 | 0.39 |
| 580 | 0.87 (3H, t, J≈6)、0.91 (3H, t, J≈6)、1.0−2.0 (22H, m)、2.3−2.9 (18H, m)、3.9−4.2 (2H, m) | 445, 155 | 0.29 |
| 581 | 0.87 (3H, t, J≈6)、1.0−2.0 (18H, m)、2.4−2.9 (16H, m)、3.52 (2H, s)、3.9−4.2 (2H, m) | 479, 202, 189 | 0.32 |
| 582 | 0.87 (3H, t, J≈6)、1.0−2.0 (18H, m)、2.3−2.9 (16H, m)、3.47 (2H, s)、3.80 (3H, s)、3.9−4.2 (2H, m)、6.84 (2H, d, J=8.8)、7.23 (2H, d, J=8.8) | 509, 232, 219 | 0.28 |
| 583 | 0.87 (3H, t, J≈6)、1.0−2.0 (20H, m)、2.4−3.0 (16H, m)、3.59 (2H, s)、3.9−4.2 (2H, m)、7.26 (4H, s) | 529, 527, 250, 237 | 0.39 |
| 584 | 0.87 (3H, t, J≈6)、1.0−2.0 (18H, m)、2.4−2.9 (12H, m)、3.0−3.2 (4H, m)、3.9−4.2 (2H, m)、6.7−7.3 (4H, m) | 501, 499, 222, 209 | 0.46 |

| | | | |
|---|---|---|---|
| 585 | 0. 87 (3H. t. J≒6)、1. 0−2. 0 (20H. m)、2. 4−2. 9 (12H. m)、3. 5−3. 8 (4H. m)、3. 9−4. 2 (2H. m) | 404. 113<br>102 | 0. 37 |
| 586 | 0. 87 (3H. t. J≒6)、1. 0−2. 0 (26H. m)、2. 4−2. 9 (12H. m)、4. 0−4. 3 (4H. m) | 402. 112 | 0. 29 |
| 587 | 0. 87 (3H. t. J≒6)、0. 91 (3H. t. J≒6)、1. 0−2. 0 (24H. m)、2. 2−2. 9 (18H. m)、3. 9−4. 2 (2H. m) | 459. 155<br>125 | 0. 26 |
| 588 | 0. 87 (3H. t. J≒6)、1. 0−2. 0 (20H. m)、2. 3−2. 9 (18H. m)、3. 62 (2H. t. J≒6)、3. 9−4. 2 (2H. m) | 447. 156<br>143. 125 | 0. 12 |
| 589 | 0. 87 (3H. t. J≒6)、1. 0−2. 0 (20H. m)、2. 4−2. 9 (16H. m)、3. 52 (2H. s)、3. 9−4. 2 (2H. m)、7. 30 (5H. s) | 493. 202<br>189 | 0. 37 |
| 590 | 0. 87 (3H. t. J≒6)、1. 0−2. 0 (20H. m)、2. 3−2. 9 (16H. m)、3. 52 (2H. s)、3. 9−4. 2 (2H. m)、7. 26 (4H. s) | 529. 527<br>236. 223<br>125 | 0. 41 |
| 591 | 0. 87 (3H. t. J≒6)、1. 0−2. 0 (20H. m)、2. 3−2. 9 (16H. m)、3. 8−4. 2 (2H. m)、4. 22 (1H. s)、7. 0−7. 6 (10H. m) | 569. 278<br>167 | 0. 66 |
| 592 | 0. 87 (3H. t. J≒6)、1. 0−2. 0 (22H. m)、2. 4−3. 0 (16H. m)、3. 60 (2H. s)、3. 9−4. 2 (2H. m)、6. 8−7. 4 (4H. m) | 525. 234<br>221 | 0. 27 |
| 593 | 0. 87 (3H. t. J≒6)、1. 0−2. 0 (20H. m)、2. 4−2. 9 (12H. m)、2. 9−3. 2 (4H. m)、3. 77 (4H. s)、3. 9−4. 2 (2H. m)、6. 6−7. 0 (4H. m) | 509. 218<br>205 | 0. 60 |
| 594 | 0. 88 (3H. t. J≒6)、1. 0−2. 0 (24H. m)、2. 4−3. 0 (12H. m)、3. 6−3. 8 (4H. m)、3. 9−4. 2 (2H. m) | 432. 101 | 0. 34 |
| 595 | 0. 88 (3H. t. J≒6)、0. 91 (3H. t. J≒6)、1. 0−2. 0 (28H. m)、2. 2−3. 0 (18H. m)、3. 9−4. 2 (2H. m) | 487. 155 | 0. 16 |
| 596 | 0. 88 (3H. t. J≒6)、1. 0−2. 0 (24H. m)、2. 3−2. 9 (16H. m)、3. 53 (2H. s)、3. 9−4. 2 (2H. m)、7. 30 (5H. s) | 521. 202<br>179 | 0. 21 |

| | | | |
|---|---|---|---|
| 597 | 0.88 (3H, t, J≒6), 1.0-2.0 (24H, m), 2.4-2.9 (16H, m), 3.47 (2H, s), 3.79 (3H, s), 3.9-4.2 (2H, m), 6.84 (2H, d, J=8.6), 7.22 (2H, d, J=8.6) | 551.232219 | 0.21 |
| 598 | 0.88 (3H, t, J≒6), 1.0-2.0 (26H, m), 2.4-3.0 (16H, m), 3.9-4.2 (2H, m), 6.8-7.4 (4H, m) | 553.234221 | 0.15 |
| 599 | 0.88 (3H, t, J≒6), 1.0-2.0 (24H, m), 2.5-2.9 (12H, m), 2.9-3.2 (4H, m), 3.86 (3H, s), 3.9-4.1 (2H, m), 6.7-7.1 (2H, m) | 537.208205 | 0.43 |
| 600 | 0.88 (3H, t, J≒6), 0.91 (3H, t, J≒6), 1.0-2.0 (32H, m), 2.2-2.9 (18H, m), 3.9-4.2 (2H, m) | 515.155 | 0.22 |
| 601 | 0.88 (3H, t, J≒6), 1.0-2.0 (28H, m), 2.4-2.9 (16H, m), 3.51 (2H, s), 3.9-4.2 (2H, m), 7.29 (5H, s) | 549.202189 | 0.36 |
| 602 | 0.88 (3H, t, J≒6), 1.0-2.0 (28H, m), 2.3-2.9 (16H, m), 3.47 (2H, s), 3.9-4.2 (2H, m), 6.8-7.4 (4H, m) | 567.549220.207 | 0.37 |
| 603 | 0.88 (3H, t, J≒6), 1.0-2.0 (30H, m), 2.4-3.0 (16H, m), 3.60 (2H, s), 3.9-4.2 (2H, m), 7.27 (4H, s) | 599.597250 | 0.30 |
| 604 | 0.88 (3H, t, J≒6), 1.0-2.0 (28H, m), 2.3-2.9 (16H, m), 3.9-4.2 (2H, m), 4.23 (1H, s), 7.0-7.5 (10H, m) | 625.228167 | 0.71 |
| 605 | 0.88 (3H, t, J≒6), 1.0-2.0 (28H, m), 2.5-3.0 (12H, m), 2.9-3.2 (4H, m), 4.0-4.3 (2H, m), 6.8-7.4 (4H, m) | 571.569222.209 | 0.71 |
| 606 | 0.88 (3H, t, J≒6), 1.0-2.0 (28H, m), 2.4-2.9 (12H, m), 3.5-3.8 (4H, m), 3.9-4.2 (2H, m) | 488.113 | 0.47 |
| 607 | 0.88 (3H, t, J≒6), 0.91 (3H, t, J≒6), 1.0-2.0 (36H, m), 2.1-2.8 (18H, m), 3.9-4.2 (2H, m) | 543.168155 | 0.26 |
| 608 | 0.88 (3H, t, J≒6), 1.0-2.0 (32H, m), 2.3-2.9 (16H, m), 3.51 (2H, s), 3.9-4.2 (2H, m), 7.29 (5H, s) | 578.202 | 0.35 |
| 609 | 0.88 (3H, t, J≒6), 1.0-2.0 (34H, m), 2.5-2.9 (16H, m), 3.62 (2H, s), 3.80 (3H, s), 3.9-4.2 (2H, m), 6.84 (2H, d, J=8.6), 7.26 (2H, d, J=8.6) | 621.246121 | 0.25 |

| | | | |
|---|---|---|---|
| 610 | 0.88 (3H, t, J≒6)、1.0−2.0 (32H, m)、2.4−2.9 (12H, m)、2.9−3.2 (4H, m)、3.76 (3H, s)、3.9−4.2 (2H, m)、6.6−7.0 (4H, m) | 592, 218 | 0.45 |
| 659 | 1.7−1.9 (4H, m)、2.6−2.9 (4H, m)、3.73 (3H, s)、4.16 (2H, s)、4.86 (2H, s), 7.0−7.4 (5H, m) | 313 | |
| 660 | 1.7−1.9 (4H, m)、2.6−3.0 (4H, m)、4.16 (2H, s)、4.87 (2H, s)、7.0−7.5 (5H, m) | 299 | |
| 661 | 1.7−1.9 (4H, m)、2.6−2.9 (4H, m)、3.2−3.5 (4H, m)、4.17 (2H, s)、4.85 (2H, s)、7.0−7.5 (5H, m) | 362, 360 | |
| 667 | 0.94 (3H, t, J≒7.4)、1.2−2.0 (8H, m)、2.6−3.0 (6H, m)、3.75 (3H, s)、4.88 (2H, s) | 279 | 0.89 |
| 668 | 0.89 (3H, t, J≒6)、1.2−2.0 (10H, m)、2.6−3.0 (6H, m)、3.75 (3H, s)、4.89 (2H, s) | 293 | 0.90 |
| 669 | 0.88 (3H, t, J≒6)、1.2−2.0 (12H, m)、2.6−3.0 (6H, m)、3.75 (3H, s)、4.88 (2H, s) | 307 | 0.92 |
| 670 | 0.88 (3H, t, J≒6)、1.2−2.0 (14H, m)、2.6−3.0 (6H, m)、3.75 (3H, s)、4.89 (2H, s) | 321 | 0.93 |
| 671 | 0.87 (3H, t, J≒6)、1.2−2.0 (16H, m)、2.6−3.0 (6H, m)、3.75 (3H, s)、4.88 (2H, s) | 335 | 0.93 |
| 672 | 0.87 (3H, t, J≒6)、1.1−2.0 (18H, m)、2.6−3.0 (6H, m)、3.75 (3H, s)、4.88 (2H, s) | 349 | 0.93 |
| 673 | 0.87 (3H, t, J≒6)、1.1−2.0 (20H, m)、2.6−3.0 (6H, m)、3.75 (3H, s)、4.88 (2H, s) | 363 | 0.94 |
| 674 | 0.87 (3H, t, J≒6)、1.1−2.0 (24H, m)、2.6−3.0 (6H, m)、3.75 (3H, s)、4.88 (2H, s) | 391 | 0.94 |
| 675 | 0.88 (3H, t, J≒6)、1.0−2.0 (28H, m)、2.6−3.0 (6H, m)、3.75 (3H, s)、4.88 (2H, s) | 419 | 0.94 |

| | | | |
|---|---|---|---|
| 676 | 0. 88 (3H. t. J≒6)、1. 0−2. 0 (32H. m)、2. 6−3. 0 (6H. m)、3. 75 (3H. s)、4. 88 (2H. s) | 447 | 0. 95 |
| 677 | 0. 99 (3H. t. J=7. 3)、1. 5−2. 0 (6H. m)、3. 75 (3H. s)、3. 89 (2H. s) | 265 | 0. 86 |
| 678 | 0. 86 (3H. t. J=7. 4)、1. 25 (2H. d. J=7. 8)、1. 6−2. 0 (6H. m)、2. 6−2. 9 (4H. m)、2. 9−3. 3 (1H. m)、3. 74 (3H. s)、4. 88 (2H. s) | 279 | 0. 88 |
| 685 | 0. 93 (3H. t. J≒6. 7)、1. 1−2. 0 (8H. m)、2. 6−3. 0 (6H. m)、3. 6−4. 1 (3H. m)、4. 4−4. 6 (2H. m) | 251 | 0. 66 |
| 686 | 0. 89 (3H. t. J≒6)、1. 2−2. 0 (10H. m)、2. 6−3. 0 (6H. m)、3. 7 (1H. br. s)、3. 8−4. 1 (2H. m)、4. 4−4. 6 (2H. m) | 265 | 0. 67 |
| 687 | 0. 88 (3H. t. J≒6)、1. 0−2. 0 (12H. m)、2. 6−3. 0 (6H. m)、3. 7 (1H. br. s)、3. 8−4. 1 (2H. m)、4. 3−4. 6 (2H. m) | 279 | 0. 67 |
| 688 | 0. 88 (3H. t. J≒6)、1. 2−2. 0 (14H. m)、2. 6−3. 0 (6H. m)、3. 7 (1H. br. s)、3. 8−4. 1 (2H. m)、4. 4−4. 6 (2H. m) | 293 | 0. 67 |
| 689 | 0. 88 (3H. t. J≒6)、1. 1−2. 0 (16H. m)、2. 6−3. 0 (6H. m)、3. 7 (1H. br. s)、3. 8−4. 1 (2H. m)、4. 4−4. 6 (2H. m) | 307 | 0. 69 |
| 690 | 0. 87 (3H. t. J≒6)、1. 0−2. 0 (18H. m)、2. 6−3. 0 (6H. m)、3. 7 (1H. br. s)、3. 8−4. 1 (2H. m)、4. 4−4. 6 (2H. m) | 321 | 0. 70 |
| 691 | 0. 88 (3H. t. J≒6)、1. 1−2. 0 (20H. m)、2. 6−3. 0 (6H. m)、3. 7−4. 1 (3H. m)、4. 4−4. 6 (2H. m) | 335 | 0. 72 |
| 692 | 0. 88 (3H. t. J≒6)、1. 0−2. 0 (24H. m)、2. 6−3. 0 (6H. m)、3. 7−4. 1 (3H. m)、4. 4−4. 6 (2H. m) | 363 | 0. 78 |
| 693 | 0. 88 (3H. t. J≒6)、1. 0−2. 0 (28H. m)、2. 5−3. 0 (6H. m)、3. 7−4. 1 (3H. m)、4. 4−4. 6 (2H. m) | 391 | 0. 81 |
| 694 | 0. 88 (3H. t. J≒6)、1. 0−2. 0 (32H. m)、2. 6−3. 0 (6H. m)、3. 7−4. 1 (3H. m)、4. 3−4. 6 (2H. m) | 419 | 0. 81 |

| | | | |
|---|---|---|---|
| 695 | 0.97 (3H, t, J=7.3)、1.5−2.0 (6H, m)、2.6−3.0 (6H, m)、3.7 (1H, br, s)、3.8−4.1 (2H, m)、4.4−4.6 (2H, m) | 237 | 0.54 |
| 696 | 0.84 (3H, t, J=7.4)、1.21 (3H, d, J≒7)、1.2−2.0 (6H, m)、2.6−2.9 (6H, m)、2.8−3.3 (1H, m)、3.8−4.1 (3H, m)、4.3−4.5 (2H, m) | 251 | 0.65 |
| 817 | 0.91 (3H, t)、0.93 (3H, t)、1.1−1.9 (12H, m)、2.0−3.0 (18H, m)、4.08 (2H, t) | 375 | 0.22 |
| 830 | 0.87 (3H, t)、1.1−1.5 (10H, m)、1.5−2.0 (6H, m)、2.4−2.9 (18H, m)、3.63 (2H, t)、4.09 (2H, t) | 419 | 0.23 |
| 837 | 0.93 (3H, t)、1.0−2.0 (14H, m)、2.0−3.0 (12H, m)、4.11 (3H, t) | 318 | 0.28 |
| 839 | 0.87 (3H, t)、1.1−2.0 (22H, m)、2.4−2.9 (12H, m)、4.12 (2H, t) | 274 | 0.30 |
| 850 | 0.93 (3H, t)、1.06 (6H, t)、1.1−2.0 (8H, m)、2.4−3.0 (12H, m)、4.08 (2H, t) | 306 | 0.69 |
| 851 | 0.87 (3H, t)、1.04 (6H, t)、1.2−1.5 (10H, m)、1.5−2.0 (6H, m)、2.5−3.0 (12H, m)、4.06 (2H, t) | 362 | 0.19 |

## Claims

### Claims for the following Contracting States : DE, FR, IT

1. A compound which is a pyrazine derivative of formula

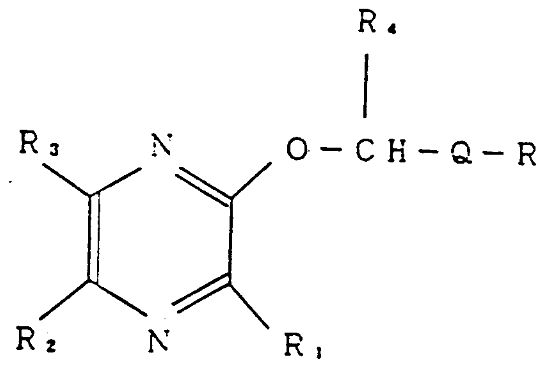

wherein:

Q is -CO- or -$CH_2$-,

R is hydroxyl, $C_1$-$C_4$ alkoxy, a halogen, -NH-$C_1$-$C_4$ alkylene-OH, -NH-$C_1$-$C_4$ alkylene-arylthio, -NH-$C_1$-$C_4$ alkylene-halogen,

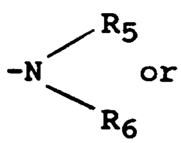 or NH-$C_1$-$C_4$ alkylene- 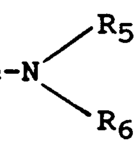 ,

in which

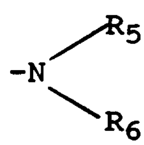

is di-$C_1$-$C_4$-alkylamino, morpholino,

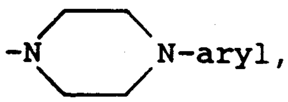

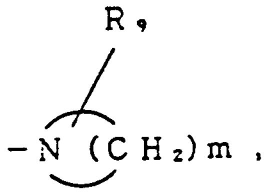, 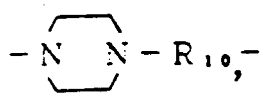 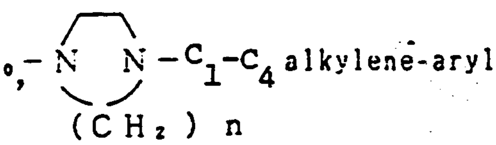

or

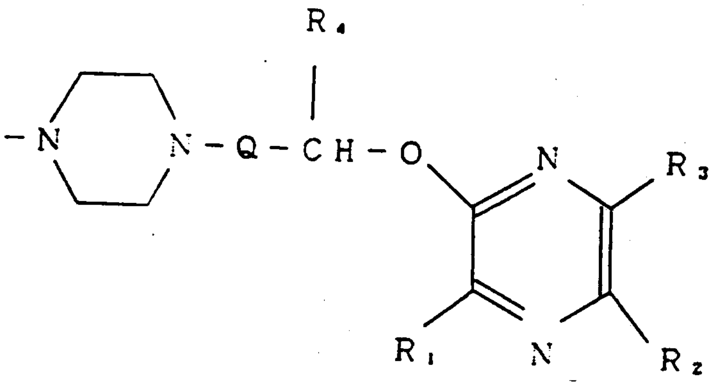

in which $R_9$ is hydrogen or $C_1$-$C_4$ alkyl, $R_{10}$ is hydrogen, $C_1$-$C_4$ alkyl, hydroxy-$C_1$-$C_4$ alkyl, hydroxy-$C_1$-$C_4$ alkoxy-$C_1$-$C_4$ alkyl or di(aryl)-$C_1$-$C_4$ alkyl, m is an integer of 4 to 6, n is 2 or 3,

$R_1$ is $C_1$-$C_{20}$ alkyl or aryl-$C_1$-$C_4$ alkyl,

$R_2$ and $R_3$ are independently each $C_1$-$C_4$ alkyl, or together form a tetramethylene bridge, and

$R_4$ is hydrogen, $C_1$-$C_4$ alkyl or aryl,

wherein each aryl is phenyl optionally substituted by 1 to 3 halogens or nitro, $C_1$-$C_4$ alkyl or $C_1$-$C_4$ alkoxy groups,

or a pharmaceutically acceptable salt thereof,

with the proviso that when Q is $-CH_2-$, $R_1$, $R_2$ and $R_3$ are each methyl and $R_4$ is hydrogen, then R is not

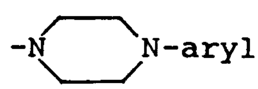

wherein aryl is

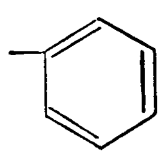, 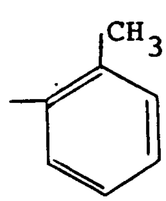 or 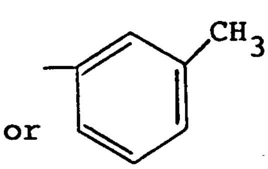

**2.** A compound according to claim 1 wherein Q is -CO-, and $R_2$ and $R_3$ are each independently $C_1$-$C_4$ alkyl.

**3.** A compound according to claim 1 of formula

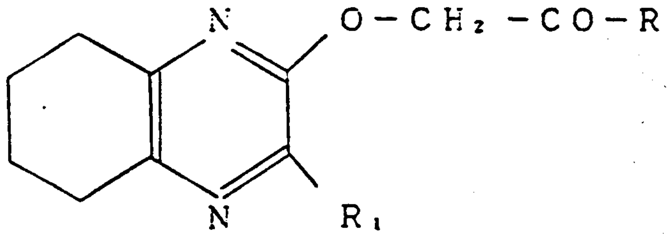

wherein R and $R_1$ are as defined in claim 1, or a pharmaceutically acceptable salt thereof.

**4.** A compound according to claim 1 where $R_4$ is hydrogen, Q is $-CH_2-$, $R_2$ and $R_3$ are each independently $C_1$-$C_4$ alkyl, and R is hydroxyl, halogen or

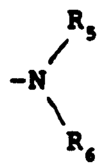

as defined in claim 1.

**5.** A compound according to claim 1 of formula

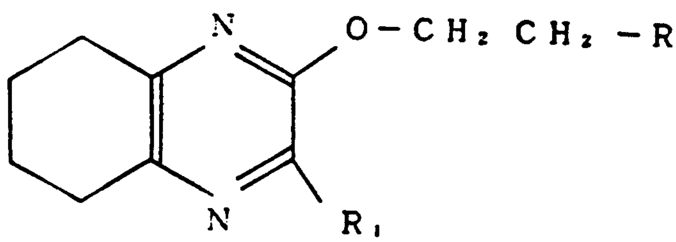

wherein R is hydroxyl, a halogen or

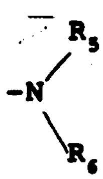

, and

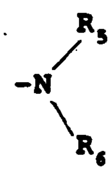

and $R_1$ are as defined in claim 1, or a pharmaceutically acceptable salt thereof.

**6.** A composition which comprises a compound of formula (I) as defined in claim 1 or a pharmaceutically acceptable salt thereof in a pharmaceutically acceptable diluent.

**7.** A compound of formula (I) as defined in claim 1 or a pharmaceutically acceptable salt thereof for use in a method of treatment by therapy.

**8.** A compound of formula (I) as defined in claim 1 or a pharmaceutically acceptable salt thereof for use in treating a circulatory or metabolic disorder.

**9.** Use of a compound of formula (I) as defined in claim 1 or a pharmaceutically acceptable salt thereof in

the manufacture of a medicament for the treatment of a circulatory or metabolic disorder.

**10.** A process for producing a compound of formula (I) as defined in claim 1 or a pharmaceutically acceptable salt thereof which comprises:

a) when Q is -CO- and R is $C_1$-$C_4$ alkoxy, by reacting a compound of formula

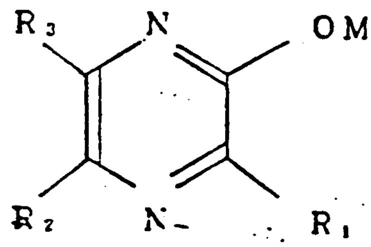

[3]

wherein M is alkaline metal, and $R_1$, $R_2$ and $R_3$ are as defined above in the compound of formula (I), with a $\alpha$-halogeno-carboxylate ester of formula

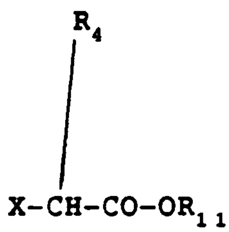

[4]

wherein X is a halogen, $R_{11}$ is $C_1$-$C_4$ alkyl and $R_4$ is as defined above in the compound of formula (I), in an organic solvent;

b) when Q is -CO- and R is hydroxyl by de-esterifying a compound of formula (I) wherein Q is -CO- and R is $C_1$-$C_4$ alkoxy;

c) when Q is -CO- and R is -NH-$C_1$-$C_4$-alkylene-OH or

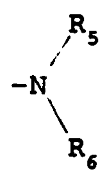

,

wherein

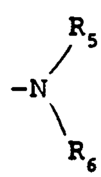

is as defined above in the compound of formula (I), by reacting an amine of formula

H-R' [5]

wherein R' is -NH-$C_1$-$C_4$-alkylene-OH or

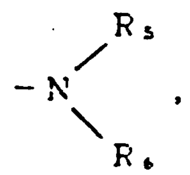

in which

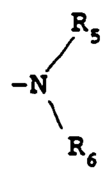

is di-$C_1$-$C_4$ alkylamino, morpholino, morpholino,

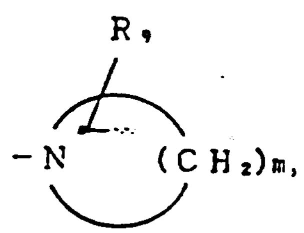 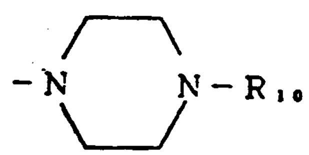 or

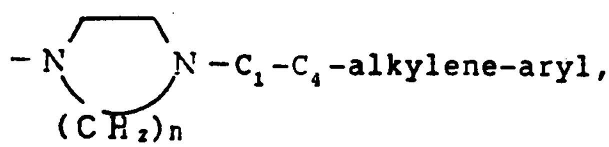

and $R_9$, $R_{10}$ aryl, m and n are as defined above in the compound of formula (I), with a compound of formula (I) wherein Q is -CO- and R is hydroxyl, or a reactive derivative thereof suitable for N-acylation;

d) when Q is -CO- and R is -NH-$C_1$-$C_4$-alkylene-halogen, by N-acylating a compound of formula

X-$C_1$-$C_4$ alkylene-$NH_2$ [6]

wherein X is a halogen, with a compound of formula (I) wherein Q is -CO- and R is hydroxyl, or a reactive derivative thereof suitable for N-acylation;

e) when Q is -CO- and R is -NH-$C_1$-$C_4$ alkylene-

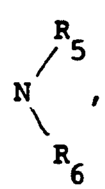

by aminating a compound of formula (I) wherein Q is -CO- and R is -NH-$C_1$-$C_4$-alkylene-halogen with an amine of formula:

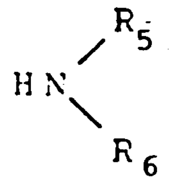

wherein

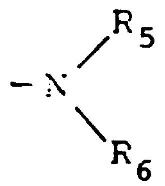

is as defined in claim 1;
f) when Q is $-CH_2-$ and R is $-NH-C_1-C_4$ alkylene-arylthio, by thioetherification of a compound of formula (I) wherein Q is $-CH_2-$ and R is $-NH-C_1-C_4$-alkylene-halogen with an aryl-thiol;
g) when Q is $-CH_2-$ and R is a hydroxyl group, by reducing a compound of formula (I) wherein Q is -CO- and R is $C_1-C_4$ alkoxy with an alkaline borohydride in the presence of a $C_1-C_4$ alcohol under heating;
h) when Q is $-CH_2-$ and R is a halogen, by halogenating a compound of formula (I) wherein Q is $-CH_2-$ and R is a hydroxyl group with a halogenating agent in a solvent; or
i) when Q is $-CH_2-$ and R is

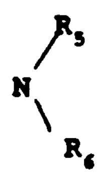

wherein

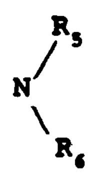

is as defined above in the compound of formula (I), by aminating a compound of formula (I) wherein Q is $-CH_2-$ and R is a halogen with an amine of formula [5] as defined above in a solvent under heating.

**Claims for the following Contracting State : ES**

**1.** A process for preparing a compound which is a pyrazine derivative of formula

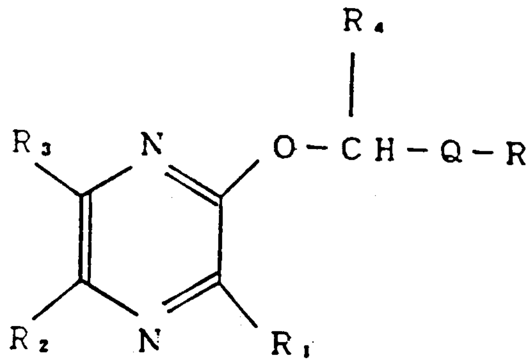

wherein:

Q is -CO- or $-CH_2-$,

R is hydroxyl, $C_1$-$C_4$ alkoxy, a halogen, -NH-$C_1$-$C_4$ alkylene-OH, -NH-$C_1$-$C_4$ alkylene-arylthio,-NH-$C_1$-$C_4$ alkylene-halogen,

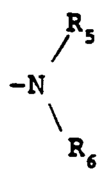 or 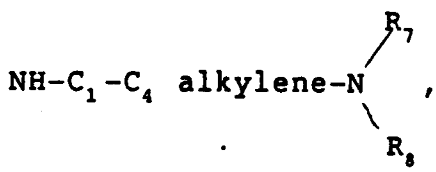 ,

in which

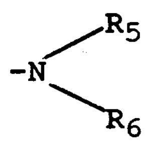

is di-$C_1$-$C_4$ alkylamino, morpholino,

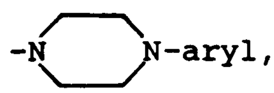

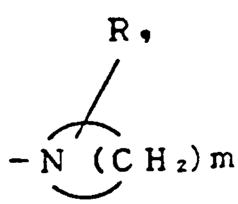 , 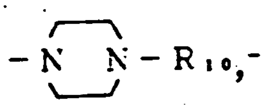 , 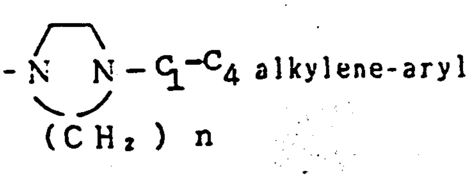

or

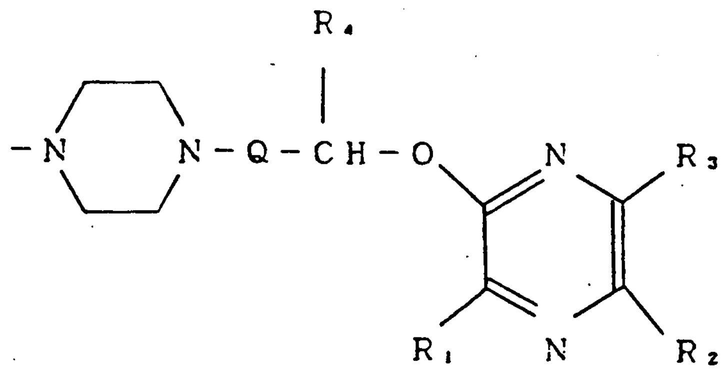

in which $R_9$ is hydrogen or $C_1$-$C_4$ alkyl, $R_{10}$ is hydrogen, $C_1$-$C_4$ alkyl, hydroxy-$C_1$-$C_4$ alkyl, hydroxy-$C_1$-$C_4$ alkoxy-$C_1$-$C_4$ alkyl or di(aryl)-$C_1$-$C_4$ alkyl, m is an integer of 4 to 6, n is 2 or 3,

$R_1$ is $C_1$-$C_{20}$ alkyl or aryl-$C_1$-$C_4$ alkyl,

$R_2$ and $R_3$ are independently each $C_1$-$C_4$ alkyl, or together form a tetramethylene bridge, and

$R_4$ is hydrogen, $C_1$-$C_4$ alkyl or aryl,

wherein each aryl is phenyl optionally substituted by 1 to 3 halogens or nitro, $C_1$-$C_4$ alkyl or $C_1$-$C_4$ alkoxy groups,

or a pharmaceutically acceptable salt thereof,

with the proviso that when Q is $-CH_2-$, $R_1$, $R_2$ and $R_3$ are each methyl and $R_4$ is hydrogen, then R is not

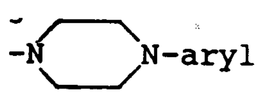

wherein aryl is

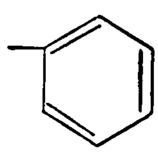 , 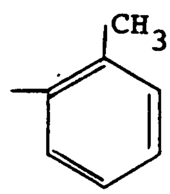 or 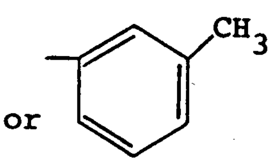

which comprises:

a) when Q is -CO- and R is $C_1$-$C_4$ alkoxy, by reacting a compound of formula

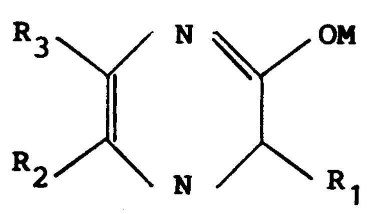 [3]

wherein M is alkaline metal, and $R_1$, $R_2$ and $R_3$ are as defined above in the compound of formula (I), with a $\alpha$-halogeno-carboxylate ester of formula

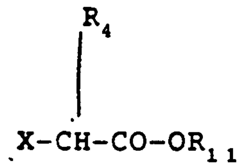

[4]

wherein X is a halogen, $R_{11}$ is $C_1$-$C_4$ alkyl and $R_4$ is as defined above in the compound of formula (I), in an organic solvent;

b) when Q is -CO- and R is hydroxyl, by de-esterifying a compound of formula (I), wherein Q is -CO- and R is $C_1$-$C_4$ alkoxy;

c) when Q is -CO- and R is -NH-$C_1$-$C_4$-alkylene-OH or

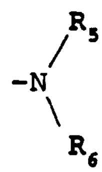,

wherein

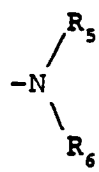

is as defined above in the compound of formula (I) by reacting an amine of formula

H-R' [5]

wherein R' is -NH-$C_1$-$C_4$-alkylene-OH or

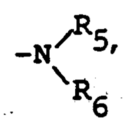,

in which

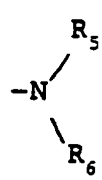

is di-$C_1$-$C_4$ alkylamino, morpholino

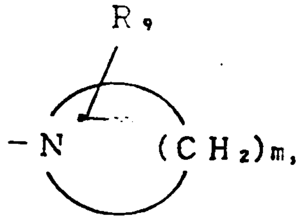 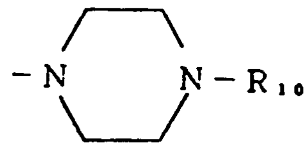 or

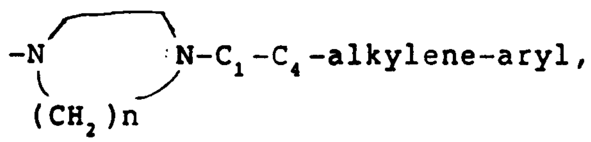

and $R_9$, $R_{10}$ aryl, m and n are as defined above in the compound of formula (I), with a compound of formula (I) wherein Q is -CO- and R is hydroxyl, or a reactive derivative thereof suitable for N-acylation;

d) when Q is -CO- and R is -NH-$C_1$-$C_4$-alkylene-halogen, by N-acylating a compound of formula

X-$C_1$-$C_4$ alkylene-$NH_2$ [6]

wherein X is a halogen, with a compound of formula (I) wherein Q is -CO- and R is hydroxyl, or a reactive derivative thereof suitable for N-acylation;

e) when Q is -CO- and R is -NH-$C_1$-$C_4$ alkylene-by

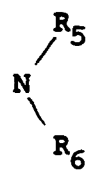 ,

aminating a compound of formula (I) wherein Q is -CO- and R is -NH-$C_1$-$C_4$-alkylene-halogen with an amine of formula:

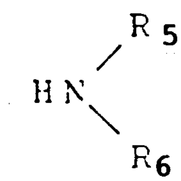

wherein

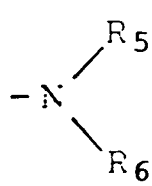

is as defined above;

f) when Q is -$CH_2$- and R is -NH-$C_1$-$C_4$ alkylene-arylthio, by thioetherification of a compound of formula (I) wherein Q is -$CH_2$- and R is -NH-$C_1$-$C_4$-alkylene-halogen with an aryl-thiol;

g) when Q is -$CH_2$- and R is a hydroxyl group, by reducing a compound of formula (I) wherein Q is -CO- and R is $C_1$-$C_4$ alkoxy with an alkaline borohydride in the presence of a $C_1$-$C_4$ alcohol under heating;

h) when Q is -$CH_2$- and R is a halogen, by halogenating a compound of formula (I) wherein Q is

$-CH_2-$ and R is a hydroxyl group with a halogenating agent in a solvent; or
i) when Q is $-CH_2-$ and R is

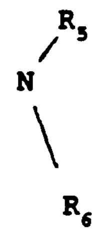

wherein

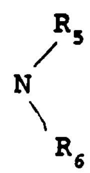

is as defined above in the compound of formula (I), by aminating a compound of formula (I) wherein Q is $-CH_2-$ and R is a halogen with an amine of formula [5] as defined above in a solvent under heating.

**2.** A process according to claim 1 wherein Q is -CO- and $R_2$ and $R_3$ are independently $C_1$-$C_4$ alkyl.

**3.** A process according to claim 1 wherein the compound of formula (I) is of formula:

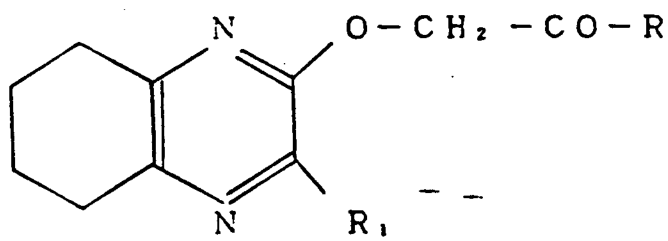

wherein R and $R_1$ are as defined in claim 1.

**4.** A process according to claim 1 wherein $R_4$ is hydrogen, Q is $-CH_2-$, $R_2$ and $R_3$ are each independently $C_1$-$C_4$ alkyl, and R is hydroxyl, halogen or

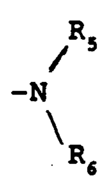

as defined in claim 1.

**5.** A process according to claim 1 wherein the compound of formula (I) is of formula:

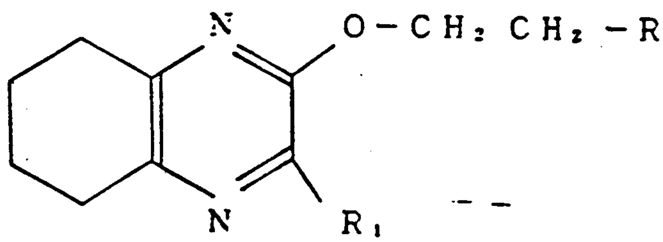

wherein R is hydroxyl, a halogen or

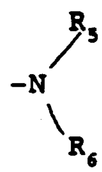

and $R_1$ and

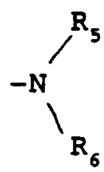

are as defined in claim 1.

**6.** A process for preparing a pharmaceutical composition which comprises mixing a compound of formula (I) as defined in claim 1, or a pharmaceutically acceptable salt thereof, with a pharmaceutically acceptable diluent.

**Revendications**

**Revendications pour les Etats contractants suivants : DE, FR, IT**

**1.** Composé, qui est un dérivé de pyrazine de la formule :

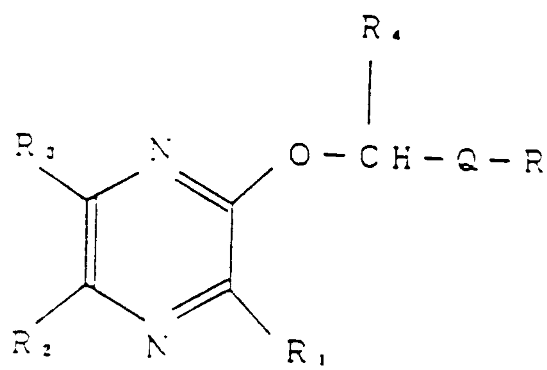

dans laquelle :

Q est -CO- ou $-CH_2-$,

R est un hydroxyle, un alcoxy à 1 à 4 atomes de carbone, un halogène, -NH-alcoylène à 1 à 4 atomes de carbone-OH, -NH-alcoylène à 1 à 4 atomes de carbone-arylthio, -NH-alcoylène à 1 à 4 atomes de

carbone-halogène,

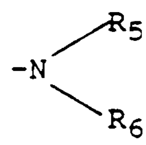

ou NH-alcoylène à 1 à 4 atomes de carbone

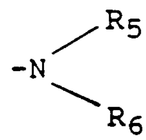

dans laquelle

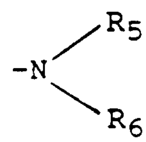,

est un groupe di-alcoylamino à 1 à 4 atomes de carbone, morpholino,

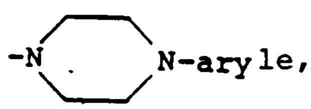

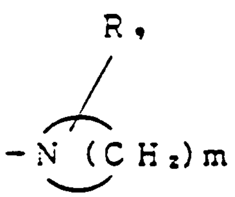 , 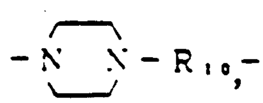 , 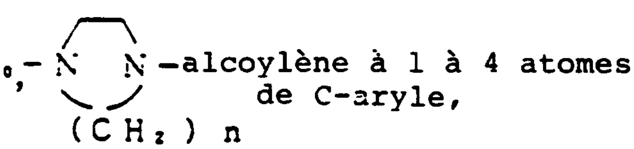

ou

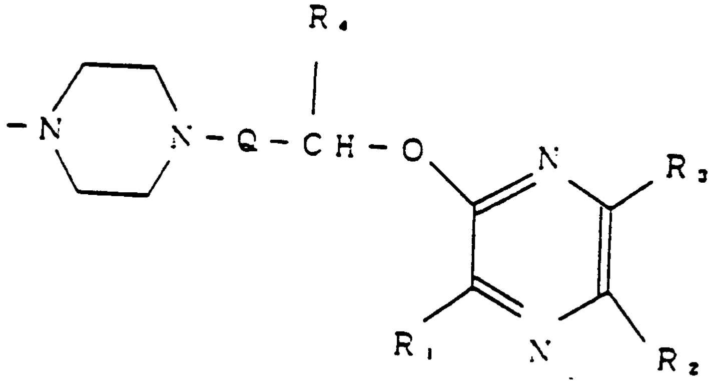

dans laquelle $R_9$ est un atome d'hydrogène ou un groupe alcoyle à 1 à 4 atomes de carbone, $R_{10}$ est un atome d'hydrogène, un groupe alcoyle à 1 à 4 atomes de carbone, un hydroxy-alcoyle à 1 à 4 atomes de carbone, un hydroxy-alcoxy à 1 à 4 atomes de carbone-alcoyle à 1 à 4 atomes de carbone

ou un di(aryl)-alcoyle à 1 à 4 atomes de carbone, m est un entier égal à 4, 5 ou 6, n est égal à 2 ou 3,

$R_1$ est un alcoyle à 1 à 20 atomes de carbone ou un arylalcoyle à 1 à 4 atomes de carbone,

$R_2$ et $R_3$ sont indépendamment l'un de l'autre chacun un alcoyle à 1 à 4 atomes de carbone ou forment ensemble un pont tétraméthylène, et

$R_4$ est un atome d'hydrogène ou un alcoyle à 1 à 4 atomes de carbone ou un aryle, chaque aryle étant un phényle facultativement substitué par 1 à 3 atomes d'halogène ou nitro, ou groupes alcoyle à 1 à 4 atomes de carbone ou alcoxy à 1 à 4 atomes de carbone, ou un de leurs sels pharmaceutiquement acceptables, sous réserve que, lorsque Q est $-CH_2-$, $R_1$, $R_2$ et $R_3$ sont chacun du méthyle et $R_4$ est un atome d'hydrogène, alors R n'est pas

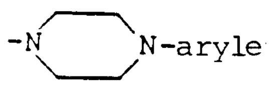

dans laquelle le groupe aryle est

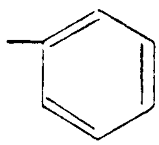,
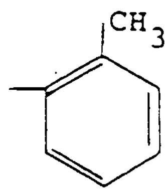
or
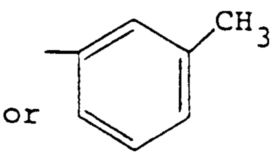

**2.** Composé selon la revendication 1, dans lequel Q est -CO-, et $R_2$ et $R_3$ sont chacun indépendamment un groupe alcoyle à 1 à 4 atomes de carbone.

**3.** Composé selon la revendication 1, de la formule :

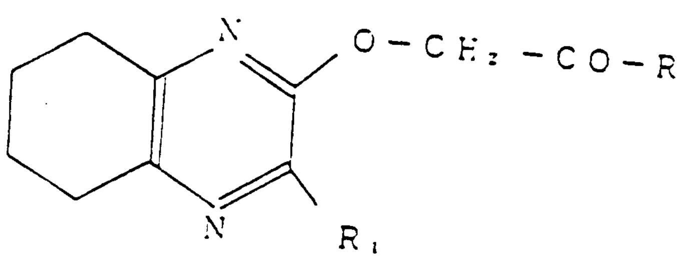

dans laquelle R et $R_1$ sont comme définis dans la revendication 1, ou un de leurs sels pharmaceutiquement acceptables.

**4.** Composé selon la revendication 1, dans lequel $R_4$ est un atome d'hydrogène, Q est $-CH_2-$, $R_2$ et $R_3$ sont chacun indépendamment un alcoyle à 1 à 4 atomes de carbone, et R est un groupe hydroxyle, un atome d'halogène,
ou

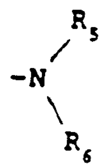

comme défini dans la revendication 1.

**5.** Composé selon la revendication 1, de la formule :

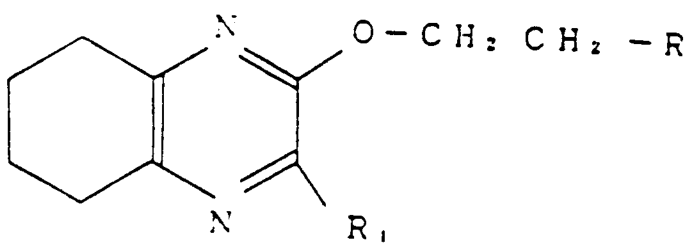

dans laquelle R est un groupe hydroxyle, un atome d'halogène, ou

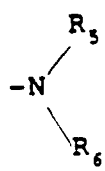 ,

et dans laquelle

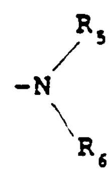

et $R_1$ sont comme définis dans la revendication 1, ou un de leurs sels pharmaceutiquement acceptables.

**6.** Composition, qui contient un composé de la formule (I), telle que définie dans la revendication 1 ou un de ses sels pharmaceutiquement acceptables dans un diluant pharmaceutiquement acceptable.

**7.** Composé de la formule (I) tel que défini dans la revendication 1, ou de ses sels pharmaceutiquement acceptables pour être utilisé dans une méthode de traitement par thérapie.

**8.** Composé de la formule (I) selon la revendication 1 ou un de ses sels pharmaceutiquement acceptables pour être utilisé dans le traitement de désordres circulatoires ou métaboliques.

**9.** Utilisation d'un composé de la formule (I) selon la revendication 1 ou un de ses sels pharmaceutiquement acceptables dans la fabrication d'un médicament pour le traitement de désordres circulatoires ou métaboliques.

**10.** Procédé pour produire un composé de la formule (I) tel que défini dans la revendication 1 ou un de ses sels pharmaceutiquement acceptables, qui comprend les stades suivants :

a) lorsque Q est -CO- et R est un alcoxy à 1 à 4 atomes de carbone, on fait réagir un composé de la formule :

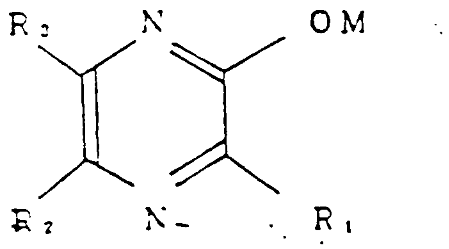

[3]

dans laquelle M est un métal alcalin et $R_1$, $R_2$ et $R_3$ sont comme définis ci-dessus dans le composé de la formule (I), avec un α-halogéno carboxylate de la formule :

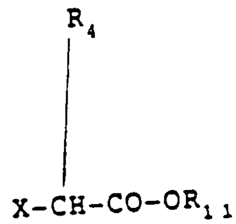

[4]

dans laquelle X est un halogène, $R_{11}$ est un alcoyle à 1 à 4 atomes de carbone et $R_4$ est comme défini ci-dessus dans le composé de la formule (I), dans un solvant organique;
b) lorsque Q est -CO- et R est un hydroxyle, on désestérifie un composé de la formule (I) dans laquelle Q est -CO- et
R est un alcoxy à 1 à 4 atomes de carbone;
c) quand Q est -CO- et R est -NH-alcoylène à 1 à 4 atomes de

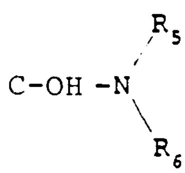

, dans lequel

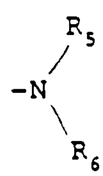

est défini
comme ci-dessus dans le composé de la formule (I), on fait réagir une amine de la formule :

H-R' (5)

dans laquelle R' est -NH-alcoylène à 1 à 4 atomes de C-OH
ou

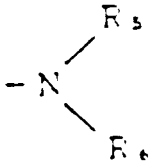

, dans laquelle

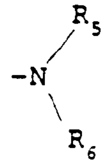

est un groupe di-alcoylamino à 1 à 4 atomes de carbone, morpholino,

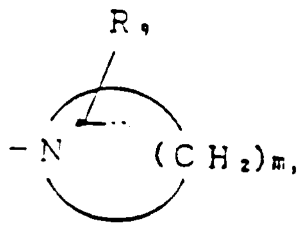

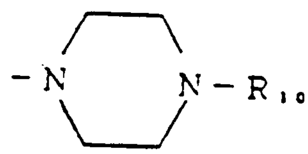

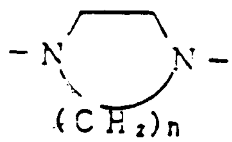 – alcoylène à 1 à 4 atomes de C-aryle,
et $R_9$, $R_{10}$ aryle, et

m et n sont comme définis ci-dessus dans le composé de la formule (I), avec un composé de la formule (I) dans lequel Q est -CO- et R est un groupe hydroxyle, ou un dérivé réactif de ce composé approprié pour une acylation en N;

d) lorsque Q est -CO- et R est -NH-alcoylène à 1 à 4 atomes de C-halogène, on acyle en N un composé de la formule :

X-alcoylène à 1 à 4 atomes de $C-NH_2$

dans laquelle X est un halogène, avec un composé de la formule (I) dans lequel Q est -CO- et R est un groupe hydroxyle, ou un dérivé réactif de ce composé convenant pour une acylation en N;

e) lorsque Q est -CO- et R est -NH-alcoylène à 1 à 4 atomes
de

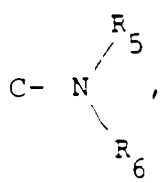

, on amine un composé de la formule (I) dans lequel Q est -CO- et R est -NH-alcoylène à 1 à 4 atomes de C-halogène avec une amine de la formule

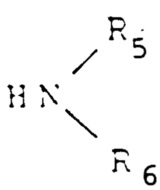 dans laquelle 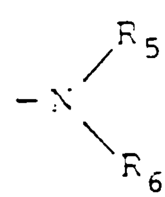

est comme défini dans la revendication 1;

f) lorsque Q est $-CH_2$ et R est -NH-alcoylène à 1 à 4 atomes de C-arylthio, on thioéthérifie un composé de la formule (I), dans lequel Q est $-CH_2-$ et R est -NH-alcoylène à 1 à 4 atomes de carbone-halogène avec un arylthiol;

g) lorsque Q est $-CH_2-$ et R est un groupe hydroxyle, on réduit un composé de la formule (I) dans lequel Q est -CO- et R est un alcoxy à 1 à 4 atomes de carbone avec un borure alcalin en présence d'un alcool à 1 à 4 atomes de carbone, en chauffant;

h) lorsque Q est $-CH_2-$ et R est un halogène, on halogène un composé de la formule (I) dans lequel Q est $-CH_2-$ et R est un groupe hydroxyle, avec un agent d'halogénation dans un solvant; ou

i) lorsque Q est $-CH_2-$ et R est

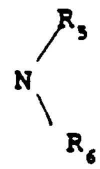

dans laquelle

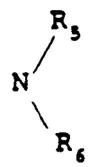

est comme défini ci-dessus dans le composé de la formule (I), on amine un composé de la formule (I) dans lequel Q est $-CH_2-$ et R est un halogène, avec une amine de la formule (5) comme définie ci-dessus, dans un solvant, en chauffant.

**Revendications pour l'Etat contractant suivant : ES**

1. Procédé pour préparer un composé, qui est un dérivé de pyrazine de la formule :

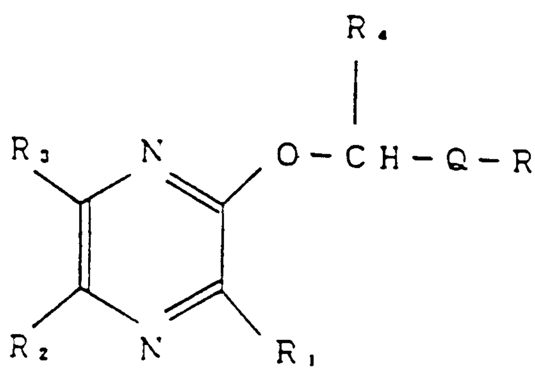

dans laquelle :

Q est -CO- ou $-CH_2-$,

R est un hydroxyle, un alcoxy à 1 à 4 atomes de carbone, un halogène, -NH-alcoylène à 1 à 4 atomes de C-OH, -NH-alcoylène à 1 à 4 atomes de C-arylthio, -NH-alcoylène à 1 à 4 atomes de C-halogène,

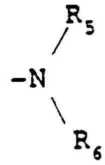

ou NH-alcoylène à 1 à 4 atomes de

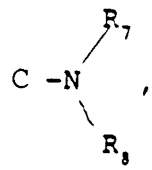

dans laquelle

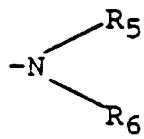

est un groupe di-alcoylamino à 1 à 4 atomes de C, morpholino,

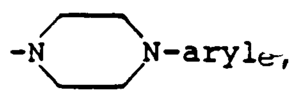

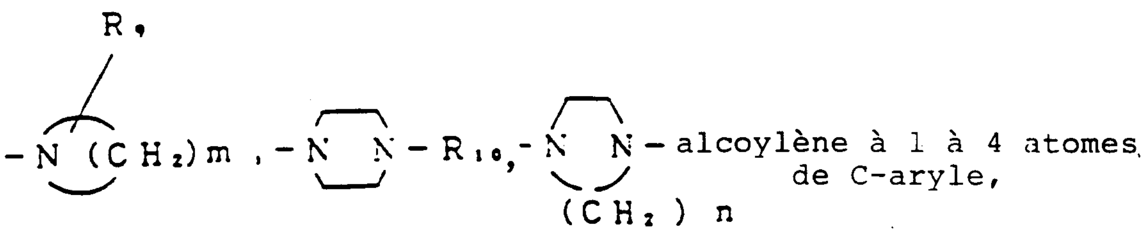

ou

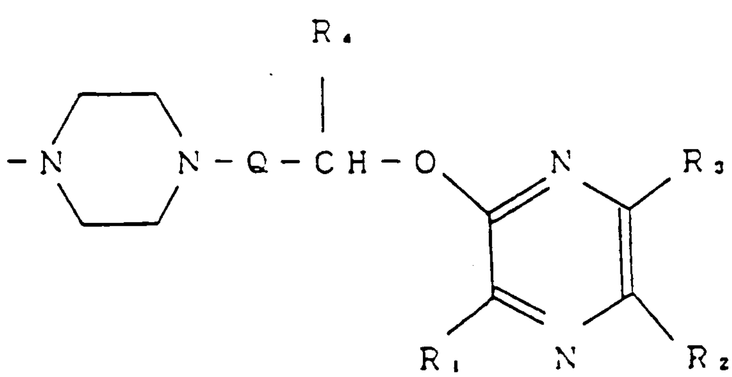

dans laquelle $R_9$ est de l'hydrogène ou un alcoyle à 1 à 4 atomes de C, $R^{10}$ est de l'hydrogène, un alcoyle à 1 à 4 atomes de carbone, un hydroxy-alcoyle à 1 à 4 atomes de carbone, un hydroxy-alcoxy à 1 à 4 atomes de carbone-alcoyle à 1 à 4 atomes de carbone ou un di(aryl)-alcoyle à 1 à 4 atomes de carbone, m est égal à 4, 5 ou 6 et n est égal à 2 ou 3,

$R_1$ est un alcoyle à 1 à 20 atomes de carbone ou un aryl-alcoyle à 1 à 4 atomes de carbone,

$R_2$ et $R_3$ sont indépendamment chacun un alcoyle à 1 à 4 atomes de carbone, ou forment ensemble

un pont tétraméthylène, et $R_4$ est de l'hydrogène, un groupe alcoyle à 1 à 4 atoomes de carbone, ou un groupe aryle,

chaque aryle étant un phényle facultativement substitué par 1 à 3 atomes d'halogène ou groupes nitro, groupes alcoyle à 1 à 4 atomes de carbone ou groupes alcoxy à 1 à 4 atomes de carbone,

ou un de leurs sels pharmaceutiquement acceptables,

sous réserve que, lorsque Q est $-CH_2-$, $R_1$, $R_2$ et $R_3$ sont chacun du méthyle et $R_4$ est de l'hydrogène, alors R n'est pas

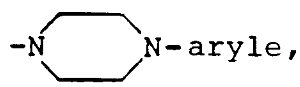

dans lequel l'aryle est

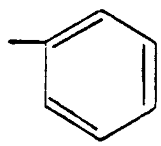
,
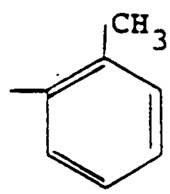
ou
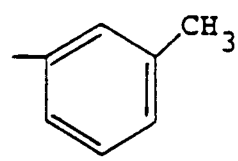

ce procédé comportant les stades suivants :

a) lorsque Q est -CO- et R est un alcoxy à 1 à 4 atomes de carbone, on fait réagir un composé de la formule :

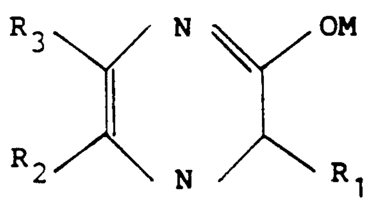
[3]

dans laquelle M est un métal alcalin et $R_1$, $R_2$ et $R_3$ sont comme définis ci-dessus dans le composé de la formule (I), avec un $\alpha$-halogéno carboxylate de la formule :

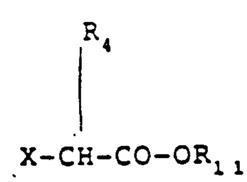
[4]

dans laquelle X est un halogène, $R_{11}$ est un alcoyle à 1 à 4 atomes de carbone et $R_4$ est comme défini ci-dessus dans le composé de la formule (I), dans un solvant organique;
b) lorsque Q est -CO- et R est un hydroxyle, on désestérifie un composé de la formule (I) dans laquelle Q est -CO- et R est un alcoxy à 1 à 4 atomes de carbone;
c) quand Q est -CO- et R est -NH-alcoylène à 1 à 4 atomes de

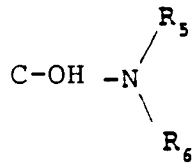,

dans lequel

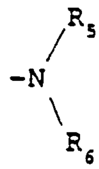

est défini comme ci-dessus dans le composé de la formule (I), on fait réagir une amine de la formule :

H-R'  (5)

dans laquelle R' est -NH-alcoylène à 1 à 4 atomes de C-OH ou

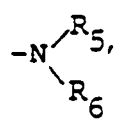

dans laquelle

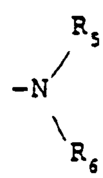

est un groupe dialcoylamino à 1 à 4 atomes de carbone, morpholino,

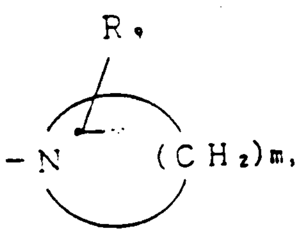 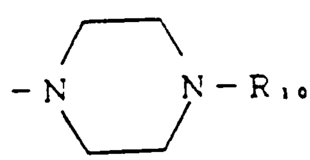

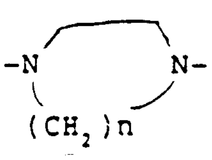 alcoylène à 1 à 4 atomes de carbone de C-aryle, et $R_9$, $R_{10}$ aryle. et

m et n sont comme définis ci-dessus dans le composé de la formule (I), avec un composé de la formule (I) dans lequel Q est -CO- et R est un groupe hydroxyle, ou un dérivé réactif de ce composé

approprié pour une acylation en N;
d) lorsque Q est -CO- et R est -NH-alcoylène à 1 à 4 atomes de C-halogène, on acyle en N un composé de la formule :

X-alcoylène à 1 à 4 atomes de C-$NH_2$

dans laquelle X est un halogène, avec un composé de la formule (I) dans lequel Q est -CO- et R est un groupe hydroxyle, ou un dérivé réactif de ce composé convenant pour une acylation en N;
e) lorsque Q est -CO- et R est -NH-alcoylène à 1 à 4 atomes de

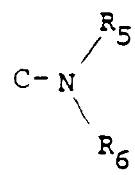
,

on amine un composé de la formule (I) dans lequel Q est -CO- et R est -NH-alcoylène à 1 à 4 atomes de C-halogène avec une amine de la formule

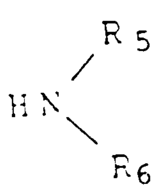 dans laquelle 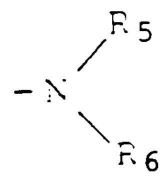

est comme défini dans la revendication 1;
f) lorsque Q est $-CH_2$ et R est -NH-alcoylène à 1 à 4 atomes de C-arylthio, on thioéthérifie un composé de la formule (I), dans lequel Q est $-CH_2-$ et R est -NH-alcoylène à 1 à 4 atomes de carbone-halogène avec un arylthiol;
g) lorsque Q est $-CH_2-$ et R est un groupe hydroxyle, on réduit un composé de la formule (I) dans lequel Q est -CO- et R est un alcoxy à 1 à 4 atomes de carbone avec un borure alcalin en présence d'un alcool à 1 à 4 atomes de carbone, en chauffant;
h) lorsque Q est $-CH_2-$ et R est un halogène, on halogène un composé de la formule (I) dans lequel Q est $-CH_2-$ et R est un groupe hydroxyle, avec un agent d'halogénation dans un solvant; ou
i) lorsque Q est $-CH_2-$ et R est

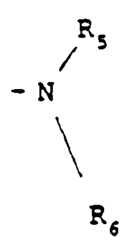

dans laquelle

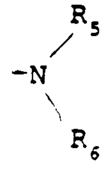

est comme défini ci-dessus dans le composé de la formule (I), on amine un composé de la formule (I) dans lequel Q est $-CH_2-$ et R est un halogène, avec une amine de la formule (5) comme définie ci-dessus, dans un solvant, en chauffant.

**2.** Procédé selon la revendication 1, dans lequel Q est -CO- et $R_2$ et $R_3$ sont indépendamment un groupe alcoyle à 1 à 4 atomes de carbone.

**3.** Procédé selon la revendication 1, dans lequel le composé de la formule (I) a la formule :

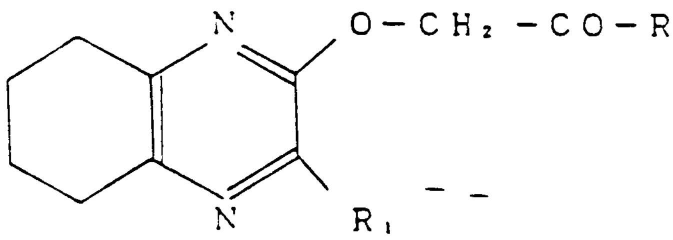

dans laquelle R et $R_1$ sont comme définis dans la revendication 1.

**4.** Procédé selon la revendication 1, dans lequel $R_4$ est de l'hydrogène, Q est $-CH_2-$, $R_2$ et $R_3$ sont chacun indépendamment un alcoyle à 1 à 4 atomes de carbone, et R est un hydroxyle, un halogène ou

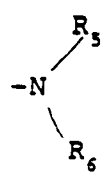

comme défini dans la revendication 1.

**5.** Procédé selon la revendication 1, dans lequel le composé de la formule (I) a la formule :

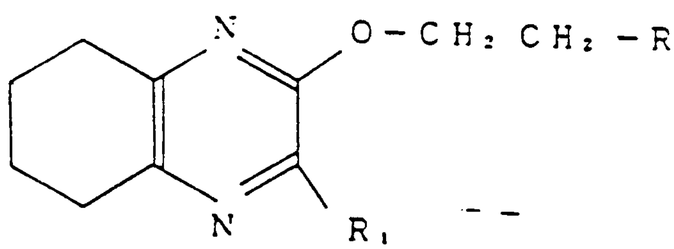

dans laquelle R est un hydroxyle, un halogène ou

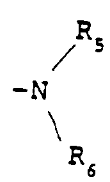

et $R_1$ et

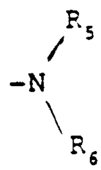

sont comme définis dans la revendication 1.

6. Procédé pour préparer une composition pharmaceutique, qui consiste à mélanger un composé de la formule (I) comme défini dans la revendication 1, ou un de ses sels pharmaceutiquement acceptables, avec un diluant pharmaceutiquement acceptable.

**Patentansprüche**
**Patentansprüche für folgende Vertragsstaaten : DE, FR, IT**

1. Pyrazinderivate der allgemeinen Formel

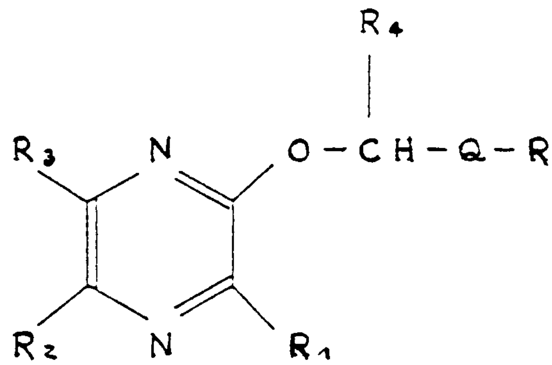

worin

Q für -CO- oder $-CH_2-$ steht,
R gleich Hydroxyl, $C_1$-$C_4$ -Alkoxy, Halogen, -NH-$C_1$-$C_4$-Alkylen-OH, -NH-$C_1$-$C_4$ -Alkylen-arylthio, NH-$C_1$-$C_4$-Alkylen-halogen,

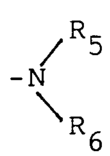

oder

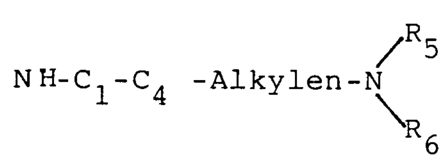

ist, worin

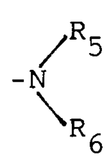

Di-$C_1$-$C_4$ -alkylamino, Morpholino,

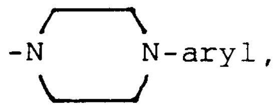

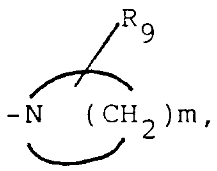

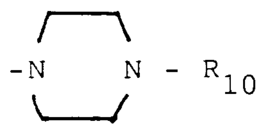

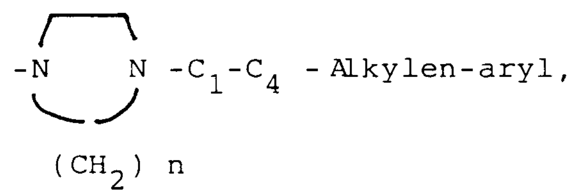

oder

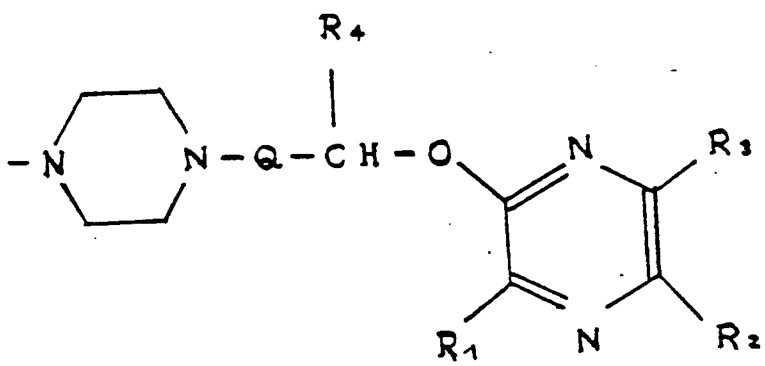

ist, worin $R_9$ für ein Wasserstoffatom oder einen $C_1$-$C_4$-Alkylrest steht, $R_{10}$ ein Wasserstoffattom, $C_1$-$C_4$-Alkyl, Hydroxy-$C_1$-$C_4$-alkyl, Hydroxy-$C_1$-$C_4$- alkoxy-$C_1$-$C_4$-alkyl oder Di(aryl)-$C_1$-$C_4$-alkyl ist, m für eine ganze Zahl von 4 bis 6 steht, n gleich 2 oder 3 ist,

$R_1$ für $C_1$-$C_{20}$ -Alkyl oder Aryl-$C_1$-$C_4$-alkyl steht,
$R_2$ und $R_3$ unabhängig voneinander jeweils $C_1$-$C_4$-Alkyl darstellen oder zusammen eine Tetramethylenbrücke bilden, und
$R_4$ Wasserstoff, $C_1$-$C_4$-Alkyl oder Aryl bedeutet,

worin jeder Arylrest einen Phenylrest bedeutet, der gegebenenfalls durch 1 bis 3 Halogenatome oder Nitrogruppen, $C_1$-$C_4$-Alkyl oder $C_1$-$C_4$-Alkoxygruppen substituiert ist, oder ein pharmazeutisch verträgliches Salz davon, mit der Maßgabe, daß -sofern Q gleich $-CH_2-$ ist, $R_1$, $R_2$ und $R_3$ jeweils Methylreste sind und $R_4$ ein Wasserstoffatom bedeutet, R nicht

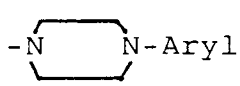

ist, worin Aryl gleich

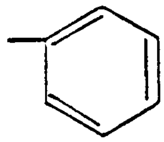, 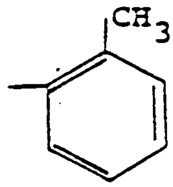 oder 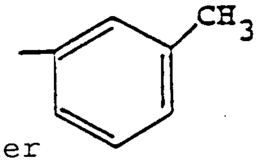

ist.

2. Verbindung nach Anspruch 1 , worin Q für -CO- steht, und $R_2$ und $R_3$ jeweils unabhängig voneinander $C_1$-$C_4$-alkyl sind.

3. Verbindung nach Anspruch 1 gemäß der Formel

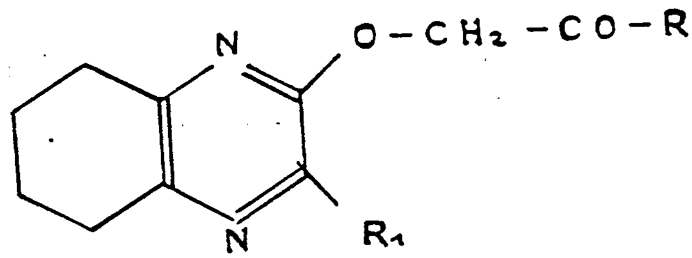

worin R und $R_1$ der Definition gemäß Anspruch 1 entsprechen, oder ein pharmazeutisch verträgliches Salz davon.

4. Verbindung nach Anspruch 1, worin $R_4$ ein Wasserstoffatom bedeutet, Q gleich $-CH_2-$ ist, $R_2$ und $R_3$ jeweils unabhängig voneinander für $C_1$- bis $C_4$-Alkyl stehen und R gleich Hydroxyl, Halogen oder

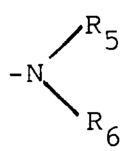

gemäß Definition in Anspruch 1 ist.

5. Verbindung gemäß Anspruch 1 der Formel

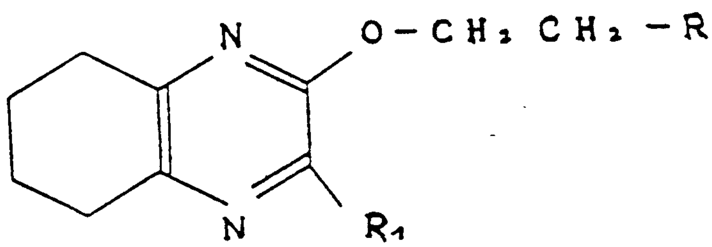

worin R für einen Hydroxylrest, ein Halogenatom oder

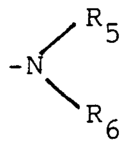 steht, und 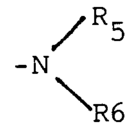

und $R_1$ der Definition gemäß Anspruch 1 entsprechen, oder ein pharmazeutisch verträgliches Salz davon.

**6.** Zusammensetzung, welche eine Verbindung der Formel (I) gemäß der Definition in Anspruch 1 oder ein pharmazeutisch verträgliches Salz davon umfaßt, in einem pharmazeutisch verträglichen Verdünnungsmittel.

**7.** Verbindung nach Formel (I) gemäß Definition in Anspruch 1 oder ein pharmazeutisch verträgliches Salz davon zur Verwendung in einem therapeutischen Behandlungsverfahren.

**8.** Verbindung gemäß Formel (I) gemäß Definition in Anspruch 1 oder ein pharmazeutisch verträgliches Salz davon für die Verwendung bei der Behandlung von Kreislauf- oder Stoffwechselerkrankungen.

**9.** Verwendung einer Verbindung der Formel (I) gemäß Definition in Anspruch 1 oder eines pharmazeutisch verträglichen Salzes davon bei der Herstellung eines Medikamtes zur Behandlung von Kreislauf- oder Stoffwechselerkrankungen.

**10.** Verfahren zu Herstellung einer Verbindung der Formel (I) gemäß Anspruch 1 oder eines pharmazeutisch verträglichen Salzes davon, wobei

a) wenn Q für -CO- steht und R gleich $C_1$-$C_4$- Alkoxy ist, eine Verbindung der Formel

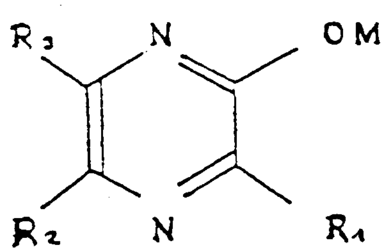

(3)

worin M für ein Alkalimetall steht und $R_1$, $R_2$ und $R_3$ der obigen Definition in der Verbindung der Formel (I) entsprechen, mit einem $\alpha$-Halogencarbonsäure ester der Formel

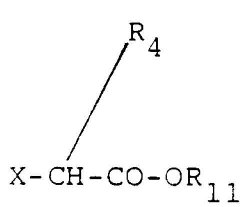

(4)

worin X ein Halogenatom darstellt, $R_{11}$ für $C_1$-$C_4$-Alkyl steht und $R_4$ der Definition in der Verbindung gemäß Formel (I) entspricht, in einem organischen Lösungsmittel umgesetzt wird,

b) wenn Q gleich -CO- ist und R für Hydroxyl steht, eine Verbindung der Formel (I), worin Q für -CO-steht und R gleich $C_1$-$C_4$-Alkoxy ist, entestert wird,

c) wenn Q gleich -CO- ist und R für NH-$C_1$-$C_4$-Alkylen-OH oder

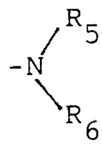 steht, worin 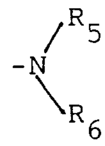

der obigen Definition in der Verbindung gemäß Formel (I) entspricht, ein Amin der Formel

H-R' (5)

worin R' gleich $-NH-C_1-C_4$-Alkylen-OH oder

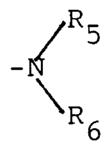

ist, worin

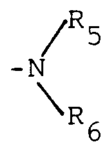

Di-$C_1$-$C_4$-Alkylamino, Morpholino,

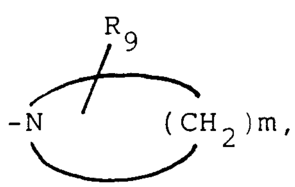

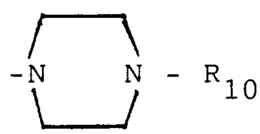

oder

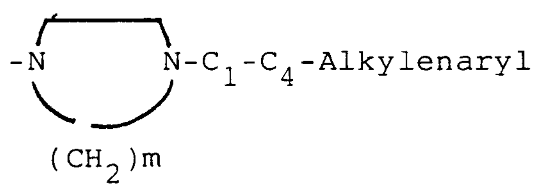

ist und
$R_9$, $R_{10}$ Aryl sind, und m und n der Definition in der Verbindung gemäß der Formel (I) ensprechen, mit einer Verbindung der Formel (I), worin Q für -CO- steht und R Hydroxyl ist, oder einem reaktiven Derivat davon, welches zur N-Acylierung geeignet ist, umgesetzt wird,
d) wenn Q gleich -CO- ist und R für $-NH-C_1-C_4$-Alkylenhalogen steht, eine Verbindung der Formel

$X-C_1-C_4$-Alkylen-$NH_2$ (6)

worin X ein Halogenatom ist, mit einer Verbindung der Formel (I), worin Q gleich -CO- ist und R für Hydroxyl steht, oder einem reaktiven Derivat davon, welches sich zur N-Acylierung eignet, N-acyliert

wird,
e) wenn Q für -CO- steht und R gleich

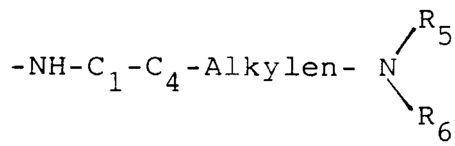

steht, eine Verbindung der Formel (I), worin Q gleich -CO- ist und R für -NH-$C_1$-$C_4$-Alkylenhalogen steht, mit einem Amin der Formel

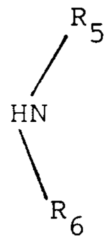

worin

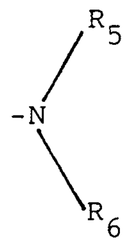

der Definition in Anspruch 1 entspricht, aminiert wird,
f) wenn Q gleich -$CH_2$- ist und R für -NH-$C_1$-$C_4$ -Alkylenarylthio steht, eine Verbindung der Formel (I), worin Q gleich -$CH_2$- ist und R für -NH-$C_1$-$C_4$ -Alkylenhalogen steht mit einem Arylthiol thioetherifiziert wird.
g) wenn Q gleich -$CH_2$- ist und R einen Hydroxylrest darstellt, eine Verbindung der Formel (I), in der Q gleich -CO- ist und R für $C_1$-$C_4$ -Alkoxy steht, mit einem Alkaliborhydrid in Gegenwart eines $C_1$-$C_4$-Alkohols unter Erwärmung reduziert wird,
h) wenn Q gleich -$CH_2$- ist und R ein Halogenatom bedeutet, eine Verbindung der Formel (I), worin Q gleich -$CH_2$- ist und R einen Hydroxylrest bedeutet, mit einem Halogenierungsmittel in einem Lösungsmittel halogeniert wird, oder
i) wenn Q gleich -$CH_2$- ist und R für

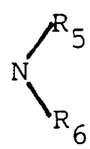

steht, worin

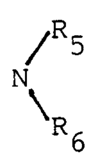

der obigen Definition in der Verbindung der Formel (I) entspricht, eine Verbindung der Formel (I), worin Q gleich -$CH_2$- ist und R ein Halogenatom bedeutet, mit einem Amin der Formel (5) gemäß obiger Definition in einem Lösungsmittel unter Erwärmung aminiert wird.

**Patentansprüche für folgenden Vertragsstaat : ES**

**1.** Verfahren zur Herstellung von Pyrazinderivaten der allgemeinen Formel

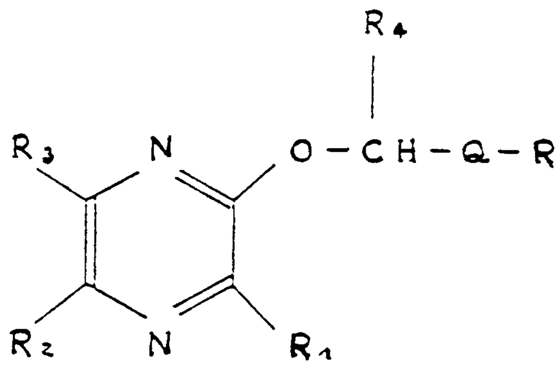

worin

Q für -CO- oder $-CH_2-$ steht,

R gleich Hydroxyl, $C_1$-$C_4$ -Alkoxy, Halogen, -NH-$C_1$-$C_4$-Alkylen-OH, -NH-$C_1$-$C_4$ -Alkylen-arylthio, NH-$C_1$-$C_4$-Alkylen-halogen,

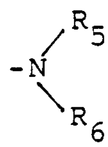 oder 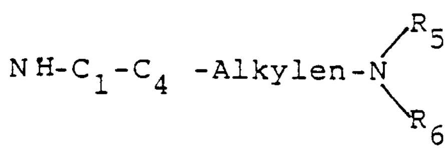

ist, worin

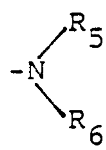

Di-$C_1$-$C_4$ -alkylamino, Morpholino,

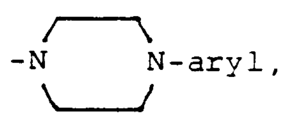

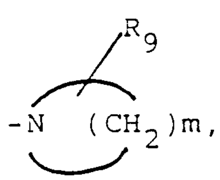, 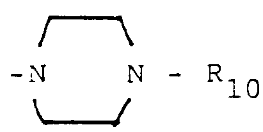, 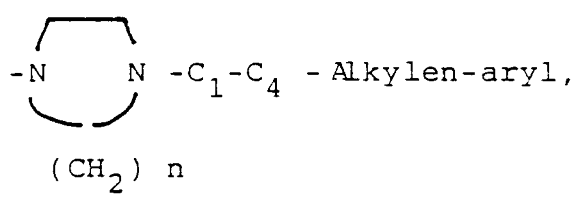

oder

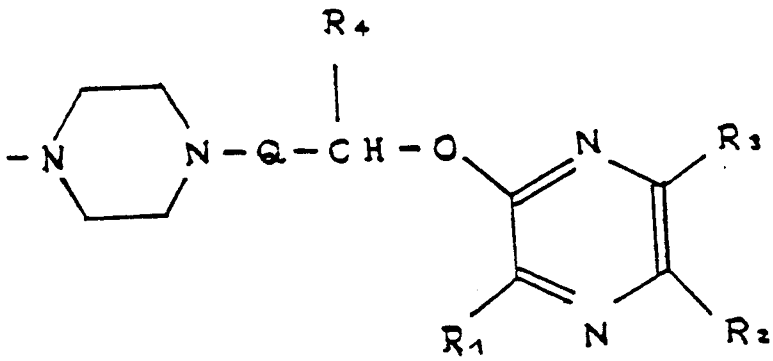

ist, worin $R_9$ für ein Wasserstoffatom oder einen $C_1$-$C_4$-Alkylrest steht, $R_{10}$ ein Wasserstoffatom, $C_1$-$C_4$-Alkyl, Hydroxy-$C_1$-$C_4$-alkyl, Hydroxy-$C_1$-$C_4$- alkoxy-$C_1$-$C_4$-alkyl oder Di(aryl)-$C_1$-$C_4$-alkyl ist, m für eine ganze Zahl von 4 bis 6 steht, n gleich 2 oder 3 ist,

$R_1$ für $C_1$-$C_{20}$ -Alkyl oder Aryl-$C_1$-$C_4$-alkyl steht,
$R_2$ und $R_3$ unabhängig voneinander jeweils $C_1$-$C_4$-Alkyl darstellen oder zusammen eine Tetramethylenbrücke bilden, und
$R_4$ Wasserstoff, $C_1$-$C_4$-Alkyl oder-Aryl bedeutet,

worin jeder Arylrest einen Phenylrest bedeutet, der gegebenenfalls durch 1 bis 3 Halogenatome oder Nitrogruppen, $C_1$-$C_4$-Alkyl oder $C_1$-$C_4$-Alkoxygruppen substituiert ist, oder ein pharmazeutisch verträgliches Salz davon, mit der Maßgabe, daß -sofern Q gleich $-CH_2-$ ist, $R_1$, $R_2$ und $R_3$ jeweils Methylreste sind und $R_4$ ein Wasserstoffatom bedeutet, R nicht

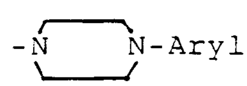

ist, worin Aryl gleich

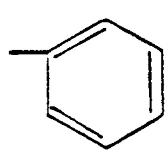

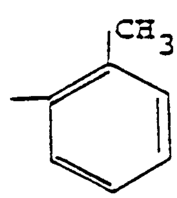

oder

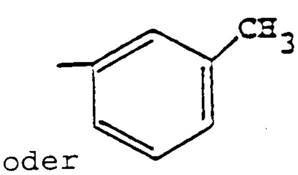

ist, wobei

a) wenn Q für -CO- steht und R gleich $C_1$-$C_4$- Alkoxy ist, eine Verbindung der Formel

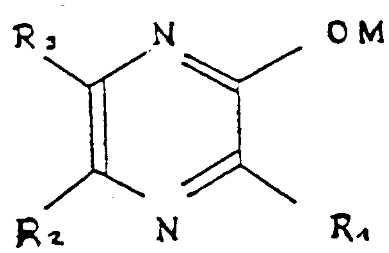

(3)

worin M für ein Alkalimetall steht und $R_1$, $R_2$ und $R_3$ der obigen Definition in der Verbindung der

Formel (I) entsprechen, mit einem α-Halogencarbonsäure ester der Formel

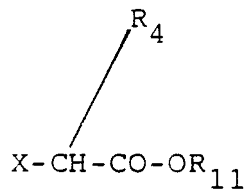

(4)

worin X ein Halogenatom darstellt, $R_{11}$ für $C_1$-$C_4$-Alkyl steht und $R_4$ der Definition in der Verbindung gemäß Formel (I) entspricht, in einem organischen Lösungsmittel umgesetzt wird,

b) wenn Q gleich -CO- ist und R für Hydroxyl steht, eine Verbindung der Formel (I), worin Q für -CO-steht und R gleich $C_1$-$C_4$-Alkoxy ist, entestert wird,

c) wenn Q gleich -CO- ist und R für-NH-$C_1$-$C_4$-Alkylen-OH oder

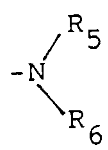

steht, worin

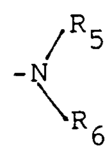

der obigen Definition in der Verbindung gemäß Formel (I) entspricht, ein Amin der Formel

H-R'   (5)

worin R' gleich -NH-$C_1$-$C_4$-Alkylen-OH oder

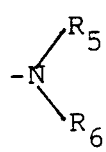

ist, worin

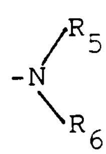

Di-$C_1$-$C_4$-Alkylamino, Morpholino,

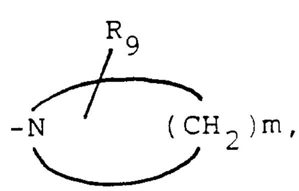

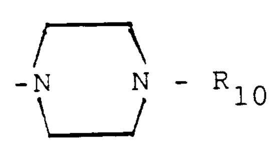

oder

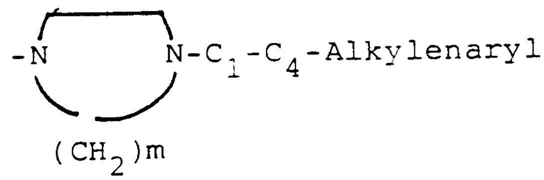

ist und

$R_9$, $R_{10}$ Aryl sind, und m und n der Definition in der Verbindung gemäß der Formel (I) ensprechen, mit einer Verbindung der Formel (I), worin Q für -CO- steht und R Hydroxyl ist, oder einem reaktiven Derivat davon, welches zur N-Acylierung geeignet ist, umgesetzt wird,

d) wenn Q gleich -CO- ist und R für -NH-$C_1$-$C_4$-Alkylenhalogen steht, eine Verbindung der Formel

X-$C_1$-$C_4$-Alkylen-$NH_2$ (6)

worin X ein Halogenatom ist, mit einer Verbindung der Formel (I), worin Q gleich -CO- ist und R für Hydroxyl steht, oder einem reaktiven Derivat davon, welches sich zur N-Acylierung eignet, N-acyliert wird,

e) wenn Q für -CO- steht und R gleich

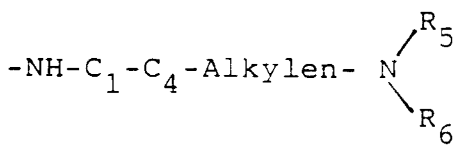

steht, eine Verbindung der Formel (I), worin Q gleich -CO- ist und R für -NH-$C_1$-$C_4$-Alkylenhalogen steht, mit einem Amin der Formel

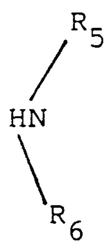 worin 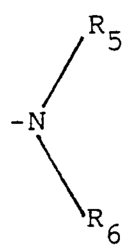

der Definition in Anspruch 1 entspricht, aminiert wird,

f) wenn Q gleich -$CH_2$- ist und R für -NH-$C_1$-$C_4$ Alkylenarylthio steht, eine Verbindung der Formel (I), worin Q gleich -$CH_2$- ist und R für -NH-$C_1$-$C_4$ Alkylenhalogen steht mit einem Arylthiol thioetherifiziert wird.

g) wenn Q gleich -$CH_2$- ist und R einen Hydroxylrest darstellt, eine Verbindung der Formel (I), in der Q gleich -CO- ist und R für $C_1$-$C_4$ -Alkoxy steht, mit einem Alkaliborhydrid in Gegenwart eines $C_1$-$C_4$-Alkohols unter Erwärmung reduziert wird,

h) wenn Q gleich -$CH_2$- ist und R ein Halogenatom bedeutet, eine Verbindung der Formel (I), worin Q gleich -$CH_2$- ist und R einen Hydroxylrest bedeutet, mit einem Halogenierungsmittel in einem Lösungsmittel halogeniert wird, oder

i) wenn Q gleich -$CH_2$- ist und R für

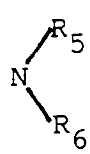

steht, worin

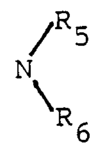

der obigen Definition in der Verbindung der Formel (I) entspricht, eine Verbindung der Formel (I), worin Q gleich $-CH_2-$ ist und R ein Halogenatom bedeutet, mit einem Amin der Formel (5) gemäß obiger Definition in einem Lösungsmittel unter Erwärmung aminiert wird.

**2.** Verfahren nach Anspruch 1, dadurch gekennzeichnet, daß Q fur -CO- steht und $R_2$ und $R_3$ jeweils einen $C_1$ -$C_4$ -Alkylrest darstellen.

**3.** Verfahren nach Anspruch 1, dadurch gekennzeichnet, daß die Verbindung gemäß Formel (I)

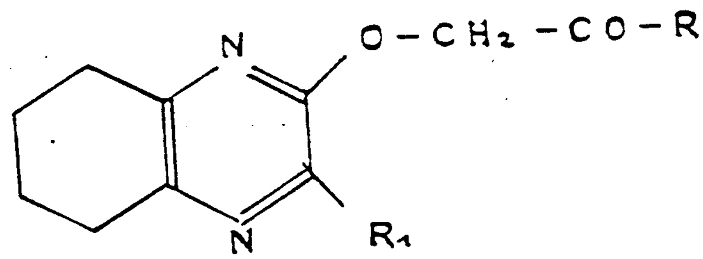

ist, worin R und $R_1$ der Definition in Anspruch 1 entsprechen.

**4.** Verfahren nach Anspruch 1, worin $R_4$ für Wasserstoff steht, Q gleich $-CH_2-$ ist, $R_2$ und $R_3$ jeweils $C_1$ -$C_4$ -Alkyl darstellen und R Hydroxyl, Halogen oder

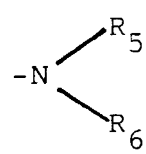

gemäß Definition in Anspruch 1 bedeutet

**5.** Verfahren nach Anspruch 1, dadurch gekennzeichnet, daß die Verbindung der Formel (I)

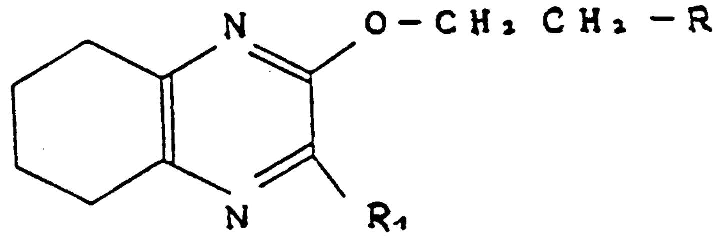

ist, worin R gleich Hydroxyl, ein Halogenatom oder

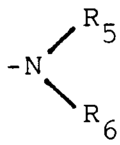

darstellt und $R_1$ und

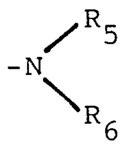

der Definition in Anspruch 1 entsprechen.

**6.** Verfahren zur Herstellung einer pharmazeutischen Zusammensetzung, dadurch gekennzeichnet, daß eine Verbindung der Formel (I) gemäß Definition in Anspruch 1 oder ein pharmazeutisch verträgliches Salz davon mit einem pharmazeutisch verträglichen Verdünnungsmittel vermischt wird.